(19) Europäisches Patentamt
European Patent Office
Office européen des brevets

(11) EP 2 506 015 A1

# (12) EUROPEAN PATENT APPLICATION

(43) Date of publication:
**03.10.2012 Bulletin 2012/40**

(51) Int Cl.:
***G01N 33/574*** *(2006.01)*

(21) Application number: **11002747.1**

(22) Date of filing: **01.04.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Universität Regensburg**
**93053 Regensburg (DE)**

(72) Inventor: **The designation of the inventor has not yet been filed**

(74) Representative: **Vossius & Partner**
**Siebertstrasse 4**
**81675 München (DE)**

## (54) A prognostic and therapeutic signature for malignant melanoma

(57) The present invention relates to a method of predicting the course of disease in a patient having a malignant melanoma, the method comprising determining in melanoma cells comprised in a sample obtained from said malignant melanoma the presence or amount of at least five biomarkers selected from the group comprising or consisting of MTAP, PTEN, Bax, Bcl-X, β-Catenin, CD20, Cox-2, CD49d and MLH1, wherein the absence or decreased amount of MTAP and β-Catenin and/or the presence or increased amount of PTEN, Bax, Bcl-X, CD20, Cox-2, CD49d and MLH1, is associated with a disadvantageous course of disease. The present invention further relates to a method of preparing a tailored pharmaceutical composition for a patient having a malignant melanoma, a kit for predicting the course of disease in a patient having a malignant melanoma, a kit for preparing a tailored pharmaceutical composition for a patient having a malignant melanoma as well as a pharmaceutical composition for use in treating or preventing malignant melanoma.

EP 2 506 015 A1

## Description

The present invention relates to a method of predicting the course of disease in a patient having a malignant melanoma, the method comprising determining in melanoma cells comprised in a sample obtained from said malignant melanoma the presence or amount of at least five biomarkers selected from the group comprising or consisting of MTAP, PTEN, Bax, Bcl-X, β-Catenin, CD20, Cox-2, CD49d and MLH1, wherein the absence or decreased amount of MTAP and β-Catenin and/or the presence or increased amount of PTEN, Bax, Bcl-X, CD20, Cox-2, CD49d and MLH1 is associated with a disadvantageous course of disease. The present invention further relates to a method of preparing a tailored pharmaceutical composition for a patient having a malignant melanoma, a kit for predicting the course of disease in a patient having a malignant melanoma, a kit for preparing a tailored pharmaceutical composition for a patient having a malignant melanoma as well as a pharmaceutical composition for use in treating or preventing malignant melanoma.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Cutaneous malignant melanoma (MM), represents the most common cause of death from skin cancer, and, apart from female lung cancer, it is the tumour entity with the highest increase of incidence worldwide.[1] Malignant melanoma is characterized by a multi-factorial aetiology. Sun exposure and genetic susceptibility have been proposed as major aetiological and predisposing factors and may explain the reported increase of incidence to some degree.[2]

The metastatic stage IV of malignant melanoma with an average 10-year survival rate ranging from 9% to 15% (depending on its pattern of metastasis)[3] cannot yet be cured and improvement in overall survival among these patients remains an elusive goal. Despite novel therapeutic approaches, the prognosis of patients suffering from metastatic stage IV malignant melanoma remains unfavourable.[4]

De facto, the prognosis of patients with malignant melanoma may only in part be derived from clinical and histological parameters. According to the AJCC 2009 classification[5], the findings of vertical tumour thickness,[6] tumour ulceration[7] and sentinel node biopsy[8] represent the most dominant prognostic factors. In stage pT1 melanomas (≤ 1.00 thickness), the mitotic rate (histologically defined as mitoses/$mm^2$) has to be considered as additional prognostic parameter.[5] These current staging methods such as tumour thickness, ulceration and invasion of the sentinel node are known to be prognostic parameters in patients with malignant melanoma. However, predictive molecular marker profiles for risk stratification and therapy optimization are not yet available for routine clinical assessment.

Rothberg et *al.* 2009[9] describe a meta-analysis of published literature to identify associations between immunohistochemical expression and survival outcomes in melanoma. Promising markers identified by Rothberg and coworkers include MUC18, MMP-2, Ki-67, PCNA and P16/INK4A. The authors conclude that these results require validation in adequately powered studies.

Rothberg and Rimm 2010[10] describe the analysis of data not eligible for the meta-analysis performed in Rothberg et *al.* 2009[9] but nonetheless of potential value in providing a prioritised list of protein candidates for further studies with the aim of identifying prospective prognostic markers. The authors provide a list of proteins that they recommend as a priority set for inclusion in studies of melanoma prognosis.

Alonso *et al.* 2004[12] describe protein expression profiles at the different stages of malignant melanoma progression. A predictor model for survival was established, including the proteins $p16^{INK4a}$, Ki67, $P21^{CIP1}$ and Bcl-6. The proteins BCLX-L, MLH-1 and TOP2A, although analysed, were not further considered in the prognostic model.

Wild et *al.* 2006[13] describe a reduced expression of MTAP in primary malignant melanomas and in melanoma metastases compared with benign nevi. However, in the overall cohort, MTAP expression was not associated with prognosis. Instead, MTAP expression was found to correlate with responsiveness to interferon therapy.

In a later study based on a larger cohort of patients, Meyer *et al.* 2010[40] further investigated whether expression of MTAP is of prognostic or therapeutic relevance in patients with melanoma. An association between MTAP immunoreactivity and overall survival as well as recurrence-free survival was shown in this patient group.

Meyer et *al.* 2009[14] describe a study to correlate Cox-2 immuno-reactivity in tumours to the outcome of patients with malignant melanoma. Cox-2 expression was found to be significantly increased from nevi to primary malignant melanoma and metastases and Cox-2 positivity was associated with shorter recurrences-free survival of the patients. The authors concluded that Cox-2 expression in primary malignant melanoma indicates an increased risk of tumour recurrence. However, no association with longer progression-free survival could be shown in patients with malignant melanoma metastases who had received bio-modulatory therapy. Thus, the authors conclude that Cox-2 might mainly contribute to early steps in melanoma progression, such as growth and invasion of primary malignant melanoma but might be less essential in the advanced metastatic setting of melanoma disease. Nonetheless, a different study by Reichle et *al.* 2007[33] describes a phase II trial showing beneficial effects of a Cox-2 inhibitor in patients with stage IV (i.e. metastatic) melanoma.

WO 2008/141275 describes a large amount of molecular markers expressed at certain stages of malignant

melanoma and states that said markers may be employed to predict the malignancy potential of a malignant melanoma and to determine the correct treatment regimen. However, no correlation of molecular markers with the course of disease, such as recurrence-free survival or overall survival, is shown in WO 2008/141275.

Despite the fact that hundreds of studies sought to assess the potential prognostic value of molecular markers in predicting the course of cutaneous malignant melanoma, according to the latest review meta-analyses,[9,10] there are no predictive molecular profiles for risk association or therapy optimisation applicable for routine clinical assessment of malignant melanoma. Furthermore, the variability of clinical behaviour in patients with malignant melanoma can only partially be explained by clinical and histological data and, thus, there is a need to identify biological marker profiles for use in assigning patients to a specific risk group.

This need is addressed by the provision of the embodiments characterised in the claims.

Accordingly, the present invention relates to a method of predicting the course of disease in a patient having a malignant melanoma, the method comprising determining in melanoma cells comprised in a sample obtained from said malignant melanoma the presence or amount of at least five biomarkers selected from the group comprising or consisting of MTAP, PTEN, Bax, Bcl-X, β-Catenin, CD20, Cox-2, CD49d and MLH1, wherein the absence or decreased amount of MTAP and β-Catenin and/or the presence or increased amount of PTEN, Bax, Bcl-X, CD20, Cox-2, CD49d and MLH1 is associated with a disadvantageous course of disease.

In accordance with the present invention, the term "predicting the course of disease" refers to the provision of an assessment whether the malignant melanoma has a favourable progression/outcome or an unfavourable progression/ outcome. The term disease, in this regard, refers to malignant melanoma. A favourable progression/outcome, also referred to herein as an advantageous course of disease, relates to no risk or a low risk of recurrence of malignant melanoma and/or a long time of disease-free survival, such as for example more than 5 years. An unfavourable progression/outcome, also referred to herein as a disadvantageous course of disease, relates to a high risk of recurrence of malignant melanoma, including e.g. the formation of local malignant melanoma as well as the formation of regional or distant metastases, and/or a short time of disease-free survival such as e.g. less than 5 years and/or a short overall survival time. The term "recurrence", as used herein, relates to the repeated outbreak of malignant melanoma, or a progression of the malignant melanoma such as for example in terms of formation of metastases from the malignant melanoma analysed but independently of whether the disease was cured before said outbreak or progression.

As used herein, the term "malignant melanoma" refers to a type of skin cancer well known in the medical art. Melanoma is the type of skin cancer that has the highest grade of malignancy. Among cells composing skin, melanin-pigment-producing cells are referred to as pigment cells or melanocytes. When these melanocytes become cancerous, a malignant melanoma is developed. Malignant melanoma is staged according to the severity of the disease into stage 0 to stage 4. Stage 0 refers to melanoma in situ with 99.9% survival. Stage I/II refers to invasive melanoma with 85 to 99% survival and is further divided into T1a (less than 1.00 mm primary tumour thickness, without ulceration and mitosis $< 1/mm^2$) , T1b (less than 1.00 mm primary tumour thickness, with ulceration or mitoses $\geq 1/mm^2$) and T2a (1.00 to 2.00 mm primary tumour thickness, without ulceration). Stage II refers to high risk melanoma with 40 to 85% survival and is further divided into T2b (1.00 to 2.00 mm primary tumour thickness, with ulceration), T3a (2.00 to 4.00 mm primary tumour thickness, without ulceration), T3b (2.00 to 4.00 mm primary tumour thickness, with ulceration), T4a (4.00 mm or greater primary tumour thickness without ulceration) and T4b (4.00 mm or greater primary tumour thickness with ulceration). Stage III refers to regional metastasis with 25 to 60% survival and is further divided into N1 (single positive lymph node), N2 (2 to 3 positive lymph nodes or regional skin/in-transit metastasis) and N3 (four positive lymph nodes or lymph node and regional skin/in-transit metastases). Finally, stage IV refers to distant metastasis with only 9 to 15% survival. Stage IV is further divided into M1a (distant skin metastasis and normal lactate dehydrogenase), M1b (lung metastasis, normal lactate dehydrogenase) and M1c (other distant metastasis or any distant metastasis with elevated lactate dehydrogenase).

The term "melanoma cells", as used herein, refers to melanocytes that have become cancerous Melanocytes, including melanoma cells, are well known to the skilled person and can be easily identified in a sample due to their location in the stratum basale of the epidermis as well as via melanocyte-specific markers including, but not limited to, Melan-A, HMB45, Protein S100, DCT and TRP2.

The term "biomarker selected from the group [...]" according to the present invention relates to the recited markers in any of their naturally occurring forms, including nucleic acid molecules such as e.g. DNA, including cDNA or genomic DNA, and RNA as well as proteins.

As used herein, the term "determining the [...] presence" refers to determining whether the analysed biomarker is present or absent in melanoma cells comprised in the sample investigated. The biomarker is considered present in accordance with the present invention when it is detected in amounts exceeding the standard procedural error, such as for example observed in the form of background staining obtained in immunohistochemical or western blot analyses. The skilled person knows how to determine such procedural errors, for example by analysing non-disease control samples or by omitting certain steps or compounds in the procedure, such as for example a primary antibody in immunohistochemical stainings or a template in nucleic acid amplification techniques etc.. In the case that the amount of

biomarker detected corresponds to or is less than the standard procedural error, e.g. the background staining in an immunohistochemical analysis, the biomarker is considered as not being present in the sample.

As used herein, the term "determining the [...] amount" refers to quantitatively or semi-quantitatively determining amounts of the respective biomarkers. Quantitative analysis refers to the determination of absolute or normalised (e.g. compared to a non-changing reference marker) values of biomarker amounts, such as e.g. copy numbers of nucleic acids or intensities of stainings in immunohistochemical or western blot techniques. Furthermore, the number of stained cells versus cells not stained may be evaluated. Semi-quantitative analysis refers to the determination of relative amounts, such as for example by visual analysis of immunohistochemically stained samples by a skilled person, such as e.g. a dermato-histopathologist or a surgical pathologist. As described in the appended examples, such analyses may be performed based on a step-wise scoring system allocating different scores to samples containing e.g. no staining, weak staining, moderate staining, strong staining, very strong staining etc..

The term "decreased amount", as used herein, refers to lower expression levels of the biomarker of interest in melanoma cells in the sample obtained from the malignant melanoma as compared to expression levels observed in a control sample, such as for example non-malignant tissues. Preferably, the term relates to statistically significant lower expression levels of the biomarker of interest in melanoma cells in the sample obtained from the malignant melanoma of interest as compared to expression levels observed in the control tissues. Expression levels observed in non-malignant tissues may for example be analysed in a parallel control experiment based on a disease-free sample, such as for example on benign nevi obtained from the same patient. The amount of biomarker is considered to be decreased when its amount is at least 10% lower in the malignant melanoma sample than in non-malignant tissues, such as for example at least 20% lower, at least 30% lower, at least 40% lower, at least 50% lower, at least 75% lower, at least 100% lower (i.e. twice as low), at least 200% lower, at least 300% lower, at least 500% lower etc..

The term "increased amount", as used herein, refers to higher expression levels of the biomarker of interest in melanoma cells in the sample obtained from the malignant melanoma as compared to expression levels observed in a control sample, such as for example non-malignant tissues. Preferably, the term relates to statistically significant higher expression levels of the biomarker of interest in melanoma cells in the sample obtained from the malignant melanoma of interest as compared to expression levels observed in the control tissues. Expression levels observed in non-malignant tissues may for example be analysed in a parallel control experiment based on a disease-free sample, such as for example on benign nevi obtained from the same patient. The amount of biomarker is considered to be abnormally increased when its amount is at least 10% higher in the malignant melanoma sample than in non-malignant tissues, such as for example at least 20% higher, at least 30% higher, at least 40% higher, at least 50% higher, at least 75% higher, at least 100% higher (i.e. twice as high), at least 200% higher, at least 300% higher, at least 500% higher etc..

The term "expression level", as used herein, refers to a value of expression of a particular marker in a sample of interest. The expression level of a marker corresponds to the number of copies of the expression product of the corresponding gene, either on a nucleic acid level (e.g. mRNA) or on the protein level. Thus, the determination of the expression level of a particular marker can, for example, be carried out on the nucleic acid level or on the level of the respective protein encoded by said gene.

Methods for the determination of expression levels of a protein on the amino acid level include but are not limited to immunohistochemical methods as described in the appended examples but also e.g. Western blotting or polyacrylamide gel electrophoresis in conjunction with protein staining techniques such as Coomassie Brilliant blue or silver-staining. For these latter methods, the total protein is loaded onto a polyacrylamide gel and separated by electrophoresis. Afterwards, the separated proteins are transferred onto a membrane, e.g. a polyvinyldifluoride (PVDF) membrane, by applying an electrical current. The proteins on the membrane are exposed to an antibody specifically recognizing the protein of interest. After washing, typically a second antibody specifically recognizing the first antibody and carrying a readout system such as a fluorescent dye is applied. The amount of the protein of interest is often determined by comparing the fluorescence intensity of the protein derived from a sample of the patient of interest with the fluorescence intensity obtained with the protein derived from a control sample. Also of use in protein quantification is the Agilent Bioanalyzer technique.

Methods for determining expression levels on the nucleic acid level include, but are not limited to, Northern blotting, PCR, RT-PCR or real RT-PCR. PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed, for example, in a 50 μl reaction mixture containing 5 μl of 10 x PCR buffer with 1.5 mM $MgCl_2$, 200 μM of each deoxynucleoside triphosphate, 0.5 μl of each primer (10 μM), about 10 to 100ng of template DNA and 1 to 2.5 units of Taq Polymerase. The primers for the amplification may be labelled or be unlabelled. DNA amplification can be performed, e.g., with a model 2400 thermal cycler (Applied Biosystems, Foster City, CA): 2 min at

94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step at 55°C for 1 min as well as a final extension step at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. The person skilled in the art knows how to optimize PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT Reaction can be performed, for example, in a 20μl reaction mix containing: 4 μl of 5x AMV-RT buffer, 2 μl of Oligo dT (100 μg/ml), 2μl of 10 mM dNTPs, 1μl total RNA, 10 Units of AMV reverse transcriptase, and $H_2O$ to 20μl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case.

The resulting products may be loaded onto an agarose gel and band intensities are compared after staining the nucleic acid molecules with an intercalating dye such as ethidiumbromide or SybrGreen.

Real-time PCR employs a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor, which was formerly quenched due to the close proximity to the 3' acceptor in the TaqMan probe sequence. Thereby, the process of amplification can be monitored directly and in real time, which permits a significantly more precise determination of expression levels than conventional end-point PCR. Also of use in Real-time RT-PCR experiments is a DNA intercalating dye such as SybrGreen for monitoring the de novo synthesis of double stranded DNA molecules.

The skilled person is aware that mutations and/or variations in the genes encoding the biomarkers in accordance with the present invention as well as in the regulatory elements of said genes (e.g. promoters, enhancers etc.) may be causative or associated with a change of expression levels of said biomarkers. For example, *PTEN* mutations and deficiencies are prevalent in many types of human cancers, leading to a loss of functional PTEN protein in those cancers (Mirmohammadsadegh *et al.* 2006[43]; Lahtz *et al.* 2010[44]; Zhou *et al.* 2000[45]; Zhang and Yu 2010[28]). Furthermore, promoter hyper-methylation has been shown to lead to a loss of MTAP expression (Behrmann et *al.* 2003). Thus, it is also envisaged herein that the determination of the presence or amount of the biomarkers in accordance with the present invention is based on the detection of mutations (i.e. genetic changes) or variations (i.e. epigenetic changes) of said biomarkers. Methods for determining mutations and/or variations in genes are well known in the art and include, without being limiting PCR based techniques, DNA sequencing-based techniques, hybridization-based techniques, single-strand conformation polymorphism analysis (SSCA), denaturating gradient gel electrophoresis (DGGE), mismatch cleavage detection, heteroduplex analysis, primer extension-based techniques, 5'-nuclease assay-based techniques, using antibodies or sequence-specific DNA-binding proteins as well as methylation-sensitive arbitrarily primed PCR (e.g. Gonzalgo et al. 1997, Cancer Res. 57:594-599), quantitative methylation-specific PCR (Q-MSP; as described e.g. in Current Protocols in Human Genetics, DOI: 10.1002/ 0471142905.hg1006s61), methylation-sensitive restriction analysis (e.g.

Singer-Sam et al. 1990, Nucl. Acids Res. 18:687), methylation-quantification of endonuclease-resistant DNA (Bettstetter *et al.* 2008[42]) or methylation-sensitive sequencing methods (such as e.g. bisulfite DNA sequencing; Frommer et al. 1992, PNAS 89:1827-1831).

Said techniques are well known to the person skilled in the art.

Non-limiting examples for nucleic acid amplification assays and means to perform such include PCR, (including nested PCR, RT-PCR, quantitative real-time detection, PCR extension assays, Nucleic Acid Sequence Base Amplification (NASBA), single-strand confirmation polymorphism (SSCP) PCR, PCR-restriction enzyme fragment length polymorphism (RFLP) analysis), amplification refractory mutation systems (ARMSTM) and amplification refractory mutation system linear extension (ALEXTM) assays. Details of such methods can be found in art, see, for example, Newton et al., Nucleic Acids Res. 17 (1989) 2503-2516; Agrawal (Ed.), "Protocols for Oligonucleotides and Analogs: Synthesis and Properties (Methods in Molecular Biology, 20)", Humana Press, 1993; Haque et al., Diagn. Mol. Pathol. 7 (1998) 248-252; Innis et al. (Ed.), "PCR Applications: Protocols for Functional Genomics", Academic Press, 1999; Chen and Janes (Ed.), "PCR Cloning Protocols: From Molecular Cloning to Genetic", 2nd edition, Humana Press, 2002; Pissard et al., Clin. Chem. 48 (2002) 769-772; Blondal et al., Nucleic Acids Res 31 (2003) e155; Steemers et al., Nature Meth. 3 (2006) 31-33; Kakavas et al., J. Clin. Lab. Anal. 20 (2006) 1-7.

Examples for sequencing assays comprise without limitation approaches of sequence analysis by direct sequencing, fluorescent SSCP in an automated DNA sequencer and Pyrosequencing. These procedures are common in the art, see e.g. Adams et al. (Ed.), "Automated DNA Sequencing and Analysis", Academic Press, 1994; Alphey, "DNA Sequencing: From Experimental Methods to Bioinformatics", Springer Verlag Publishing, 1997; Ramon et al., J. Transl. Med. 1 (2003) 9; Meng et al., J. Clin. Endocrinol. Metab. 90 (2005) 3419-3422.

Examples for hybridization assays comprise without limitation Northern and Southern blot assays, heteroduplex analysis, detection of mutations by sequence specific oligonucleotide hybridization, allele-specific oligonucleotide hybridization on DNA chips, assays based on Illumina's® technology, assays based on the BeadArray® technology, see, for example, Barnes et al., Nucleic Acids Res. 33 (2005) 5914-5923; Fan et al., Biotechniques 39 (2005) 583-588; Shen et al., Mutat. Res.-Fund. Mol. M. 573 (2005) 70-82; Steemers and Gunderson, Pharmacogenomics, 6 (2005) 777-782.

The term "at least five biomarkers", as used herein, refers to five or more biomarkers. Preferably, said term relates to at least six biomarkers, more preferably at least seven biomarkers. The term also relates to at least eight biomarkers or at least nine biomarkers. The term further encompasses exactly five or exactly six or exactly seven or exactly eight or exactly nine biomarkers. In accordance with this method of the invention, the presence or amount of at least five biomarkers selected from the recited list is determined. Also encompassed by the method is that additional biomarkers and/or reference markers are analysed, wherein said additional biomarkers may or may not be selected from the recited list of biomarkers. In other words, whereas the at least five biomarkers have to be chosen from MTAP, PTEN, Bax, Bcl-X, β-Catenin, CD20, Cox-2, CD49d and MLH1, further different biomarkers and/or reference markers may additionally be analysed in accordance with the present invention.

The term "reference marker", as used herein, refers to a marker that is present in melanocytes at substantially constant levels. In other words, the expression level of a reference marker should not differ (apart from deviations caused by the limits of accuracy of established detection methods) between samples of different origin and between benign and malignant samples. Due to the essentially unchanged amounts of such reference markers in different samples, they may be employed to normalise values of biomarker amounts, e.g. by comparison between the expression levels of said non-changing reference marker with the expression level of the biomarker of interest. Often, housekeeping genes are used as reference markers. Examples of reference markers include, without being limiting, GAPDH, RPLPO, PGK1, HSP90AB1, cyclophilin, actin and many more. Further examples are detailed for example in Eisenberg et al. 2003 (Trends Genet 19:362-5) and Velculescu et al. 1999 (Nat Genet 23:387-8.).

In accordance with the present invention, the term "normalising values of biomarker amounts" or "normalising the expression levels" relates to a correction of the measured value. This correction is usually carried out in order to control for bias introduced during the process of sample collection and analysis, which can for example arise due to variations based on different laboratories and/or different machines used, due to differences in staining protocols or differences between extraction protocols that may co-purify inhibitors, and due to different reverse transcription and PCR efficiencies. Importantly, normalisation enables a direct comparison of values obtained from individual patients and/or in different laboratories.

Several strategies for normalisation are known in the art. For example, in case of immunohistochemical methods or quantitative PCR measurement, normalising may be carried out against the expression level(s) of (an) internal reference gene(s)/reference protein(s), which is determined in the same sample; against sample size; against total amount of RNA or genomic DNA or protein; or against an artificially introduced molecule of known amount.

Normalisation is preferably achieved by mathematically dividing the expression values from the marker to be investigated by the expression values of a reference marker. This is particularly preferred if the expression values are given in a linear scale. If the expression values are expressed in a logarithmic scale, normalisation is achieved by subtracting the expression value of the reference marker from the expression value of the marker of interest.

In case of microarray analysis normalisation techniques including, without being limiting, RMA, GCRMA, MAS5, dChip and VSN and others could be used to process raw data to achieve comparability. Details of these methods can be found in Stafford (2008) "Methods in microarray Normalisation" (ISBN-13: 978-1420052787) and Quakenbush Nat. Gene. 2002 (Nat Genet;32 Suppl:496-501)).

In accordance with the present invention, the term "MTAP" refers to S-methyl-5'-thioadenosine phosphorylase, which plays a major role in polyamine metabolism and is important for the salvage of both adenine and methionine. MTAP protein is characterised by the EC number 2.4.2.28. Human MTAP is for example represented by the Entrez Gene ID 4507 and UniProt.ID Q13126 and is shown for example in SEQ ID NOs: 1 and 2. MTAP has been described in the art, for example in Behrmann *et al.,* 2003.

As used herein, the term "PTEN" refers to the phosphatidylinositol-3,4,5-trisphosphate 3-phosphatase and dual-specificity protein phosphatase, which plays a major role as a tumour suppressor. PTEN acts as a dual-specificity protein phosphatase, dephosphorylating tyrosine-, serine- and threonine-phosphorylated proteins. PTEN also acts as a lipid phosphatase, removing the phosphate in the D3 position of the inositol ring from phosphatidylinositol 3,4,5-trisphosphate, phosphatidylinositol 3,4-diphosphate, phosphatidylinositol 3-phosphate and inositol 1,3,4,5-tetrakisphosphate. PTEN protein is characterised by the EC numbers 3.1.3.16, 3.1.3.48 and 3.1.3.67. Human PTEN is for example represented by the Entrez Gene ID 5728 and UniProt ID P60484 and is shown for example in SEQ ID NOs: 3 and 4 PTEN has been described in the art, for example in Zhang and Yu 2010[28].

The term "Bax", as used herein refers to the BCL2-associated X protein, which accelerates programmed cell death by binding to, and antagonising the apoptosis repressor BCL2 or its adenovirus homolog E1B 19k protein. Bax also induces the release of cytochrome c, activation of CASP3, and thereby apoptosis. Human Bax is for example represented by the Entrez Gene ID 581 and UniProt ID Q07812 and is shown for example in SEQ ID NOs: 5 and 6. Bax has been described in the art, for example in Lowe et al. 2004.

As used herein, the term "Bcl-X" refers to the Bcl-2-like protein 1, which is a potent inhibitor of cell death and inhibits the activation of caspases. Human Bcl-X is for example represented by the Entrez Gene ID 598 and UniProt ID Q07817 and is shown for example in SEQ ID NOs: 7 and 8. Bcl-X has been described in the art, for example in Lowe *et al.* 2004.

As used herein, the term "β-Catenin" refers to CTNNB1, which is involved in the regulation of cell adhesion. The majority of β-catenin is localized to the cell membrane and is part of E-cadherin/ catenin adhesion complexes which are proposed to couple cadherins to the actin cytoskeleton. It is also involved in signal transduction through the Wnt pathway and nuclear β-catenin is involved in transcriptional regulation by association with transcription factors of the TCF/LEF family. Human β-Catenin is for example represented by the Entrez Gene ID 1499 and UniProt ID P35222 and is shown for example in SEQ ID NOs: 9 and 10. β-Catenin has been described in the art, for example in Delmas *et al.* 2007.

The term "CD20", as used herein refers to the B-lymphocyte cell-surface antigen B1, also having the gene name MS4A1. CD20 is considered to play an important role in the regulation of B-cell activation and proliferation. Human CD20 is for example represented by the Entrez Gene ID 931 and UniProt ID P11836 and is shown for example in SEQ ID NOs: 11 and 12. CD20 has been described in the art, for example in Zabierowski and Herlyn 2008.

In accordance with the present invention, the term "Cox-2" refers to the prostaglandin-endoperoxide synthase 2, also referred to as PTGS2. Cox-2 mediates the formation of prostaglandins from arachidonate and may also have a role as a major mediator of inflammation and/or a role for prostanoid signaling in activity-dependent plasticity. The Cox-2 protein is characterised by the EC number 1.14.99.1. Human CD20 is for example represented by the Entrez Gene ID 5743 and UniProt ID P35354 and is shown for example in SEQ ID NOs: 13 and 14. Cox-2 has been described in the art, for example in Meyer *et al.* 2009.

As used herein, the term "CD49d" refers to integrin alpha 4, also referred to as ITGA4, antigen CD49D or alpha 4 subunit of VLA-4 receptor. Integrin alpha-4/beta-1 (VLA-4) and alpha-4/beta-7 are receptors for fibronectin. They recognise one or more domains within the alternatively spliced CS-1 and CS-5 regions of fibronectin. They are also receptors for VCAM1. Integrin alpha-4/beta-1 recognises the sequence Q-I-D-S in VCAM1. Integrin alpha-4/beta-7 is also a receptor for MADCAM1. It recognises the sequence L-D-T in MADCAM1. On activated endothelial cells integrin VLA-4 triggers homotypic aggregation for most VLA-4-positive leukocyte cell lines. It may also participate in cytolytic T-cell interactions with target cells. Human CD49d is for example represented by the Entrez Gene ID 3676 and UniProt ID P13612 and is shown for example in SEQ. ID NOs: 15 and 16. CD49d has been described in the art, for example in Kuphal *et al.* 2005.

The term "MLH1", as used herein refers to the DNA mismatch repair protein Mlh1, which heterodimerises with PMS2 to form MutL alpha, a component of the post-replicative DNA mismatch repair system. It also heterodimerises with MLH3 to form MutL y, which plays a role in meiosis and has further been implicated in DNA damage signaling, a process which induces cell cycle arrest and can lead to apoptosis in case of major DNA damages. Human MLH1 is for example represented by the Entrez Gene ID 4292 and UniProt ID P40692 and is shown for example in SEQ ID NOs: 17 and 18. MLH1 has been described in the art, for example in Korabiowska *et al.* 2006.

In accordance with this method of the present invention, the absence or decreased amount of MTAP and/or

β-Catenin is associated with an disadvantageous course of the disease, i.e. the malignant melanoma. In other words, when one or both of these markers are found to be absent or lowered in melanoma cells comprised in a sample obtained from a patient, then this increases the likelihood of said patient to have a recurrence of the disease. The presence of one or both of these markers or elevated levels thereof, however, render it more likely that the patient will not have a recurrence of the disease. Furthermore, in accordance with this method of the invention, the presence or increased amount of PTEN, Bax, Bcl-X, CD20, Cox-2, CD49d and/or MLH1 is associated with a disadvantageous course of disease. Thus, when one or more of these markers are found to be expressed or elevated in melanoma cells comprised in a sample obtained from a patient, then this renders it more likely that the patient will have a recurrence of the disease while the absence of one or more of these markers or decreased levels thereof increase the likelihood of said patient to not have a recurrence of the disease.

In accordance with the present invention, it was found that a set of 9 specific markers enables the characterisation of melanoma cells comprised in a tissue sample of a malignant melanoma, thereby providing an independent prediction model for clinical outcome and individualised, targeted therapy options in patients with malignant melanoma. A prognostic score based on the expression levels of said limited set of markers achieved a higher prognostic accuracy than any other previously used combination of prognostic markers in malignant melanoma and will, therefore, greatly facilitate risk adapted therapy of malignant melanoma patients. In other words, based on a simple and cost-effective analysis of markers selected from this limited set, it is now possible to derive an assessment of the risk of an individual malignant melanoma patient for being a poor prognosis patient (disadvantageous course of disease), i.e. showing a high risk of disease recurrence or being a good prognosis patient (advantageous course of disease), i.e. showing a low risk of disease recurrence. Knowledge of these marker expression profiles additionally allows risk-adapted treatment, which is of benefit for patients with malignant melanoma. For example, a diagnosis of expression of MTAP enables practitioners to identify patients that may benefit from interferon treatment, therefore providing a new basis for a clear targeted use of this expensive immunotherapeutic agent and prevention of a considerable rate of serious side effects.

In particular, using tissue microarrays (TMA), samples of 364 patients with primary malignant melanoma were retrospectively analyzed. A panel of 70 immunohistochemical (IHC) antibodies for proteins involved in cell cycle, apoptosis, DNA mismatch repair, differentiation, proliferation, cell adhesion, signaling and metabolism was investigated. A marker selection procedure based on univariate Cox regression and multiple testing correction was employed to correlate the IHC expression data with the clinical follow-up (overall and recurrence-free survival). The model was thoroughly evaluated with two different cross validation experiments, a permutation test and a multivariate Cox regression analysis. The predictive power of the identified marker signature was validated on a second independent external test cohort (n=225), thus rendering the total of patients 589. This study adheres to the reporting recommendations for tumour marker prognostic studies, i.e. it implements the REMARK guidelines (J Natl Cancer Institute 2005; 97:1180-4).

The prognostic power of these 70 markers was assessed, yielding the 11 markers MTAP, PTEN, Bax, Bcl-X, β-Catenin, CD20, Cox-2, CD49d, MLH1, TOP2A and Frizzled 7, which were significantly associated with overall survival. While all eleven markers are significantly associated with overall survival, it was possible to further reduce the number of markers required for a prognostic assessment to nine (MTAP, PTEN, Bax, Bcl-X, β-Catenin, CD20, Cox-2, CD49d and MLH1) based on the Cox regression coefficients and multiple testing correction with FDR. These nine markers were correlated with death from any cause. Two of these markers were protective markers (associated with a hazard ratio of less than 1.00) and seven were risk markers (associated with a hazard ratio of more than 1.00) (Fig. 4A).

For the sake of clinical feasibility and cost saving, any marker set suitable for routine clinical assessment should comprise a limited number of markers. It was therefore the aim to provide a maximum of prognostic and therapeutically relevant information by as few markers as possible combined in a clear signature. Accordingly, it was shown that a further reduction of the nine-marker signature still leads to prognostic and therapeutically relevant information. As a proof of principle, this was carried out using a seven-marker signature (Bax, Bcl-X, β-Catenin, CD20, COX-2, MTAP, PTEN), which was found to also provide prognostic and therapeutically relevant information. Immunohistochemically stained TMA Specimens illustrating the Seven-Marker Signature for one Patient with High-Risk and one Patient with Low-Risk Melanoma is shown in Table 3.

| A: Protein | B: Localization | C: Low-risk signature melanoma (no. 140) | D: High-risk signature melanoma (no. 137) |
|---|---|---|---|
| **Bax:**<br>Bcl-2 family protein, proapoptotic | Cytoplasmic | 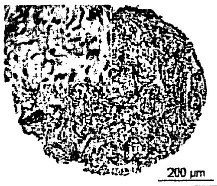 | 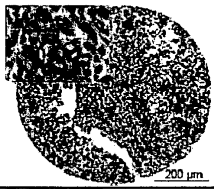 |
| **β-Catenin:**<br>Key downstream effector in Wnt signaling pathway, implicated in two major biological processes: embryonic development and tumorigenesis | Cytoplasmic and nuclear | 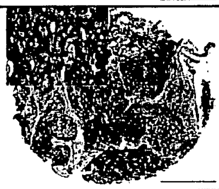 | 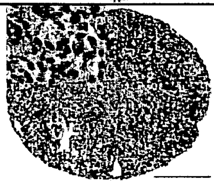 |
| **CD 20:**<br>Pan-B-cell marker, stem cell marker candidate | Cell membrane and cytoplasmic | 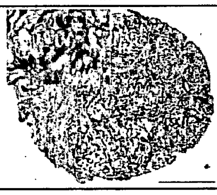 | 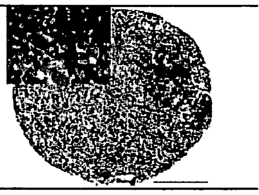 |

**Table 3. Immunohistochemically stained TMA Specimens illustrating the Seven-Marker Signature for one Patient with High-Risk and one Patient with Low-Risk Melanoma.** The low-risk melanoma (Column C) showed a strong cytoplasmic staining for β-Catenin and MTAP, respectively. Immunoreactivity of these two protective markers was not found in the high-risk melanoma (Column D). In contrast, the high-risk melanoma demonstrated a moderate to strong cytoplasmic staining for Bax, CD20, Bcl-X, PTEN and COX-2.

| | | | |
|---|---|---|---|
| **Bcl-X:** Bcl-2 family protein, antiapoptotic | Cytoplasmic | 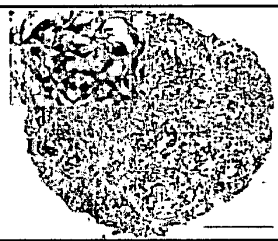 | 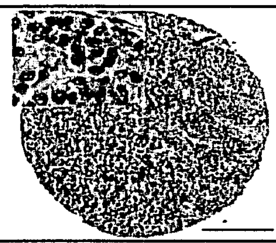 |
| **MTAP:** Constitutive enzyme of the polyamine metabolism; modulator of interferon-dependent signaling | Cytoplasmic | 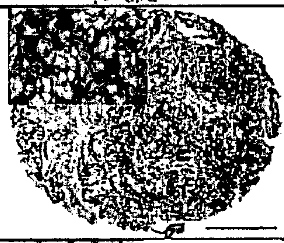 | 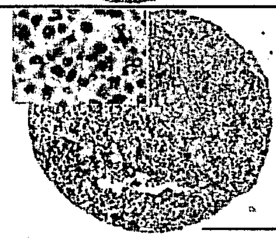 |
| **PTEN:** Tumor suppressor implicated in a variety of human cancers; major negative regulator of the PI3K/Akt signaling pathway | Cytoplasmic and nuclear | 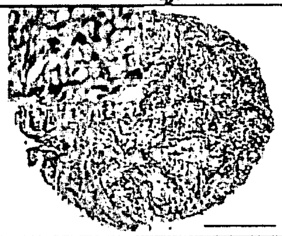 | 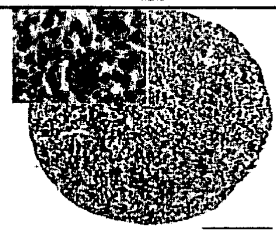 |
| **Cox-2:** Cyclooxygenase for biosynthesis of prostaglandins under inflammatory conditions; overexpressed in a variety of tumors | Cytoplasmic | 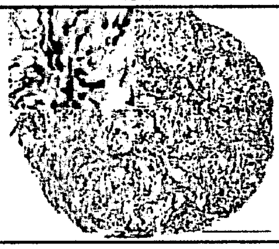 | 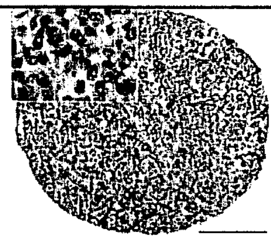 |

The data obtained for the seven-marker signature can reasonably be extended to marker sets comprising only five marker or six markers, due to the high coincidence and correlation of some of the markers, such as e.g. Bax and Cox-2 expression in the melanoma samples analysed (see figure 5). In other words, when a highly correlated first marker is comprised in the set of markers analysed, then the information provided by a second marker correlated to said first marker might be limited and, consequently, such markers can be omitted.

With a total of 24,674 punch specimens of primary malignant melanoma analyzed by IHC, this TMA study is more comprehensive than previous studies described in the art (to the inventors best knowledge). The detected signature might serve as a prognostic tool enabling physicians to selectively choose, at the time of diagnosis and initial surgery, the subset of high recurrence risk Stage I-II patients for adjuvant therapy. Selective treatment of those patients that are more likely to develop distant metastatic disease could potentially lower the burden of untreatable metastatic melanoma and promote the therapeutic management of malignant melanoma.

Means and methods to derive a risk estimate are well known in the art and, based on the information provided in accordance with the present invention, the skilled person is able to derive a risk estimation.

One exemplary method is based on the following prognostic score calculation:

$$\text{score}(x) = \left(\sum_{i=1}^{D}(\beta_i x_i)\alpha_i\right) \Big/ \left(\sum_{i=1}^{D}\alpha_i\right), \quad \alpha_i = \begin{cases} 1, & \text{if } x_i \text{ exists} \\ 0, & \text{if } x_i \text{ is missing} \end{cases}$$

wherein D is the number of markers analysed, $\beta_i$ are the coefficients of the univariate Cox model, e.g. -0.621 for MTAP, -0.34 for β-Catenin, 0.547 for CD20, 0.391 for Bcl-X, 0.297 for COX-2, 0.272 for PTEN, 0.407 for CD49d, 0.254 for MLH1

and 0.441 for Bax as shown in Figure 3F and $\boldsymbol{x}_i$ is the value determined for the individual marker i in patient $\boldsymbol{x}$. $\alpha_i$ corrects for markers not present in said patient or not evaluated. Based on this score calculation, a patient is diagnosed to have an advantageous course of disease when the score is below a reference score and to have a disadvantageous course of disease when the score is above said reference score.

In accordance with the present invention, the term "reference score" relates to a cut-off point above or below which a diagnosis of the course of disease can be made, i.e. as advantageous or disadvantageous course. Said reference score may for example be a mean, i.e. average, score determined based on a malignant melanoma patient cohort comprising patients with advantageous course of disease as well as disadvantageous course of disease without any bias towards one of these groups. Such un-biased patient cohorts will be available to hospitals and can be analysed to derive the reference score. Preferably, a prognostic score significantly below the reference score is indicative of an advantageous course of disease and a prognostic score significantly above the reference score is indicative of a disadvantageous course of disease.

For example, based on the above score calculation and on a scale for $x_i$ ranging from -0.5 to +0.5, a patient is diagnosed to have an advantageous course of disease when the score based on the seven-marker signature (Bax, Bcl-X, β-Catenin, CD20, COX-2, MTAP, PTEN) is below 0.1346739 and to have a disadvantageous course of disease when the score is above 0.1346739, as shown in Figure 1A.

Alternatively, the skilled person may initially determine in a sufficiently large patient group, such as for example at least 10, more preferably at least 75 and most preferably at least 100 patients, the presence and amount of the markers MTAP, PTEN, Bax, Bcl-X, β-Catenin, CD20, Cox-2, CD49d and MLH1. Preferably, the patient group is a representative group of malignant melanoma patients predicted by conventional methods to be poor prognosis or good prognosis patients. The data obtained from this group may then be correlated with disease progression, as detailed in the examples below. For example, after measurement of at least 10, more preferably at least 75 and most preferably at least 100 patients in a heterogeneous prognostic group and determination of the above mentioned marker values, correlation of the follow up data with the marker values using the univariate Cox model results in the derivation of specific coefficients. The calculated scores (sum of the marker values multiplied with the coefficients) can be sorted due to their magnitude. The cut offs should be selected due to clinical relevance. For example the magnitudes may be split at the 50th percentile, whereas a lower score is indicative for good prognosis and a higher score is indicative for poor prognosis. Alternatively, the patients may be grouped into more than two groups, depending on the clinical information required. It will be understood by the skilled person that such an initial determination of the presence and amount of markers does not need to be carried out every time but may instead be carried out when first establishing the method of predicting the course of malignant melanoma in patients. The data obtained by such initial experiments may also be stored in databases accessible to other researchers, thus obviating the need for these researchers to establish such initial data.

Alternatively, the risk of an individual patient may also be evaluated based on a simplified score calculation wherein the coefficients from the univariate Cox proportional hazard models are used in a weighted linear combination to predict the risk score for each patient as shown in Figure 3F. Such a simplified score calculation would be: -0.6*MTAP - 0.3*β-Catenin + 0.5*CD20 + + 0.4*BCLX + 0.4*Bax + 0.3*PTEN + 0.3*COX2. In other words, the score obtained for MTAP is multiplied with -0.6, the score obtained for β-Catenin is multiplied with -0.3, the score obtained for CD20 is multiplied with 0.5 and so on. The sum of all these weighted scores provides a prognostic risk score for each patient. For example and as shown in the appended examples, when a scoring system from 0 to 4 was employed, a risk score below 0.135 was found to be indicative of a good prognosis for said patient while a risk score above 0.135 was found to be indicative of a poor prognosis for said patient.

In a preferred embodiment of the method of the invention, the at least five biomarkers include PTEN and/or MTAP.

Thus, the set of biomarkers employed in accordance with this preferred embodiment comprises at least PTEN or MTAP, more preferably it comprises PTEN and MTAP.

MTAP expression was shown in accordance with the present invention to be the strongest marker for a favourable disease outcome (coefficient of -0.621) and is, furthermore, of therapeutic relevance. In the adjuvant treatment of malignant melanoma, interferon alpha is currently the only clinically accepted therapeutic agent providing a significant (recurrence-free) survival benefit for a small but distinct percentage of patients.[34] On account of the serious side effects and the high costs of the therapy, it is advantageous to determine those patients with a realistic chance to benefit from interferon treatment. It has recently been shown that there is a clear association between MTAP expression in the primary melanoma and melanoma progression and, even more importantly, response to interferon treatment.[13,28,35] Biomarkers like MTAP might therefore enable practitioners to assess which patients may benefit from interferon treatment and could thus provide a new basis for a clear targeted use of this expensive immunotherapeutic agent and prevent the serious side effects associated with the treatment with interferon.

The tumor suppressor phosphatase and tensin homolog PTEN was identified as another signature protein. PTEN counteracts one of the most critical cancer promoting pathways, [28] the phosphatidylinositol 3-kinase (PI3K)/Akt signaling pathway. An established consequence of PTEN inactivation is the constitutive aberrant activation of the PI3K-signaling pathway that drives uncontrolled cell growth, proliferation, and survival (Zhang and Yu, 2010[28]). Thus, including PTEN in the analysis not only provides a diagnostic tool, but may also be of predictive relevance. In general, cancer cells contain multiple genetic and epigenetic abnormalities. Despite this complexity, their growth and survival can often be impaired by the inactivation of a single oncogene. This phenomenon, called "oncogene addiction," provides a rationale for molecular targeted therapy. The efficacy of this strategy requires novel methods, including integrative genomics and systems biology, to identify the state of oncogene addiction (i.e., the "Achilles heel") in specific cancers. Combination therapy may also be required to prevent the escape of cancers from a given state of oncogene addiction (Weinstein and Joe 2008[47]). Thus, including PTEN in the analysis not only provides a diagnostic tool, but also is also of therapeutic relevance.

In another preferred embodiment of the method of the invention, at least seven biomarkers are determined.

In a more preferred embodiment, the at least 7 biomarkers are MTAP, PTEN, Bax, Bcl-X, β-Catenin, CD20 and Cox-2.

In accordance with the present invention, it has been shown that this set of seven biomarkers is closely associated with the prognosis of patients with malignant melanoma and may therefore serve as an independent predictor for overall and recurrence-free survival in patients with malignant melanoma.

Among the 362 patients of the primary cohort, patients with said high-risk seven-marker signature had a shorter median overall survival than the patients with a low-risk seven-marker signature (88 months versus not reached) and the difference between the two patient groups was highly significant (p=0.0000000042) (Fig. 1D). The high-risk seven-marker signature was associated with a median recurrence-free survival of 33 months, whereas the low-risk seven-marker signature was associated with a median recurrence-free survival of 88 months (LRT p=0.00034) (Fig. 1 E). According to multivariate Cox regression analysis, the seven-marker risk score, tumour thickness, sex, and age were significantly associated with death from any cause among the 356 patients (6 observations were deleted due to missing values) (Table 1). Furthermore, a subgroup analysis of 253 patients with a tumour depth of ≤ 2 mm revealed that those 148 patients with a high-risk marker signature had a significant (p=0.0053) shorter overall survival (Fig. 3A) and recurrence-free survival (p=0.008) than the 105 patients with a low-risk marker signature (Fig. 3B).

**Table 1. Clinical Characteristics of the Primary Cohort of Patients with MM (TMA 1).** Comparing high-risk patients (first column) with low-risk patients (second column) based on their seven-marker risk score shows significant difference in tumour thickness (p<0.001) and no difference in sex (p=1) and age (p=0.263). Furthermore, hazard ratios and p-values are reported for a multivariate Cox regression model comprising all listed variables. Regarding overall survival the seven-marker risk score is statistically significant (p<0.001) independent of sex, age and tumour thickness. Continuous variables are reported with mean and standard deviation and categorical variables are listed with number of counts and percentages.

| | **High risk** | **Low risk** | **p-Value:** | **Multivariate Cox Regression Analysis** | |
|---|---|---|---|---|---|
| | **(N=181)** | **(N=181)** | **high vs. low risk** | **Hazard ratio (95% CI)** | **p-Value** |
| **7-Marker risk score** | 0.267 ± 0.092 | 0.0017 ± 0.12 | <<0.0001 #1 | 13.54 (4.27-42.97) | 0.0000098 *** |
| **Age - yr** | 59.5±15.0 | 57.7 ±14.9 | 0.263 #1 | 1.03 (1.02-1.05) | 0.000002 *** |
| **Sex - no. of patients (%)** | | | | | |
| Male | 105 (58) | 89 (49.2) | 1 #2 | 1.98 (1.36-2.89) | 0.00034 *** |
| Female | 76 (42) | 92 (50.8) | | | |
| **Tumor thickness - mm** | 2.52 ± 2.38 | 1.40 ± 2.21 | 0.00000646 #1 | 1.17 (1.12-1.23) | 0.00000000023 *** |

#1 Welch two sample t-test
#2 Fisher's exact test
*** p-Value < 0.001

Notably, the predictive power of the signature was carefully validated and confirmed on a secondary, independent external test cohort including melanoma samples of 225 patients from a different hospital. In this external test cohort it was confirmed that patients with a high-risk marker signature had a significantly ($p=0.000017$) different survival expectance and shorter median overall survival compared to patients with a low-risk signature (95 months versus not reached) (Fig. 2A). According to multivariate Cox regression including sex, age, tumour thickness, ulceration and nodal status, the seven-marker signature was significantly associated with overall survival ($p=0.0000098$, Table 1). Additionally, the recurrence-free survival differed significantly between the two risk groups ($p=0.004$; Fig. 2B).

Thus, the seven-marker signature (Bax, Bcl-X, β-Catenin, CD20, COX-2, MTAP, PTEN) is closely associated with the prognosis of patients with malignant melanoma, as the signature was found to be an independent predictor for overall and recurrence-free survival in patients with malignant melanoma. The seven-marker signature could also predict high recurrence risk patients with localized primary malignant melanoma stage pT1-2 (tumour thickness ≤2.00 mm) and worse prognosis. In particular, three of these markers (CD20, COX-2, MTAP) were shown to offer direct therapeutic implications.

In a further preferred embodiment of the method of the invention, at least nine biomarkers are determined.

By increasing the number of biomarkers analysed, the sensitivity and specificity of the analysis can be increased.

In accordance with this embodiment, all nine biomarkers MTAP, PTEN, Bax, Bcl-X, β-Catenin, CD20, Cox-2, CD49d and MLH1 are determined.

It was found in accordance with the present invention that this set of nine biomarkers is closely associated with the prognosis of patients with malignant melanoma and may therefore serve as an independent predictor for overall and recurrence-free survival in patients with malignant melanoma.

Table 1 summarises the characteristics of 362 patients in the study. Among these 362 patients of the primary cohort, tumours associated with high risk scores also expressed risk markers, whereas tumours associated with low risk scores expressed protective markers (Fig. 1A). Patients with a high-risk nine-marker signature had a lower median overall survival than patients with a low-risk nine-marker signature (90 months versus not reached) (Fig. 1 B). Patients with tumours with a high-risk marker signature were associated with a lower median recurrence-free survival than patients with tumours with a low-risk gene signature (36 months versus 88) (Fig. 1C).

The present invention further relates to a method of preparing a tailored pharmaceutical composition for a patient having a malignant melanoma, the method comprising (i) determining in melanoma cells comprised in a sample obtained from said malignant melanoma the presence or amount of at least five biomarkers selected from the group comprising or consisting of MTAP, PTEN, Bax, Bcl-X, β-Catenin, CD20, Cox-2, CD49d and MLH1, wherein the absence or decreased amount of β-Catenin and MTAP and/or the presence or increased amount of PTEN, Bax, Bcl-X, CD20, Cox-2, CD49d and MLH1 is associated with a disadvantageous course of disease; (ii) deriving a treatment regimen for the individual patient based on the presence or amount of markers determined in step (i); and (iii) providing at least one pharmaceutical compound based on the treatment regimen derived in step (ii).

In accordance with this embodiment, the course of disease is determined for a patient having a malignant melanoma and, additionally, the data obtained by determining the presence or amount of at least five biomarkers selected from the group comprising or consisting of MTAP, PTEN, Bax, Bcl-X, β-Catenin, CD20, Cox-2, CD49d and MLH1 is employed to derive a treatment regimen for the individual patient, i.e. to prepare a tailored pharmaceutical composition.

The term "pharmaceutical composition", as used herein, relates to a composition for administration to a patient, preferably a human patient. The pharmaceutical composition of the invention comprises a therapeutic compound, such as for example a compound selected from the compounds recited below, alone or in combination. It may, optionally, comprise further molecules capable of altering the characteristics of these compounds thereby, for example, stabilizing, modulating and/or activating their function. The composition may e.g. be in solid or liquid form and may be, *inter alia,* in the form of (a) powder(s), (a) tablet(s), (a) solution(s) or (an) aerosol(s). The pharmaceutical composition of the present invention may, optionally and additionally, comprise a pharmaceutically acceptable carrier. By "pharmaceutically acceptable carrier" is meant a non-toxic solid, semisolid or liquid filler, diluent, encapsulating material or formulation auxiliary of any type. Examples of suitable pharmaceutically acceptable carriers are well known in the art and include phosphate buffered saline solutions, water, emulsions, such as oil/water emulsions, various types of wetting agents, sterile solutions, organic solvents including DMSO etc.. Compositions comprising such carriers can be formulated by well known conventional methods.

These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. The skilled person knows that the effective amount of a pharmaceutical composition administered to an individual will, inter alia, depend on the nature of the compound. For example, if said compound is a polypeptide, the total pharmaceutically effective amount of pharmaceutical composition administered parenterally per dose will be in the range of about 1 μg protein/kg/day to 10 mg protein/kg/day of

patient body weight, although, as noted above, this will be subject to therapeutic discretion. More preferably, this dose is at least 0.01 mg protein/kg/day, and most preferably for humans between about 0.01 and 1 mg protein/kg/day. Furthermore, if for example said compound is an iRNA agent, such as an siRNA, the total pharmaceutically effective amount of pharmaceutical composition administered will typically be less than about 75 mg per kg of body weight, such as for example less than about 70, 60, 50, 40, 30, 20, 10, 5, 2, 1, 0.5, 0.1, 0.05, 0.01, 0.005, 0.001, or 0.0005 mg per kg of body weight. More preferably, the amount will be less than 2000 nmol of iRNA agent (e.g., about 4.4 x 1,016 copies) per kg of body weight, such as for example less than 1,500, 750, 300, 150, 75, 15, 7.5, 1.5, 0.75, 0.15, 0.075, 0.015, 0.0075, 0.0015, 0.00075 or 0.00015 nmol of iRNA agent per kg of body weight. The length of treatment needed to observe changes and the interval following treatment for responses to occur vary depending on the desired effect. The particular amounts may be determined by conventional tests which are well known to the person skilled in the art.

Pharmaceutical compositions of the invention may for example be administered orally, rectally, parenterally, intracisternally, intraperitoneally, topically (as by powders, ointments, drops or transdermal patch), bucally, or as a nasal spray. The term "parenteral" as used herein refers to modes of administration, which include intravenous, intramuscular, intrasternal, subcutaneous and intraarticular injection and infusion.

The therapeutic compound, in accordance with the present invention, may for example be selected from the group consisting of antibodies, aptamers, siRNAs, shRNAs, miRNAs, ribozymes, antisense nucleic acid molecules, and a small molecule. Therapeutic compounds further include but are not limited to, for example, peptides such as soluble peptides, including Ig-tailed fusion peptides and members of random peptide libraries (see, e.g., Lam et al. (1991) Nature 354: 82-84; Houghten et al. (1991) Nature 354: 84-86) and combinatorial chemistry-derived molecular libraries made of D-and/or L-configuration amino acids or phosphopeptides (e.g., members of random and partially degenerate, directed phosphopeptide libraries, see, e.g., Songyang et al. (1993) Cell 72: 767-778).

The term "antibody" as used in accordance with the present invention comprises polyclonal and monoclonal antibodies, as well as derivatives or fragments thereof, which still retain the binding specificity. Antibody fragments or derivatives comprise, inter alia, Fab or Fab' fragments as well as Fd, $F(ab')_2$, Fv or scFv fragments; see, for example Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also includes embodiments such as chimeric (human constant domain, non-human variable domain), single chain and humanised (human antibody with the exception of non-human CDRs) antibodies. The term antibodies also encompasses peptidomimetics.

Various techniques for the production of antibodies are well known in the art and described, e.g. in Harlow and Lane (1988) and (1999), loc. cit.. Further, techniques described for the production of single chain antibodies (see, inter alia, US Patent 4,946,778) can be adapted to produce single chain antibodies specific for the target of this invention. Also, transgenic animals or plants (see, e.g., US patent 6,080,560) may be used to express (humanized) antibodies specific for the target of this invention. Most preferably, the antibody is a monoclonal antibody, such as a human or humanized antibody. For the preparation of monoclonal antibodies, any technique which provides antibodies produced by continuous cell line cultures can be used. Examples for such techniques are described, e.g. in Harlow and Lane (1988) and (1999), loc. cit. and include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of the biomarkers (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, inter alia, viruses or plasmid vectors.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

More specifically, aptamers can be classified as nucleic acid aptamers, such as DNA or RNA aptamers, or peptide aptamers. Whereas the former normally consist of (usually short) strands of oligonucleotides, the latter preferably consist of a short variable peptide domain, attached at both ends to a protein scaffold.

Nucleic acid aptamers are nucleic acid species that, as a rule, have been engineered through repeated rounds of *in vitro* selection or equivalently, SELEX (systematic evolution of ligands by exponential enrichment) to bind to various molecular targets such as small molecules, proteins, nucleic acids, and even cells, tissues and organisms.

Peptide aptamers usually are peptides or proteins that are designed to interfere with other protein interactions inside cells. They typically consist of a variable peptide loop attached at both ends to a protein scaffold. This double

structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site, which is a -Cys-Gly-Pro-Cys- loop in the wild protein, the two cysteins lateral chains being able to form a disulfide bridge. Peptide aptamer selection can be made using different systems, but the most widely used is currently the yeast two-hybrid system.

Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as *in vivo* diagnostic imaging. Several modifications, such as 2'-fluorine-substituted pyrimidines, polyethylene glycol (PEG) linkage, fusion to albumin or other half life extending proteins etc. are available to scientists such that the half-life of aptamers can be increased for several days or even weeks.

The term "peptide" as used herein describes a group of molecules consisting of up to 30 amino acids, whereas the term "protein" as used herein describes a group of molecules consisting of more than 30 amino acids. Peptides and proteins may further form dimers, trimers and higher oligomers, i.e. consisting of more than one molecule which may be identical or non-identical. The corresponding higher order structures are, consequently, termed homo- or heterodimers, homo- or heterotrimers etc.. The terms "peptide" and "protein" (wherein "protein" is interchangeably used with "polypeptide") also refer to naturally modified peptides/proteins wherein the modification is effected e.g. by glycosylation, acetylation, phosphorylation and the like. Such modifications are well-known in the art.

Antibodies or aptamers may further be used as a targeting moiety to deliver therapeutically active compounds, such as known anti-cancer drugs (e.g. chemotherapeutic agents such as Dacarbazine, Fotemustine or Cisplatin), to the malignant melanoma cells of a patient.

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene of which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have 2-nt 3' overhangs on either end. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery *in vivo* through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics).

A short hairpin RNA (shRNA) is a sequence of RNA that makes a tight hairpin turn that can be used to typically silence gene expression via RNA interference. shRNA can for example use a vector introduced into cells, in which case the U6 promoter is utilized to ensure that the shRNA is always expressed. This vector is usually passed on to daughter cells, allowing the gene silencing to be inherited. The shRNA hairpin structure is cleaved by the cellular machinery into

siRNA, which is then bound to the RNA-induced silencing complex (RISC). This complex binds to and cleaves mRNAs which match the siRNA that is bound to it.

Preferably, si/shRNAs to be used in the present invention are chemically synthesized using conventional methods that, for example, appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules which, as endogenous RNA molecules, regulate gene expression. Binding to a complementary mRNA transcript triggers the degradation of said mRNA transcript through a process similar to RNA interference. Accordingly, miRNA may be employed as an inhibitor of the of the biomarkers in accordance with the present invention.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome. Non-limiting examples of well-characterized small self-cleaving RNAs are the hammerhead, hairpin, hepatitis delta virus, and *in* vitro-selected lead-dependent ribozymes, whereas the group I intron is an example for larger ribozymes. The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity *in vitro* and in some cases also *in vivo.* The best results are usually obtained with short ribozymes and target sequences.

A recent development, also useful in accordance with the present invention, is the combination of an aptamer recognizing a small compound with a hammerhead ribozyme. The conformational change induced in the aptamer upon binding the target molecule is supposed to regulate the catalytic function of the ribozyme.

The term "antisense nucleic acid molecule" is known in the art and refers to a nucleic acid which is complementary to a target nucleic acid. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

A "small molecule" according to the present invention may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 amu, or less than about 1000 amu such as less than about 500 amu, and even more preferably less than about 250 amu. The size of a small molecule can be determined by methods well-known in the art, e.g., mass spectrometry. The small molecules may be designed, for example, based on the crystal structure of the target molecule, where sites presumably responsible for the biological activity, can be identified and verified in *in vivo* assays such as *in vivo* high-throughput screening (HTS) assays. Such small molecules may be particularly suitable to inhibit protein-protein-interaction by blocking specific bindings sites of the target molecule. Suitable small molecules currently employed in the treatment of cancer include, without being limiting, small molecule inhibitors for inhibiting the Bcl-2 apoptosis inhibitor family (Azmi, and Mohammad, 2009).

Also encompassed herein are modified versions of these therapeutic compounds.

The term "modified versions of these therapeutic compounds" in accordance with the present invention refers to versions of the compounds that are modified to achieve i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, color, taste, odor, stability, state), and/or viii) improved general specificity, organ/tissue specificity, and/or ix) optimised

application form and route by (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (k) conversion of alkyl substituents to cyclic analogues, or (l) derivatisation of hydroxyl groups to ketales, acetales, or (m) N-acetylation to amides, phenylcarbamates, or (n) synthesis of Mannich bases, imines, or (o) transformation of ketones or aldehydes to Schiffs bases, oximes, acetales, ketales, enolesters, oxazolidines, thiazolidines; or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

The term "tailored pharmaceutical composition" in accordance with the present invention, relates to a pharmaceutical composition that is adjusted to the individual needs of a particular patient. In other words, a tailored pharmaceutical composition is a patient-specific medication. For the practitioner, the assessment of the markers in accordance with the invention provides a helpful tool to answer the crucial question "whom to treat, and how to treat", especially in the adjuvant setting after surgical excision of early-stage and localized primary malignant melanoma (Stage I to IIa).

Several of the markers employed in accordance with the present invention have previously been shown to be suitable targets or indicators in the treatment of cancers. Thus, therapeutic compounds targeting said markers or targeting pathways associated with said markers are already available in the art. For example, CD20, COX-2 and MTAP are three markers of the present invention that offer direct therapeutic implications, since the corresponding drugs have been approved by the FDA.

The CD20-antigen is known to be an effective therapeutic target in the treatment of patients with CD20-positive B-Cell-Non-Hodgkin-Lymphomas. For example, the monoclonal chimeric antibody Rituximab has been described for immunotherapy.[29] The antibody binds specifically with CD20-antigen presented on the surface of normal and malignant B-lymphocytes and causes a cell- and complement-mediated cytotoxic death of these cells. According to a small phase II pilot trial in stage IV melanoma patients recently presented at the ASCO meeting (2010), the anti-CD20-antibody Rituximab may potentially be suitable for immunotargeting of CD20-positive melanoma subpopulations. Consequently, the presence of CD20 in melanoma cells of a patient as determined with the method of the present invention is indicative for a therapeutic treatment of said patient with compounds targeting CD20, such as for example Rituximab. Further CD20 inhibitors are for example, the yttrium-[90]-labeled 2138 murine antibody designated Y2B8 (U.S. Pat. No. 5,736,137); murine IgG2a 131 optionally labeled with 131 I to generate the 131 I-B1 antibody (BEXXAR®) (U.S. Pat. No. 5,595,721); murine monoclonal antibody 1F5 (Press et al. Blood 69(2): 584-591 (1987)); chimeric 2H7 antibody (U.S. Pat. No. 5,677,180); and monoclonal antibodies L27, G28-2, 93-1 133, B-Cl or NU-B2 available from the International Leukocyte Typing Workshop (Valentine et al., In: Leukocyte Typing III (McMichael, Ed., p. 440, Oxford University Press (1987)).

Cyclooxygenase 2 represents another promising therapeutic target. Cyclooxygenases (COXs) catalyze the first rate-limiting step in the conversion of arachidonic acid to prostaglandins. In contrast to COX-1, the COX-2 isoenzyme is not detectable in most normal tissues and rapidly induced by various stimuli such as inflammatory reactions.[30] It is also expressed in various tumour types and levels of COX-2 expression have been shown to correlate with invasiveness and prognosis in some tumour entities, including epithelial and melanocytic skin cancer.[14,31] Epidemiological studies showed that prolonged COX-2 inhibition through acetylsalicylic acid or other nonsteroidal anti-inflammatory drugs (NSAIDs) might offer some protection against colon cancer and some other malignancies.[32] So far the benefit of COX-2-inhibitors has not been studied in the adjuvant treatment of early-stage melanomas to prevent metastasis. In the second-line treatment of advanced metastatic melanoma disease, however, a survival benefit was shown for targeted combined therapy using COX-2-inhibitors and PPARG-agonists for anti-inflammatory treatment together with low-dose metronomic chemotherapy.[33] Considering this observation and the fact that melanoma patients with COX-2-positive primary tumours bear a significantly higher risk of tumour recurrence,[14] it is concluded that the presence of COX-2 in melanoma cells of a patient as determined with the method of the present invention is indicative for a therapeutic treatment of said patient with compounds targeting COX-2, such as for example COX-2 inhibitors including, but not limited, Celecoxib, Etoricoxib or Parecoxib for primary adjuvant treatment of these patients.

Furthermore, in the adjuvant treatment of malignant melanoma, interferon alpha is currently the only clinically accepted therapeutic agent providing a significant (recurrence-free) survival benefit for a small but distinct percentage of patients.[34] On account of the serious side effects and the high costs of the therapy, it is advantageous to determine those patients with a realistic chance to benefit from interferon treatment. It has recently been shown that there is a clear association between MTAP expression in the primary melanoma and melanoma progression and, even more importantly, response to interferon treatment.[13,28,35] Biomarkers like MTAP might therefore enable practitioners to assess which

patients may benefit from interferon treatment and could thus provide a new basis for a clear targeted use of this expensive immunotherapeutic agent and prevent the serious side effects associated with the treatment with interferon.

Also Bcl-X has been targeted in preclinical tests and several targeting agents are in the clinical testing phase by now:[36] Bcl-X is related to the anti-apoptotic Bcl-2 protein family. Over-expression of these anti-apoptotic proteins protects cancer cells against death signals of apoptosis. Interestingly, tumours expressing high levels of Bcl-2 or Bcl-X are often found to be resistant to chemotherapeutic agents or radiation therapy.[37] In recent years, there has been an exponential growth in the identification and synthesis of non-peptidic cell permeable "small molecule inhibitors" (SMIs) against anti-apoptotic proteins like Bcl-2 or Bcl-X (see e.g. Azmi and Mohammad 2009). SMIs inhibit distinct protein-protein interactions by blocking specific binding sites of the target molecule, thus supporting the apoptotic machinery.[36] Inhibition of Bcl-X may exert a synergistic effect with conventional treatments like chemo- or radiation therapy and with respect to melanoma therapy, this effect would be a decisive therapeutic success.

For PTEN oncogenic pathway addiction, as described herein above, has been described in detail in the literature, for example in Weinstein and Joe 2008[47], Zhang and Yu 2010[28], Mirmohammadsadegh *et al.* 2006[43], Lahtz *et al.* 2010[44] or Zhou *et al.* 2000[45].

In accordance with the present invention, the marker signature represents a highly promising clinical tool to predict a patient's prognosis. Most importantly, the marker signature is expected to improve the clinical management and adjuvant treatment of early-stage malignant melanoma with a high risk of recurrence. In the treatment of advanced metastatic melanoma, novel immune-based anti-tumour therapies targeting signal transduction pathways or tumour immunity barriers by monoclonal antibodies like selective BRAF inhibitors[38] or anti-cytotoxic T-lymphocyte antigen 4 (CTLA-4) antibodies[39] have already entered clinical studies. This promising therapeutic option in the treatment of advanced metastatic malignant melanoma development, together with the set of molecular markers identified in accordance with the present invention is expected to provide new risk-oriented indications for an individualized targeted anti-tumour therapy of malignant melanoma.

In a preferred embodiment of the method of preparing a tailored pharmaceutical composition, the at least five biomarkers include CD20, Cox-2 and/or MTAP.

Thus, the set of biomarkers employed in accordance with this preferred embodiment comprises at least CD20, or Cox-2, or MTAP, or CD20 and Cox-2, or CD20 and MTAP, or Cox-2 and MTAP, or CD20 and Cox-2 and MTAP.

In another preferred embodiment of the method of preparing a tailored pharmaceutical composition, at least seven biomarkers are determined.

In a more preferred embodiment of the method of preparing a tailored pharmaceutical composition, the at least seven biomarkers are MTAP, PTEN, Bax, Bcl-X, β-Catenin, CD20 and Cox-2.

In a further preferred embodiment of the method of preparing a tailored pharmaceutical composition, at least nine biomarkers are determined.

In accordance with this embodiment of the method of preparing a tailored pharmaceutical composition, all nine biomarkers MTAP, PTEN, Bax, Bcl-X, β-Catenin, CD20, Cox-2, CD49d and MLH1 are determined.

In a further more preferred embodiment of the method(s) of the invention, the sample is obtained from the primary tumour, a lymph node or a metastasis.

The term "primary tumour" in accordance with the present invention refers to a malignant tumour (also referred to herein as a cancer) at a first site, i.e. in a first organ or part of the body. In general, when the area of cancer cells at the originating site become clinically detectable, it is referred to as a primary tumour. In the present case of malignant melanoma, said primary tumour is a malignant tumour of melanocytes, which are present in the skin (i.e. the primary tumour is skin cancer) but also in the mucous membrane and the eye. Some cancer cells also acquire the ability to penetrate and infiltrate surrounding normal tissues in the local area, forming a new tumour. The newly formed "daughter" tumour in the adjacent site within the tissue is called a local metastasis while the formation of a new tumour in a non-adjacent site is called a distant metastasis.

In accordance with the present invention, the term "lymph node" refers a small organ of the immune system that is important in the proper functioning of the immune system, as it acts as a filter or trap for foreign particles. Lymph nodes are widely distributed throughout the body including the armpit and stomach/gut and linked by lymphatic vessels. Lymph nodes become inflamed or enlarged in various conditions, which can range from throat infections to life-threatening diseases such as cancers.

In another more preferred embodiment of the method(s) of the invention, the sample is a tissue sample, a blood sample or lymph.

In a further more preferred embodiment of the method(s) of the invention, the presence or amount of the biomarkers is analysed by methods determining genetic or epigenetic modifications or transcriptional or protein levels or a combination thereof.

Methods for determining genetic or epigenetic modifications or transcriptional or protein levels have been defined herein above.

In another more preferred embodiment of the method(s) of the invention, the presence or amount of the biomarkers is determined by immunohistochemistry, mass spectrometry, Western Blot, Northern Blot, PCR, RNA *in situ* hybridisation or a combination thereof.

All of the above methods are well known in the art. Preferably, immunohistochemical methods include, without being limiting, tissue microarrays (TMA) as described in the appended examples. Preferably, when employing TMAs, analysis is carried out in two representative areas per TMA-spot, wherein each area comprises about 100 cells, such as for example exactly 100 cells.

In a further more preferred embodiment of the method(s) of the invention, the biomarker is protein.

The present invention further relates to a kit for predicting the course of disease in a patient having a malignant melanoma, the kit comprising: (a) means for determining the presence or amount of the set of biomarkers as defined in accordance with the methods of the present invention in a sample obtained from said malignant melanoma, and (b) instructions how to use the kit.

In its broadest sense, the term "kit" does not require the presence of any other compounds, vials, containers and the like. Preferably, the various components of the kit may be packaged in one or more containers such as one or more vials. Consequently, the various components of the kit may be present in isolation or combination. The containers or vials may, in addition to the components, comprise preservatives or buffers for storage.

"Means for determining the presence or amount of [...] biomarkers" are well known in the art and include, without being limiting, antibodies specifically binding (i.e. without cross-reacting with unrelated markers) to the biomarkers in accordance with the present invention; nucleic acid probes for the detection of the biomarkers on the nucleic acid level, such as for example nucleic acid probes specifically hybridising with parts or full-length nucleic acid molecules (DNA as well as RNA) encoding said biomarkers; sequencing primers for the analysis and detection of specific sequences of the DNA encoding the biomarkers, e.g. sequences containing mutations known to interfere with the expression of said biomarkers; amplification primers for amplifying transcribed nucleic acid molecules of the respective biomarkers; primers specific for methylated DNA for use in quantitative methylation-specific PCR (Q-MSP) (as described e.g. in Current Protocols in Human Genetics, DOI: 10.1002/ 0471142905.hg1006s61); and also methylation-sensitive restriction enzymes.

The term "comprising" in the context of the kit(s) of the invention denotes that further components can be present in the kit. Non-limiting examples of such further components include, as mentioned, preservatives, buffers for storage, enzymes etc.

Also encompassed by this embodiment is that the kit comprises further means for determining the presence or amount of biomarkers or reference markers different from the biomarkers of the present invention. Such biomarkers or reference markers different from the biomarkers of the present invention include, without being limiting, additional tumour markers for malignant melanoma, such as for example protein S100, HMB45, Melan-A, anti-Pan Melanoma antibodies as well as reference markers, including, without being limiting, GAPDH, RPLP0, PGK1, HSP90AB1, cyclophilin, actin.

If the kit comprises such additional means for determining the presence or amount of biomarkers or reference markers different from the markers in accordance with the present invention, it is preferred that at most 10.000 such additional markers are comprised in the kit of the invention. More preferably, at most 5.000, such as for example at most 2.000 and more preferably at most 1.000 additional nucleic markers are comprised in the kit of the invention. More preferably, at most 800, such as for example at most 600, more preferable at most 400, such as for example at most 300, at most 200, at most 100 and more preferably at most 80 additional markers are comprised in the kit of the invention. Even more preferably, at most 50, such as for example at most 40, more preferable at most 30, such as for example at most 20, at most 10, at most 9, at most 8, at most 7, at most 6, at most 5, at most 4, at most 3, at most 2 and yet more preferably at most 1 additional marker(s) is/are comprised in the kit of the invention. Also preferred is that the kit of the invention only comprises means for determining the presence or amount of the set of biomarkers as defined in accordance with the methods of the present invention.

Furthermore, the present invention also relates to a kit for deriving a treatment regimen for an individual patient having a malignant melanoma, the kit comprising: (a) means for determining the presence or amount of the set of biomarkers as defined in accordance with the present invention in a sample obtained from said malignant melanoma, (b) instructions how to use the kit.

The definitions as well as the preferred embodiments provided herein above with regard to the kit for predicting the course of disease apply *mutatis mutandis* also to this embodiments relating to a kit for preparing a tailored pharmaceutical composition as outlined above.

The present invention also relates to a pharmaceutical composition for use in treating or preventing malignant melanoma, wherein the pharmaceutical composition comprises (an) inhibitor(s) of CD20, Cox-2 and/or PTEN and/or (an) agent(s) affecting MTAP signalling pathways.

The term "inhibitor", in accordance with the present invention, relates to a compound lowering the activity of a target molecule, i.e. CD20, Cox-2 and/or PTEN. The inhibitor may act preferably by performing one or more of the following effects: (i) the transcription of the gene encoding the protein to be inhibited is lowered, (ii) the translation of the

mRNA encoding the protein to be inhibited is lowered, (iii) the protein performs its biochemical function with lowered or abolished efficiency in presence of the inhibitor, and (iv) the protein performs its cellular function with lowered or abolished efficiency in presence of the inhibitor.

Compounds falling in class (i) include compounds interfering with the transcriptional machinery and/or its interaction with the promoter of said gene and/or with expression control elements remote from the promoter such as enhancers but also with epigenetic control mechanisms, thus altering for example the methylation status of the promoter of a target gene. Compounds of class (ii) comprise antisense constructs and constructs for performing RNA interference (e.g. siRNA, shRNA, miRNA) well known in the art (see, e.g. Zamore (2001) Nat. Struct. Biol. 8(9), 746; Tuschl (2001) Chembiochem. 2(4), 239). Compounds of class (iii) interfere with molecular function of the protein to be inhibited, in the present case with the molecular function of CD20, Cox-2 and/or PTEN as described herein above. Accordingly, active site binding compounds are envisaged. Class (iv) includes compounds which do not necessarily bind directly to the target proteins, but still interfere with their activity, for example by binding to and/or inhibiting the function or expression of members of a pathway which comprises the target proteins. These members may be either upstream or downstream of the target protein within said pathway.

In a preferred embodiment, the level of activity (including, as defined above, the level expression) is less than 90%, more preferred less than 80%, less than 70%, less than 60% or less than 50% of the activity in the absence of the inhibitor. Yet more preferred are inhibitors lowering the level to less than 25%, less than 10%, less than 5% or less than 1% of the activity in the absence of the inhibitor.

The efficiency of the inhibitor can be quantified by comparing the level of activity in the presence of the inhibitor to that in the absence of the inhibitor. For example, as an activity measure may be used: the change in amount of mRNA formed, the change in amount of protein formed, the change in amount of activity of CD20, Cox-2 and/or PTEN, and/or a change in the cellular phenotype or in the phenotype of an organism.

The function of any of the inhibitors referred to in the present invention may be identified and/or verified by using high throughput screening assays (HTS). High-throughput assays, independently of being biochemical, cellular or other assays, generally may be performed in wells of microtiter plates, wherein each plate may contain, for example 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to the observed biological activity.

Furthermore, the determination of binding of potential inhibitors can be effected in, for example, any binding assay, preferably biophysical binding assay, which may be used to identify binding test molecules prior to performing the functional/activity assay with the inhibitor. Suitable biophysical binding assays are known in the art and comprise fluorescence polarisation (FP) assay, fluorescence resonance energy transfer (FRET) assay and surface plasmon resonance (SPR) assay.

In cases where the inhibitor acts by affecting the expression level of the target protein, the determination of the expression level of the protein can, for example, be carried out on the nucleic acid level or on the amino acid level, as described herein above.

In a preferred embodiment, the inhibitor is an antibody, an aptamer, an siRNA, an shRNA, an miRNA, a ribozyme, an antisense nucleic acid molecule or a small molecule.

The term "agents affecting MTAP signalling pathways", in accordance with the present invention, relates to agents which do not necessarily bind directly to MTAP, but interfere with MTAP signalling activity, for example by binding to and/or inhibiting the function or expression of members of the MTAP pathway. For example, Wild *et al.* 2006[13] describe that MTAP expression correlates with responsiveness to interferon therapy, thus rendering interferon a suitable therapeutic agent in malignant melanomas expressing MTAP. Further details have been described e.g. in Behmann *et al.* 2003.

Inhibitors of CD20, Cox-2 and/or agents affecting MTAP signalling pathways and/or the oncogenic pathway addiction of PTEN are well known in the art and include, without being limiting, any of the compounds recited herein above.

In a more preferred embodiment, the pharmaceutical composition comprises Rituximab, Celecoxib or interferon alpha.

Rituximab is an antibody also sold under the trade names MabThera® (by Roche) or Rituxan®, (by Biogen Idec/Genentech) that has the DrugBank Accession number DB00073 and the ATC code (Anatomical Therapeutic Chemical Classification System) L01XC02.

Celecoxib, also known as Celebrex, Celebra or Onsenal (sold by Pfizer), is a sulfa non-steroidal anti-inflammatory drug and has the DrugBank Accession number APRD00373 as well as the ATC code L01XX33 M01AH01. Celecoxib has the structural formula:

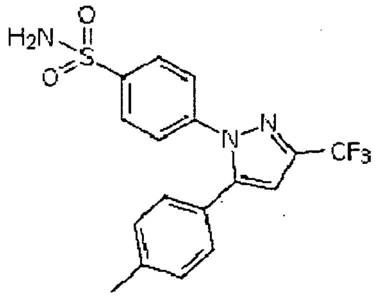

Unless otherwise defined, all technical and scientific terms used herein have the same meaning as commonly understood by one of ordinary skill in the art to which this invention belongs. In case of conflict, the patent specification, including definitions, will prevail.

The figures show:

**Figure 1: The Seven-Marker Signature and Survival of 362 Patients with Primary malignant melanoma**. Panel **A** shows the IHC expression profiles of 362 tumour specimens from the primary cohort ordered by their predicted risk score. Each column represents an individual patient consisting of the expression values of the seven-marker signature (5 risk markers and 2 protective markers). The magnitude of the corresponding risk score is plotted below for 181 low risk patients (light grey; left hand side of the Expression profile) and 181 high risk patients (dark grey; right hand side of the Expression profile). IHC expression values were scaled between 0 (light grey) and 1 (dark grey) for plotting only. White cells represent missing values (n.a.). Panels **B - E** show Kaplan-Meier estimates of overall and recurrence-free survival for high risk patients and low risk patients from the primary cohort according to the nine-marker signature (Panels B, C) and its reduced version, the seven-marker signature (Panels D, E), respectively. Equality in survival expectance of the subgroups is assessed by the log-rank test. Removing the two "less specific" markers (MLH1 and CD49d) from the signature does not reduce the statistical power of the predicted risk score. The difference between high risk patients and low risk patients is highly significant ($p < 0.001$) for the seven-marker signature.

**Figure 2: Validation of the Seven-Marker Signature and the FDR Marker Selection Procedure.** Kaplan-Meier estimates of overall (Panel **A**) and recurrence-free survival (Panel B) for the independent external test cohort of 225 patients (TMA 2) confirm the predictive prognostic power of the signature ($p<0.001$). In addition, the FDR marker selection procedure was tested by a 10-fold cross validation experiment on the 362 patients of the primary cohort (TMA 1) resulting in still significant estimates for overall survival ($p<0.001$; Panel **C**) and recurrence-free survival ($p=0.013$; Panel **D**).

**Figure 3: The Seven-Marker Signature and Survival of Patients with a Tumour Thickness ≤2.0 mm**. Kaplan-Meier estimates show a significantly lower overall ($p=0.0053$, Panel A) and recurrence-free survival ($p=0.008$, Panel B) for patients with a comparatively low tumour thickness ≤2.0 mm but high-risk score. **C, D. Leave-One-Out Cross Validation**. To investigate the generalization error of the models produced by the FDR signature learning procedure a leave-one-out cross validation experiment was conducted on the primary cohort of 362 MM patients. The resulting risk score could significantly ($p<0.001$) differentiate between patients with higher or lower overall survival expectance. The two patient groups also significantly ($p=0.0057$) differ in recurrence-free survival. **E. Permutation Test.** In addition to the cross validation experiments a permutation test was conducted to assess if the signature learning procedure is over fitting the data set. The resulting signature, which was learned on permuted overall survival data, was not able ($p=1$) to discriminate between patients with differing survival expectance. This result indicates that the proposed learning procedure does not over fit the data. F. **Coefficients and Confidence Intervals of the Seven-Marker Signature.** The coefficients from the univariate Cox proportional hazard models are used in a weighted linear combination to predict the risk score for each patient. Markers with negative coefficients represent protective markers (MTAP, β-Catenin); those with positive coefficients risk markers (Bax, CD20, Bcl-X, PTEN and COX-2).

**Figure 4. Hazard Ratios of the Nine-Marker Signature learned by the FDR selection procedure.** Markers with a hazard ratio smaller than 1.00 represent protective markers (MTAP, β-Catenin). Those with hazard ratios larger than 1.00 represent risk markers (Bax, Bcl-X, CD20, CD49d, COX-2, MLH1 and PTEN).

**Figure 5.** Correlation between markers of the invention

The examples illustrate the invention:

**Example 1: Materials and Methods**

**Tissue Microarrays (TMAs)**

TMAs were constructed as described previously[12-14] and based on primary melanoma material, collected between 1994 and 2006. **TMA 1,** the primary cohort, contained tissue punch samples from 364 consecutive (non-selected), formalin-fixed, paraffin-embedded malignant melanoma of 364 different patients and were from the Department of Dermatology, University Hospital of Regensburg, Germany. **TMA 2**, the secondary cohort, which was used as independent external validation cohort, consisted of consecutive (non-selected) melanoma samples from 235 patients of the Department of Dermatology, University Hospital Hamburg-Eppendorf, Germany. For patients with multiple subsequent neoplasms, only initial and single primary malignant melanomas were included. H&E-stained slides of all malignant melanomas were evaluated by two histopathologists. The clinico-pathological characteristics of the two independent cohorts of melanoma patients are given in Table 4. Clinical follow-up data, provided by the local tumour registries, were available for all patients of the primary cohort (n=364) and 231 patients of the secondary cohort. Patients were censored at 120 months, if their follow-up exceeded the 10-year scope of the study. The study for both cohorts was approved by the local scientific ethics committees (approvals no.: 07/093 for Regensburg and MC-028/08 for Hamburg). The retrospective study was conducted according to the Declaration of Helsinki Principles.

**Table 4**

| | | **Primary Cohort** | | **Ext. Test cohort** | |
|---|---|---|---|---|---|
| **TMA characteristics** | | **N** | **%** | **N** | **%** |
| **Origin** | | Regensburg | | | Hamburg |
| **Patients** | | | | | |
| | No. of patients | 364 | | 235 | |
| | No. follow-up | 364 | 100.0 | 231 | 98.3 |
| | No. of patients with at least 1 signature | 362 | 99.5 | 225 | 95.7 |
| **TMA Spots** | | | | | |
| | No. of biomarkers | 70 | | 7 | |
| | Valid spots | 23106 | 90.7 | 1541 | 93.7 |
| | Missing spots | 2374 | 9.3 | 104 | 6.3 |
| **Clinicopathological** | | **N** | **%** | **N** | **%** |
| **Age** | | | | | |
| | <=60 | 180 | 49.5 | 139 | 59.1 |
| | >60 | 184 | 50.5 | 92 | 39.1 |
| | unknown | | | 4 | 1.7 |
| **Sex** | | | | | |
| | Male | 195 | 53.6 | 126 | 53.6 |
| | Female | 169 | 46.4 | 105 | 44.7 |
| | unknown | | | 4 | 1.7 |
| **Tumor thickness** | | | | | |
| | <= 1mm | 163 | 44.8 | 110 | 46.8 |
| | 1.01--2mm | 92 | 25.3 | 47 | 20.0 |
| | 2.01--4mm | 61 | 16.8 | 36 | 15.3 |
| | > 4mm | 42 | 11.5 | 36 | 15.3 |
| | unknown | 6 | 1.6 | 6 | 2.6 |

(continued)

| Clinicopathological | | N | % | N | % |
|---|---|---|---|---|---|
| Clark level | | | | | |
| | 1 | 2 | 0.5 | 1 | 0.4 |
| | 2 | 75 | 20.6 | 39 | 16.6 |
| | 3 | 106 | 29.1 | 80 | 34.0 |
| | 4 | 149 | 40.9 | 90 | 38.3 |
| | 5 | 14 | 3.8 | 19 | 8.1 |
| | unknown | 18 | 4.9 | 6 | 2.6 |
| Growth pattern | | | | | |
| | SSM | 170 | 46.7 | 146 | 62.1 |
| | NMM | 56 | 15.4 | 49 | 20.9 |
| | LMM | 42 | 11.5 | 12 | 5.1 |
| | ALM | 28 | 7.7 | 8 | 3.4 |
| | NOS | 68 | 18.7 | 20 | 8.5 |
| **Immunohistochemical data** | | **N** | **%** | **N** | **%** |
| **Bax** | | | | | |
| | 0 | 5 | 1.4 | 5 | 2.1 |
| | 1 | 60 | 16.5 | 49 | 20.9 |
| | 2 | 95 | 26.1 | 81 | 34.5 |
| | 3 | 88 | 24.2 | 56 | 23.8 |
| | 4 | 88 | 24.2 | 29 | 12.3 |
| | unknown | 28 | 7.7 | 15 | 6.4 |
| b-Catenin | | | | | |
| | 0 | 10 | 2.7 | 4 | 1.7 |
| | 1 | 121 | 33.2 | 43 | 18.3 |
| | 2 | 106 | 29.1 | 108 | 46.0 |
| | 3 | 71 | 19.5 | 53 | 22.6 |
| | 4 | 17 | 4.7 | 11 | 4.7 |
| | unknown | 39 | 10.7 | 16 | 6.8 |
| CD20 | | | | | |
| | 0 | 333 | 91.5 | 154 | 65.5 |
| | 1 | 12 | 3.3 | 48 | 20.4 |
| | 2 | 4 | 1.1 | 16 | 6.8 |
| | 3 | 1 | 0.3 | 1 | 0.4 |
| | unknown | 14 | 3.8 | 16 | 6.8 |
| BCL-X | | | | | |
| | 0 | 151 | 41.5 | 66 | 28.1 |
| | 1 | 167 | 45.9 | 117 | 49.8 |
| | 2 | 26 | 7.1 | 38 | 16.2 |
| | 3 | 1 | 0.3 | 4 | 1.7 |

(continued)

| Immunohistochemical data | | N | % | N | % |
|---|---|---|---|---|---|
| | unknown | 19 | 5.2 | 10 | 4.3 |
| MTAP | | | | | |
| | 0 | 56 | 15.4 | 103 | 43.8 |
| | 1 | 245 | 67.3 | 101 | 43.0 |
| | 2 | | | 16 | 6.8 |
| | unknown | 63 | 17.3 | 15 | 6.4 |
| PTEN | | | | | |
| | 0 | 57 | 15.7 | 23 | 9.8 |
| | 1 | 140 | 38.5 | 87 | 37.0 |
| | 2 | 116 | 31.9 | 76 | 32.3 |
| | 3 | 28 | 7.7 | 25 | 10.6 |
| | 4 unknown | 4 19 | 1.1 5.2 | 8 16 | 3.4 6.8 |
| **Cox-2** | | | | | |
| | 0 | 121 | 33.2 | 20 | 8.5 |
| | 1 | 188 | 51.6 | 103 | 43.8 |
| | 2 | 39 | 10.7 | 73 | 31.1 |
| | 3 | 5 | 1.4 | 21 | 8.9 |
| | 4 | | | 2 | 0.9 |
| | unknown | 11 | 3.0 | 16 | 6.8 |
| **CD49d** | | | | | |
| | 0 | 63 | 17.3 | n.a | |
| | 1 | 137 | 37.6 | | |
| | 2 | 78 | 21.4 | | |
| | 3 | 33 | 9.1 | | |
| | 4 | 3 | 0.8 | | |
| | unknown | 50 | 13.7 | | |
| **MLH1** | | | | | |
| | 0 | 65 | 17.9 | n.a | |
| | 1 | 130 | 35.7 | | |
| | 2 | 99 | 27.2 | | |
| | 3 | 37 | 10.2 | | |
| | 4 | 13 | 3.6 | | |
| | unknown | 20 | 5.5 | | |

**Table 4: Characterization and Comparison of the Primary Cohort (TMA 1) and the External Test Cohort (TMA 2).** Reported are the number of counts and the associated percentages for all specimens on the tissue microarrays. CD49d and MLH1 are not contained in the final seven-marker signature and therefore were not analyzed on the external test TMA 2. Missing values are listed as "unknown".

## Immunohistochemical Analysis

Paraffin-embedded preparations of melanoma tissues were screened for protein expression according to standardized immunohistochemical (IHC) protocols as described previously.[12-14] The primary antibodies used in this study were selected for reporting on key aspects of apoptosis, cell cycle, signal transduction, cell adhesion, melanoma differentiation and proliferation, and tumour metabolism.

All IHC investigations were based on an avidin-biotin peroxidase method with a 3-amino-9-ethylcarbazole (AEC) chromatogen. After antigen retrieval (steam boiler with citrate-buffer, pH 6.0 or with Tris-EDTA-buffer, pH 9.0 for 20 min), immunohistochemistry was carried out applying the ZytoChemPlus HRP Broad Spectrum Kit (Zytomed Systems, Berlin, Germany) according to the manufacturer's instructions. IHC stainings were performed for 70 different primary antibodies (source and concentration are listed in Table 5). Cytoplasmic and nuclear markers were visualized with AEC solution (AEC+ High Sensitivity Substrate Chromogen, ready-to-use, DAKO, Glostrup, Denmark). The red colour of the AEC substrate chromogen (3-amino-9-ethylcarbazole) is very beneficial to rule out the possibility of a role of endogenous melanin in the observed reactivity. All sections were counterstained with hematoxylin (DAKO). Negative controls were obtained by omitting the primary antibody. Two dermatohistopathologists performed a blinded evaluation of the stained slides without knowledge of clinical data. The specificity of the commercial antibodies has been thoroughly tested by Western blotting using melanocytes and a variety of human cell lines including several melanoma cell lines.

**Table 5. Properties of the 70 Biomarker Candidates for Malignant Melanoma immunohistochemically analyzed in this Study.** All antibodies investigated are listed indicating source, dilution, pattern of reactivity and positive control. The described signature was statistically learned by the FDR selection procedure from this pool of 70 biomarkers.

| Protein Name | HUGO Approved Symbol | Functional Group | Source | Clone | Dilution | Cellular Localization | Positive Control |
|---|---|---|---|---|---|---|---|
| Akt 3 | AKT3 | Signaling, Apoptosis | Abgent | Rabbit. Polyclonal | 1:100 | Cytoplasmic | Breast cancer |
| Phospho-Akt (Thr308) | Phospho-AKT3 | Signaling, Apoptosis | Cell Signaling | Rabbit, 244F9H2 | 1:10 | Cytoplasmic | Breast cancer |
| Bax | BAX | Apoptosis | Cell Signaling | Rabbit, polyclonal | 1:10 | Cytoplasmic | Lung cancer |
| Bcl2 | BCL2 | Apoptosis | Dako | Mouse, 124 | 1:100 | Cytoplasmic | Kidney |
| Bcl-X | BCL2L1 | Apoptosis | Diagnostic Biosystems | Mouse, 2H12 | 1:10 | Cytoplasmic, cell membrane | Tonsil |
| Bcl2L1 | BCL2L1 | Apoptosis | Abcam | Mouse, SPM165 | 1:200 | perinuclear | Melanoma |
| BMI1 | BMI1 | Transcription | Abgent | Rabbit, polyclonal | 1:25 | Cytoplasmic | Breast cancer |
| B-Raf | BRAF | Signaling, Apoptosis | Epitomics | Rabbit, EP152Y | 1:50 | Cytoplasmic | Prostate cancer |
| E-Cadherin | CDH1 | Cell-cell contact | Chemicon | Mouse, 3GB5 | r-t-u | Cell membrane | Breast cancer |
| P-Cadherin | CDH3 | Cell-cell contact | BD Biosciences | Mouse. 56 | 1:20 | Cell membrane | Placenta |
| β-Catenin | CTNNB1 | Cell-cell contact | Cell Signaling | Rabbit, polyclonal | 1:250 | Cytoplasmic, nuclear, cell membrane | Breast cancer |
| Phospho-β-Catenin | Phospho-CTNNB1 | Cell-cell contact | Cell Signaling | Rabbit, polyclonal | 1:50 | Nuclear | Breast cancer |
| Caveolin | CAV1 | Cell-cell contact | BD Biosciences | Rabbit, polyclonal | 1:1000 | Cell membrane | Placenta |
| CD 20 | MS4A1 | Differentiation, Stem cell marker cand. | Zytorned | Mouse, L26 | r-t-u | Cell membrane cytoplasmic | Tonsil |
| CD 44 | CD44 | Cell-cell contact, Stem cell marker cand. | Lab Vision | Mouse. 156-3C11 | 1:2000 | Cell membrane | Tonsil |

(continued)

| Protein Name | HUGO Approved Symbol | Functional Group | Source | Clone | Dilution | Cellular Localization | Positive Control |
|---|---|---|---|---|---|---|---|
| CD 49d | ITGA4 | Cell-cell contact, Stem cell marker cand. | Acris | Rabbit, polyclonal | 1:50 | Cell membrane | Breast cancer |
| CD 117 (c-kit) | KIT | Proliferation, Stem cell marker cand. | Dako | Rabbit, polyclonal | 1:600 | Cell membrane cytoplasmic | Colorectal cancer |
| CD 166 | ALCAM | Cell-cell contact, Stem cell marker cand. | Abcam | Mouse, MOG/07 | 1:50 | Cell membrane | Prostate cancer |
| CD 171 | L1CAM | Cell-cell contact | Sigma | Rabbit | 1:350 | Cytoplasmic | Breast cancer |
| CDK2 | CDK2 | Cell cycle | Thermo Scientific | Mouse, 2B6 + 8D4 | 1:200 | Cytoplasmic, nuclear | Tonsil |
| c-Myc | MYC | Cell cycle, Proliferation | Santa Cruz | Mouse, monoclonal | 1:500 | Cytoplasmic, nuclear | Colorectal cancer |
| Cox-2 | MT-CO2 | Metabolism | Cayman | Mouse, monoclonal | 1:200 | Cytoplasmic | Colorectal cancer |
| CXCR4 | CXCR4 | Cell-cell contact, Stem cell marker cand. | Acris | Rabbit, polyclonal | 1:750 | Cytoplasmic, cell membrane | Breast cancer |
| Gyclin A | CCNA1 | Cell cycle | Novo Castra | Mouse, 6E6 | 1:50 | nuclear | Tonsil |
| Cyclin D1 | CCND1 | Cell cycle | Novo Castra | Mouse, DCS-6 | 1:20 | Nuclear, cytoplasmic | Breast cancer |
| Eph B2 | EPHB2 | Cell-cell contact | Abcam | Rabbit, polyclonal | 1:100 | Cell membrane cytoplasmic | Breast cancer |
| ephrin-B2 | EFNB2 | Cell-cell contact | Santa Cruz | Rabbit, polyclonal | 1:50 | Cell membrane Cytoplasmic | Melanoma |
| Ezrin | EZR | Signaling | Abcam | Mouse, 3C12 | 1:100 | Cell membrane | Lung Cancer |
| Fas | FAS | Apoptosis | Cell Signaling | Rabbit, C18C12 | 1:10 | Cytoplasmic | Colorectal cancer |
| FZD-7 | FZD7 | Signaling, | Acris | Rabbit. polyclonal | 1:250 | Cytoplasmic | Placenta |

(continued)

| Protein Name | HUGO Approved Symbol | Functional Group | Source | Clone | Dilution | Cellular Localization | Positive Control |
|---|---|---|---|---|---|---|---|
| Glut-1 | SLC2A1 | Metabolism | Acris | mouse, SPM498 | 1:200 | Cell membrane | Breast cancer |
| HIF-1α | HIF1A | Metabolism | R+D | Mouse, 241812 | 1:20 | Cytoplasmic, nuclear | Breast cancer |
| Anti-Melanosome HMB45 | -/- | Differentiation | Dako | Mouse, HMB45 | 1:50 | Cytoplasmic | Melanoma |
| IGF-2 | IGF2 | Proliferation | Abcam | Rabbit, polyclonal | 1:200 | Cytoplasmic | Placenta |
| iNOS | ISYNA1 | Metabolism | Abcam | Rabbit, polyclonal | 1:200 | Cytoplasmic | Lung cancer |
| Ki-67 | MKI67 | Cell cylce, Proliferation | Dako | Mouse, MIB-1 | 1:100 | Nuclear | Skin |
| MHC 1 | MYH11 | Cell-cell contact | Abcam | Mouse, 3F8 | 1:100 | Cell membrane | Kidney |
| Melan A | MLANA | Differentiation | Novo Castra | Mouse, A103 | 1:50 | Cytoplasmic | Skin |
| MITF | MITF | Differentiation | Dako | Mouse, D5 | 1:50 | Nuclear | Melanoma |
| MLH1 1 | MLH1 | DNA repair | BD Pharmingen | Mouse, G168-15 | 1:50 | Nuclear, cytoplasmic | Colorectal cancer |
| MSH2 | MSH2 | DNA repair | Calbiochem | Mouse, FE11 | 1:40 | Nuclear, cytoplasmic | Colorectal cancer |
| MTAP | MTAP | Metabolism | ProteinTech Group | Rabbit, polyclonal | 1:500 | Cytoplasmic | Breast cancer |
| MTSS1 | MTSS1 | Cytoskeletal remodeling | Abnova | Mouse, 2G9 | 1:500 | Nuclear, cytoplasmic | Colorectal cancer |
| MUM1p | IRF4 | Transcription | Santa Cruz | Mouse, monoclonal | 1:10 | Nuclear, Cytoplasmic | Breast cancer |
| NF-κB | RELA | Transcription | Santa Cruz | Mouse, F-6 | 1:500 | Nuclear, Cytoplasmic | Breast cancer |
| N-Ras | NRAS | Signaling | Santa Cruz | Mouse, F155 | 1:50 | Cytoplasmic | Lymph node |
| N-Ras | NRAS | Signaling | Santa Cruz | Mouse, F155 | 1:50 | Cytoplasmic | Lymph node |
| p14 | CDKN2A | Cell cycle | Cell Signaling | Mouse, 4C6/4 | 1:10 | Nuclear, cytoplasmic | Breast cancer |

(continued)

| Protein Name | HUGO Approved Symbol | Functional Group | Source | Clone | Dilution | Cellular Localization | Positive Control |
|---|---|---|---|---|---|---|---|
| p15 | CDKN2B | Cell cycle | Acris | Mouse, 15P06 | 1:25 | Nuclear | Colorectal cancer |
| p16 | CDKN2A | Cell cycle | Santa Cruz | Mouse, F-12 | 1:50 | Nuclear | Colorectal cancer |
| p21 | CDKN1A | Cell cycle | Dako | Mouse, SX118 | 1:50 | Nuclear | Colorectal cancer |
| p27 | CDKN1B | Cell cycle | Dako | Mouse, SX53G8 | 1:50 | Nuclear | lymph node |
| p53 | TP53 | Cell cycle, Apoptosis | Dako | Mouse, DO-7 | 1:25 | Nuclear, cytoplasmic | Breast cancer |
| p75(NGFR) | NGFR | Differentiation | Abcam | Rabbit, EP1039Y | 1:100 | Cell membrane | Placenta |
| PGF | PGF | Proliferation | ProteinTech Group | Rabbit, Polyclonal | 1:50 | Cytoplasmic | Breast cancer |
| PMP2 | PMP2 | Differentiation | ProteinTech Group | Rabbit, polyclonal | 1:100 | Cytoplasmic | Glioma |
| PPAR $\alpha$ | PPARA | Signaling | Abcam | Rabbit, polyclonal | 1:500 | Cytoplasmic, nuclear | Breast cancer |
| PTEN | PTEN | Signaling | Cell Signaling | Rabbit. 138G6 | 1:100 | Cytoplasmic. nuclear | Colorectal cancer |
| Rb | RB1 | Cell cycle | Calbiochem | Mouse, LM95.1 | 1:50 | Nuclear | Colorectal cancer |
| Phospho-Rb | Phospho-RB1 | Cell cycle | Cell Signaling | Rabbit, polyclonal | 1:50 | Nuclear | Colorectal cancer |
| Ro-52 | TRIM21 | Autoimmunology | Santa Cruz | Mouse, D-12 | 1:200 | Nuclear | n. d. |
| Survivin | BIRC5 | Apoptosis | Cell Signaling | Rabbit, 71G4 | 1:100 | Nuclear | Colorectal cancer |
| SKP2 | SKP2 | Cell cycle | Zytomed | Rabbit, polyclonal | 1:200 | Nuclear, cytoplasmic | Prostate cancer |
| STAT1 | STA1 | Signaling | Cell Signaling | Rabbit, 42H3 | 1:200 | Cytoplasmic, nuclear | Colorectal cancer |
| Phospho-STAT1 (S727) | | Signaling | Abcam | Rabbit, polyclonal | 1:100 | Cytoplasmic, nuclear | Breast cancer |
| S1P1 | S1PR1 | cell cycle | Cayman | Rabbit. polyclonal | 1:50 | Cytoplasmic | Breast cancer |

(continued)

| Protein Name | HUGO Approved Symbol | Functional Group | Source | Clone | Dilution | Cellular Localization | Positive Control |
|---|---|---|---|---|---|---|---|
| TGF-β1 | TGFB1 | Proliferation | Zytomed | Rabbit, polyclonal | 1:25 | Cytoplasmic. cell membrane | Colorectal cancer |
| Topoisomerase IIα | TOP2A | DNA repair | Dako | Mouse, Ki-S1 | 1:100 | Nuclear, Cytoplasmic | Placenta |
| VEGFR-2 | KDR | Proliferation | Cell Signaling | Rabbit, 55B11 | 1:200 | Cytoplasmic, | Colorectal cancer |
| XIAP (Birc4) | XIAP | Apoptosis | Lifespan | Rabbit, polyclonal | 1:1000 | Cytoplasmic, | Placenta |

A dermatohistopathologist and a surgical pathologist performed a blinded, stringent evaluation of the stained slides as previously described[13,14]. Cytoplasmic and nuclear immuno-reactivity were evaluated using a stepwise scoring system (0 to 4+): 0 (negative): no cytoplasmic staining or 0% of cell nuclei stained; 1+: weak cytoplasmic staining or less than 20% of cell nuclei stained; 2+: moderate cytoplasmic staining or 21 to 50% of cell nuclei stained; 3+: strong cytoplasmic staining or 51 to 90% of cell nuclei stained; 4+: very strong cytoplasmic staining or nuclear staining greater than 90%. This semi-quantitative scoring system was consistently used for all 70 markers analysed. Cytoplasmic markers were estimated according to the staining intensity found in the melanoma cells of the individual TMA spot. For nuclear markers, the percentage of melanoma cell nuclei with positive staining was assessed. TMA spots with a lack of tumour tissue or presence of necrosis or crush artefact were excluded from the analysis.

**Statistical Analysis**

An estimation of statistical power versus total sample size N for different hazard ratios was performed. Accordingly, the available sample size of 364 analyzable patients on TMA1 would be sufficient to detect a difference concerning survival with a significance of $p<0.05$ and a power of almost 100%. Calculations were performed using the respective models of the PASS 2008 software (NCSS, Kaysville, UT).

One of the main statistical problems in large scale IHC studies are missing values in the design matrix due to missing or corrupt spots on the TMA. The more markers are investigated the higher the chance that at least one value is missing per patient. Frequently, this problem is tackled by either sacrificing a larger number of patient records or by employing volatile multiple imputation techniques. In this study 9.3% of values are missing which would reduce the set of patients with all IHC measurements from 364 to 170. Algorithms like random survival forests[15] and ensemble learning with gradient boosting[16] are capable of dealing with missing values, but lead to models, which are not intuitively interpretable and difficult to implement in clinical practice. To overcome these problems the following learning procedure was employed which is invariant to missing values and results in an easily interpretable and a practically applicable linear model.

Prognostic power of the 70 markers was assessed by learning univariate proportional hazard models,[17] yielding 11 markers significantly associated with overall survival. To correct for multiple testing, the false discovery rate (FDR) procedure[18] was applied with a FDR of 0.15 reducing the set of significantly associated markers to 9. A risk score was calculated for each patient by a linear combination of the univariate Cox regression coefficients $\beta$ and the corresponding IHC measurements $\boldsymbol{x}$. Finally, the score is normalised by the number of markers measured:

$$score(\boldsymbol{x}) = \left(\sum_{i=1}^{|\boldsymbol{x}|}(\beta_i x_i)\, 1_{[\exists x_i]}\right) \Bigg/ \left(\sum_{i=1}^{|\boldsymbol{x}|} 1_{[\exists x_i]}\right)$$

Based on this risk score, patients were assigned to a high risk group and a low risk group, split at the 50$^{th}$ percentile (median) of all scores. Thus, the final model consists of the coefficient vector $\beta$ and the median threshold $\theta$.

A simplified version of this risk score calculation is as described herein above, i.e.:

$$\text{score}(x) = \left(\sum_{i=1}^{D}(\beta_i x_i)\alpha_i\right) \Big/ \left(\sum_{i=1}^{D}\alpha_i\right), \quad \alpha_i = \begin{cases} 1, & \text{if } x_i \text{ exists} \\ 0, & \text{if } x_i \text{ is missing} \end{cases}$$

Nonparametric Kaplan-Meier estimators[19] were used to analyse overall survival and recurrence-free survival. Differences between survival estimates were assessed with the log-rank test (LRT).[20] P-values below 0.05 were considered to indicate statistical significance. Statistical analyses were conducted using R version 2.11.[21]

**Statistical Validation**

The validity of the learning procedure and hence the accuracy of the signature was assessed in three different validation experiments. First, leave-one-out cross validation was employed by excluding one patient at a time from the training set and subsequently scoring the left out patient with the signature learned from the rest of the patients. Repeating

this procedure 364 times yields a leave-one-out score estimate for each patient in the study. The resulting difference between high risk patients and low risk patients was highly significant (p<0.001) and is depicted in Fig. 3C and D.

Second, 10-fold cross validation was conducted by partitioning the dataset into 10 parts of equal size using 90% of the patients for learning and 10% for validation. The procedure was repeated 10 times resulting in a 10-fold score for each patient. As expected, the resulting differentiation between high risk and low risk patient was worse in terms of the LRT p-value but was still highly significant (p<0.001) as shown in Fig. 2C and D.

The third validation experiment was conducted to assess if the proposed marker selection procedure is prone to over fitting. To this end, the target variable was randomly permuted and a model was learned to predict the risk score based on this distorted data. Fig. 3E illustrates that it was impossible to learn a meaningful score (p>0.5) based on the permuted labels. Although a large number of markers were analysed to learn the signature, this result indicates that the proposed algorithm does not over fit.

**Example 2:** The Nine-Marker Signature and Survival

The proposed learning procedure based on the Cox regression coefficients and multiple testing correction with FDR yielded nine markers which were correlated with death from any cause. Two of these markers were protective markers (associated with a hazard ratio of less than 1.00) and seven were risk markers (associated with a hazard ratio of more than 1.00) (Fig. 4).

Among the nine markers were Bax and Bcl-X, two major regulators of the "intrinsic" mitochondrial apoptosis pathway.[22] Moreover, β-Catenin, a key downstream effector in the Wnt signaling pathway,[23] and, CD20, a known B-cell marker recently suggested as candidate marker for melanoma stem cells[24]. CD49d, an α4-integrin (ITGA4) participating in cell-surface mediated signaling and adhesion, was included, too.[25] Apart from this, COX-2, a cyclooxygenase also referred to as Prostaglandin H Synthase 2 with overexpression in a variety of tumors including melanoma tumors was part of the signature.[14] Two other markers were MLH1, a DNA mismatch repair protein,[26] and MTAP, a "housekeeping enzyme" in polyamine metabolism and modulating protein of interferon response mechanisms.[13,27] Finally, the tumor suppressor phosphatase and tensin homolog PTEN was identified as another signature protein. PTEN counteracts one of the most critical cancer promoting pathways,[28] the phosphatidylinositol 3-kinase (PI3K)/Akt signaling pathway. Clinically, *PTEN* mutations and deficiencies are prevalent in many types of human cancers and loss of functional PTEN has substantial impact on multiple aspects of cancer development. MTAP and β-Catenin were the only protective markers, whereas the other seven markers (Bax, Bcl-X, CD20, CD49d, COX-2, MLH1, PTEN) were assigned risk markers.

Table 1 lists the characteristics of 362 patients in the study (two patients were removed due to lack of all nine markers from the signature). Among these 362 patients of the primary cohort tumors with high risk scores expressed risk markers, whereas tumors with low risk scores expressed protective markers (Fig. 1A). Patients with a high-risk nine-marker signature had a lower median overall survival than those with a low-risk nine-marker signature (90 months versus not reached) (Fig. 1 B). Patients with tumors with a high-risk marker signature were associated with a lower median recurrence-free survival than patients with tumors with a low-risk gene signature (36 months versus 88) (Fig. 1C).

The cross validation experiments showed comparable results and demonstrated that learning a marker signature for overall survival is feasible and reproducible (Fig. 2C D). For leave-one-out cross validation, patients with high risk scores had a median survival of 94 month whereas median survival for patients with low risk signature was not reached (Fig. 3C). The difference in survival expectance between patients with high-risk score and low-risk score was highly significant (p=0.000067). Although 10-fold cross validation has lower bias and higher variance the difference between the high risk and low risk group (94 month versus not reached) was still significant (p=0.00017) as shown in Fig. 2C. In contrast to the cross validation experiments it was not possible to learn a signature to predict permuted labels (p>0.5), which indicates that the proposed learning procedure is not over fitting. In the permutation test median survival was not reached by any risk-group (Fig. 3E).

**Example 3:** The Seven-Marker Signature and Survival

The aim of this study was to provide a maximum of prognostic and therapeutically relevant information by a minimum of markers combined in a clear signature. For the sake of clinical feasibility and cost saving, an IHC marker set suitable for routine clinical assessment should be based on a limited number of antibodies. Accordingly, the 9-marker signature was reduced by the risk marker with the lowest Cox regression coefficients β, i.e. MLH1 (β = 0.254). Subsequently, the remaining 8 risk markers were evaluated regarding their impact on cancer development and progression and potential therapeutic implications. In this setting, CD49d, an α4-integrin (ITGA4) participating in cell-surface mediated signaling and adhesion, was considered to be the most dispensable marker. In particular, Western blot analysis of this 70-180 kDa protein did not reveal one specific but several bands for a panel of melanoma cell lines and melanocytes, respectively. Specificity of all other IHC antibodies of the signature could be confirmed by immunoblotting.

Among the 362 patients of the primary cohort, patients with a high-risk seven-marker signature (Bax, Bcl-X, β-Catenin, CD20, COX-2, MTAP, PTEN) had a shorter median overall survival than the patients with a low-risk seven-marker signature (88 months versus not reached) and the difference between the two patient groups was highly significant (p=0.0000000042) (Fig. 1 D). The high-risk seven-marker signature was associated with a median recurrence-free survival of 33 months, whereas the low-risk seven-marker signature was associated with a median recurrence-free survival of 88 months (LRT p=0.00034) (Fig. 1 E).

According to multivariate Cox regression analysis, the seven-marker risk score, tumor thickness, sex, and age were significantly associated with death from any cause among the 356 patients (6 observations were deleted due to missing values) (Table 1).

A subgroup analysis of 253 patients with a tumor depth of ≤2 mm revealed that those 148 patients with a high-risk marker signature had a significant (p=0.0053) shorter overall survival (Fig. 3A) and recurrence-free survival (p=0.008) than the 105 patients with a low-risk marker signature (Fig. 3B).

**Example 4:** Validation of the Seven-Marker Signature on an External Test Cohort

The clinical characteristics of the 225 patients in the external test cohort are listed in Table 2. Patients with a high-risk marker signature had a significantly (p=0.000017) different survival expectance and shorter median overall survival compared to patients with a low-risk signature (95 months versus not reached) (Fig. 2A). According to multivariate Cox regression including sex, age, tumor thickness, ulceration and nodal status, the seven-marker signature was significantly associated with overall survival (p=0.0000098, Table 1). Additionally, the recurrence-free survival differed significantly between the two risk groups (p=0.004; Fig. 2B).

Table 2. Clinical Characteristics of the the External Test Cohort of Patients with MM (TMA 2). Comparing high-risk patients (first column) with low-risk patients (second column) based on their seven-marker risk score shows significant difference in tumour thickness (p<0.001) and no difference in sex (p=1) and age (p=0.267). Furthermore, hazard ratios and p-values are reported for a multivariate Cox regression model comprising all listed variables. Regarding overall survival the seven-marker risk score is statistically significant (p<0.001) independent of sex, age and tumour thickness. Continuous variables are reported with mean and standard deviation and categorical variables are listed with number of counts and percentages.

| | High risk (N=181) | Low risk (N=181) | p-Value: high vs. low risk | Multivariate Cox Regression Analysis: Hazard ratio (95% Cl) | p-Value |
|---|---|---|---|---|---|
| 7-Marker risk score | 0.270±0.098 | 0.04 ±0.071 | <<0.0001 #1 | 24.91 (3.84-161.71) | 0.00076 *** |
| Age-yr | 55.2±16 | 52.6±6.6 | 0.267 #1 | 1.02 (1.00-1.04) | 0.016 * |
| Sex- no. of patients (%) | | | | | |
| Male | 81 (55.1) | 41 (54.7) | 1#2 | 0.67 (0.39-1.14) | 0.14 |
| Female | 66(44.9) | 34 (45.3) | | | |
| Tumor thickness- mm | 2.55± 2.67 | 1.08±1.17 | 0.0000000512 #1 | 1.28 (1.19-1.38) | 0.000000000028 *** |

#1Welch two sample t-test
#2Fisher's exact test
* p-Value < 0.05
*** p-Value < 0.001

**References**

1. Jemal A, Siegel R, Ward E, et al. Cancer statistics, 2008. CA Cancer J Clin, 2008;58:71-96.

2. Lens MB, Dawes M. Global perspectives of contemporary epidemiological trends of cutaneous malignant melanoma. Br J Dermatol 2004;150:179-85.

3. Balch CM, Buzaid AC, Soong SJ, et al. Final version of the American Joint Committee on Cancer staging system for cutaneous melanoma. J Clin Oncol 2001;19:3635-48.

4. Agarwala SS. Current systemic therapy for metastatic melanoma. Expert Rev Anticancer Ther 2009; 9:587-95.

5. Balch CM, Gershenwald JE, Soong SJ, et al. Final Version of 2009 AJCC Melanoma Staging and Classification. J Clin Oncol 2009;27:6199-206.

6. Breslow A: Thickness, cross-sectional areas, and depth of invasion in the prognosis of cutaneous melanoma. Ann Surg 1970;172:902-8.

7. Grande Sarpa H, Reinke K, Shaikh L, et al. Prognostic significance of extent of ulceration in primary cutaneous melanoma. Am J Surg Pathol 2006;30:1396-400.

8. Morton DL, Thompson JF, Cochran AJ, et al. Sentinel-node biopsy or nodal observation in melanoma. N Engl J Med 2006;355:1307-17.

9. Gould Rothberg BE, Bracken MB, Rimm DL. Tissue biomarkers for prognosis in cutaneous melanoma: a systematic review and meta-analysis. J Natl Cancer Inst 2009;101:452-74.

10. Gould Rothberg BE, Rimm DL. Biomarkers: The useful and the not so useful - an assessment of molecular prognostic markers for cutaneous melanoma. J Invest Dermatol 2010;130:1971-87.

11. Reporting recommendations for tumour marker prognostic studies (REMARK). J Natl Cancer Institute 2005; 97: 1180-4.

12. Alonso SR, Ortiz P, Pollan M, et al. Progression in cutaneous malignant melanoma is associated with distinct expression profiles: a tissue microarray-based study. Am J Pathol 2004;164:193-203.

13. Wild PJ, Meyer S, Bataille F, et al. Tissue microarray analysis of MTAP expression in melanocytic skin tumours. Arch Dermatol 2006;142:471-6.

14. Meyer S, Vogt T, Landthaler M, et al. Cyclooxygenase 2 (COX2) and Peroxisome Proliferator-Activated Receptor Gamma (PPARG) are stage-dependent prognostic markers of malignant melanoma. PPAR Res 2009 Jul 20 (Epub ahead of print).

15. Ishwaran H, Kogalur UB, Blackstone EH, Lauer MS. Random survival forests. Ann Appl Stat 2008;2:841-60.

16. Hothorn T, Buehlmann P, Dudoit S, Molinaro A, van Der Laan MJ. Survival ensembles. Biostatistics 2006;7: 355-73.

17. Cox DR. Regression models and life-tables. J R Stat Soc Series B 1972;34:187-220.

18. Benjamini Y, Hochberg Y. Controlling the false discovery rate: a practical and powerful approach to multiple testing. J R Stat Soc Series B Stat Methodol 1995;57:289-300.

19. Kaplan EL, Meier P. Nonparametric estimation from incomplete observations. J Amer Statist Assn 1958;53: 457-81.

20. Mantel N. Evaluation of survival data and two new rank order statistics arising in its consideration. Cancer Chemother Rep 1966;50:163-70.

21. R Development Core Team (2009). R: A language and environment for statistical computing. R Foundation for Statistical Computing, Vienna, Austria. ISBN 3-900051-07-0, URL http://www.R-project.org.

22. Lowe SW, Cepero E, Evan G. Intrinsic tumour suppression. Nature 2004;432:307-15.

23. Delmas V, Beermann F, Martinozzi S, et al. Beta-catenin induces immortalization of melanocytes by suppressing p161NK4a expression and cooperates with N-Ras in melanoma development. Genes Dev 2007;21:2923-35.

24. Zabierowski SE, Herlyn M. Melanoma stem cells: the dark seed of melanoma. J Clin Oncol 2008;26:2890-4.

25. Kuphal S, Bauer R, Bosserhoff AK. Integrin signaling in malignant melanoma. Cancer Metastasis Rev 2005;24: 195-222.

26. Korabiowska M, Brinck U, Stachura J, et al. Exonic deletions of mismatch repair genes MLH1 and MSH2 correlate with prognosis and protein expression levels in malignant melanomas. Anticancer Res 2006;26:1231-5.

27. Behrmann I, Wallner S, Komyod W, et al. Characterization of methylthioadenosine phosphorylase (MTAP) expression in malignant melanoma. Am J Pathol 2003;163:683-90.

28. Zhang S and Yu D. PI(3)king apart PTEN's role in cancer. Clin Cancer Res 2010;16:4325-30.

29. Avivi I, Robinson S, Goldstone A. Clinical use of rituximab in haematological malignancies. Br J Cancer 2003; 89:1389-94.

30. Hla T and Neilson K. Human cyclooxygenase-2 cDNA. Proc Natl Acad Sci U S A 1992;89:7384-8.

31. Denkert C, Köbel M, Berger S, et al. Expression of cyclooxygenase 2 in human malignant melanoma. Cancer Res 2001;61:1733-40.

32. Thun MJ, Henley SJ, Gansler T. Inflammation and cancer: an epidemiological perspective. Novartis Found Symp 2004;256:6-21.

33. Reichle A, Vogt T, Coras B, et al. Targeted combined anti-inflammatory and angiostatic therapy in advanced melanoma: a randomized phase II trial. Melanoma Res 2007;17:360-4.

34. Ascierto PA, Kirkwood JM. Adjuvant therapy of melanoma with interferon: lessons of the past decade. J Transl Med 2008;6:62.

35. Meyer S, Wild PJ, Vogt T, et al. Methylthioadenosine phosphorylase represents a predictive marker for response to adjuvant interferon therapy in patients with malignant melanoma. Exp Dermatol 2010;19:e251-7.

36. Azmi AS, Mohammad RM. Non-peptidic small molecule inhibitors against Bcl-2 for cancer therapy. J Cell Physiol 2009;218:13-21.

37. Heere-Ress E, Thallinger C, Lucas T, et al. Bcl-X(L) is a chemoresistance factor in human melanoma cells that can be inhibited by antisense therapy. Int J Cancer 2002;99:29-34.

38. Hauschild A, Agarwala SS, Trefzer U, et al. Results of a phase III, randomized, placebo-controlled study of sorafenib in combination with carboplatin and paclitaxel as second-line treatment in patients with unresectable stage III or stage IV melanoma. J Clin Oncol 2009;27:2823-30.

39. Hodi FS, O'Day SJ, McDermott DF, et al. Improved survival with ipilimumab in patients with metastatic melanoma. N Engl J Med 2010;363:711-23.

40. Meyer S, Wild PJ, Vogt T, Bataille F, Ehret C, Gantner S, Landthaler M, Klinkhammer-Schalke M, Hofstaedter F, Bosserhoff AK. Methylthioadenosine phosphorylase represents a predictive marker for response to adjuvant interferon therapy in patients with malignant melanoma. Experimental Dermatology 2010; 19(8), e251-e257.

41. Bakshi RP et al 2001: Functional and regulatory characteristics of eukaryotic type II DNA topoisomease (review). Crit Rev Biochem Mol Biol. 36: 1-37.

42. Bettstetter M, Dechant S, Ruemmele P, Vogel C, Kurz K, Morak M, Keller G, Holinski-Feder E, Hofstaedter F, Dietmaier W. MethyQESD, a robust and fast method for quantitative methylation analyses in HNPCC diagnostics using formalin-fixed and paraffin-embedded tissue samples. Lab Invest. 2008 Dec;88(12):1367-75.

43. Mirmohammadsadegh A, Marini A, Nambiar S, Hassan M, Tannapfel A, Ruzicka T, Hengge UR. Epigenetic silencing of the PTEN gene in melanoma. Cancer Res. 2006 Jul 1;66(13):6546-52.

44. Lahtz C, Stranzenbach R, Fiedler E, Helmbold P, Dammann RH. Methylation of PTEN as a prognostic factor in malignant melanoma of the skin. J Invest Dermatol. 2010 Feb;130(2):620-2.

45. Zhou XP, Gimm O, Hampel H, Niemann T, Walker MJ, Eng C. Epigenetic PTEN silencing in malignant melanomas without PTEN mutation. Am J Pathol. 2000 Oct;157(4):1123-8.

46. Song L et al 2006: Identification and functional analysis of candidate genes regulating mesenchymal stem cell self-renewal and multipotency. Stem Cells. 24: 1707-18.

47. Weinstein, B and Joe, A. Oncogene Addiction. Cancer Res May 1, 2008 68; 3077

## Claims

1. A method of predicting the course of disease in a patient having a malignant melanoma, the method comprising determining in melanoma cells comprised in a sample obtained from said malignant melanoma the presence or amount of at least five biomarkers selected from the group comprising or consisting of MTAP, PTEN, Bax, Bcl-X, β-Catenin, CD20, Cox-2, CD49d and MLH1, wherein the absence or decreased amount of MTAP and β-Catenin and/or the presence or increased amount of PTEN, Bax, Bcl-X, CD20, Cox-2, CD49d and MLH1is associated with a disadvantageous course of disease.

2. The method according to claim 1, wherein the at least five biomarkers include PTEN and/or MTAP.

3. The method according to claim 1 or 2, wherein at least seven biomarkers are determined.

**4.** The method according to claim 3, wherein the at least 7 biomarkers are MTAP, PTEN, Bax, Bcl-X, β-Catenin, CD20 and Cox-2.

**5.** A method of preparing a tailored pharmaceutical composition for a patient having a malignant melanoma, the method comprising

(i) determining in melanoma cells comprised in a sample obtained from said malignant melanoma the presence or amount of at least five biomarkers selected from the group comprising or consisting of MTAP, PTEN, Bax, Bcl-X, β-Catenin, CD20, Cox-2, CD49d and MLH1, wherein the absence or decreased amount of β-Catenin and MTAP and/or the presence or increased amount of PTEN, Bax, Bcl-X, CD20, Cox-2, CD49d and MLH1is associated with a disadvantageous course of disease;
(ii) deriving a treatment regimen for the individual patient based on the presence or amount of markers determined in step (i); and
(iii) providing at least one pharmaceutical compound based on the treatment regimen derived in step (ii).

**6.** The method according to claim 5, wherein the at least five biomarkers include CD20, Cox-2 and/or MTAP.

**7.** The method according to claim 5 or 6, wherein at least seven biomarkers are determined.

**8.** The method according to claim 7, wherein the at least 7 biomarkers are MTAP, PTEN, Bax, Bcl-X, β-Catenin, CD20 and Cox-2.

**9.** The method according to any one of claims 1 to 8, wherein the sample is obtained from the primary tumour, a lymph node or a metastasis.

**10.** The method according to any one of claims 1 to 9, wherein the sample is a tissue sample, a blood sample or lymph.

**11.** The method according to any one of claims 1 to 10, wherein the presence or amount of the biomarkers is analysed by methods determining genetic or epigenetic modifications or transcriptional or protein levels or a combination thereof.

**12.** The method according to any one of claims 1 to 11, wherein the presence or amount of the biomarkers is determined by immunohistochemistry, mass spectrometry, Western Blot, Northern Blot, PCR, RNA in situ hybridisation or a combination thereof.

**13.** The method according to any one of claims 1 to 12, wherein the biomarker is protein.

**14.** A kit for predicting the course of disease in a patient having a malignant melanoma, the kit comprising:

(a) means for determining the presence or amount of the set of biomarkers as defined by any one of claims 1 to 4 in a sample obtained from said malignant melanoma,
(b) instructions how to use the kit.

**15.** A kit for deriving a treatment regimen for an individual patient having a malignant melanoma, the kit comprising:

(a) means for determining the presence or amount of the set of biomarkers as defined by step (i) of any one of claims 5 to 8 in a sample obtained from said malignant melanoma,
(b) instructions how to use the kit.

**16.** A pharmaceutical composition for use in treating or preventing malignant melanoma, wherein the pharmaceutical composition comprises (an) inhibitor(s) of CD20, Cox-2 or (an) agent(s) affecting MTAP signalling pathways and/or the oncogenic pathway addiction of PTEN.

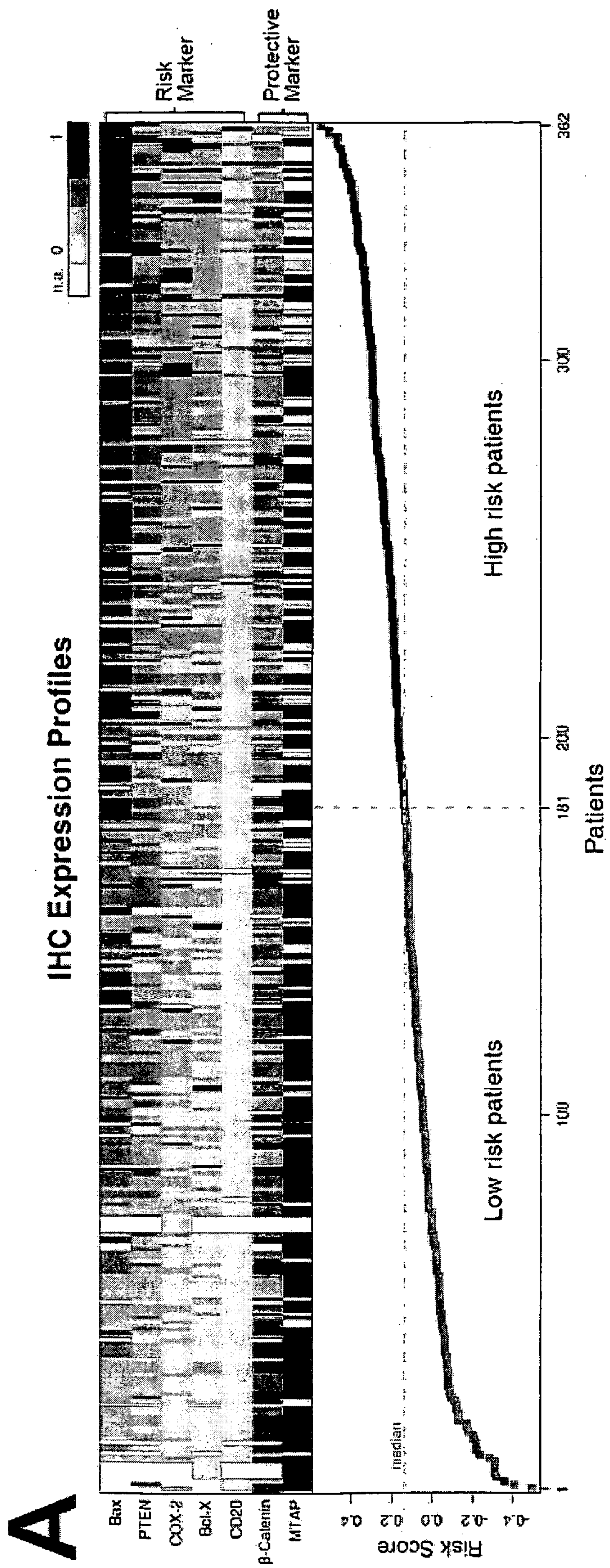
A
IHC Expression Profiles
n.a. 0 1
Bax
PTEN
COX-2
Bcl-X
CD20
β-Catenin
MTAP
Risk Marker
Protective Marker
Risk Score
0.4
0.2
0.0
-0.2
-0.4
median
Low risk patients
High risk patients
1
100
181
200
300
362
Patients

Figure 1

B

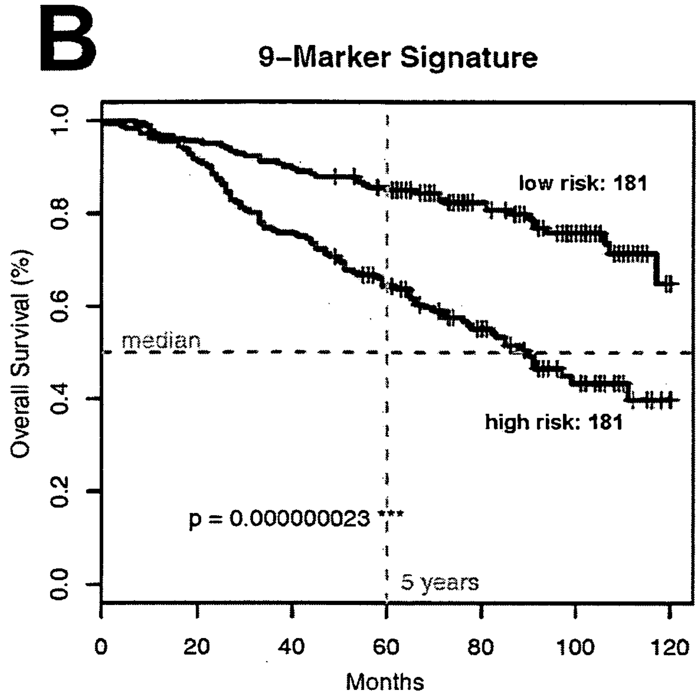


C

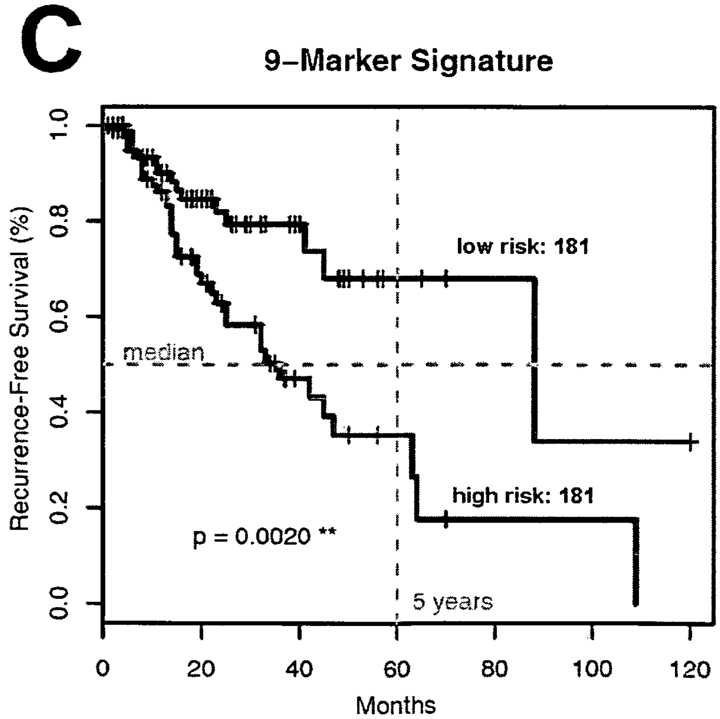


Figure 1 continued

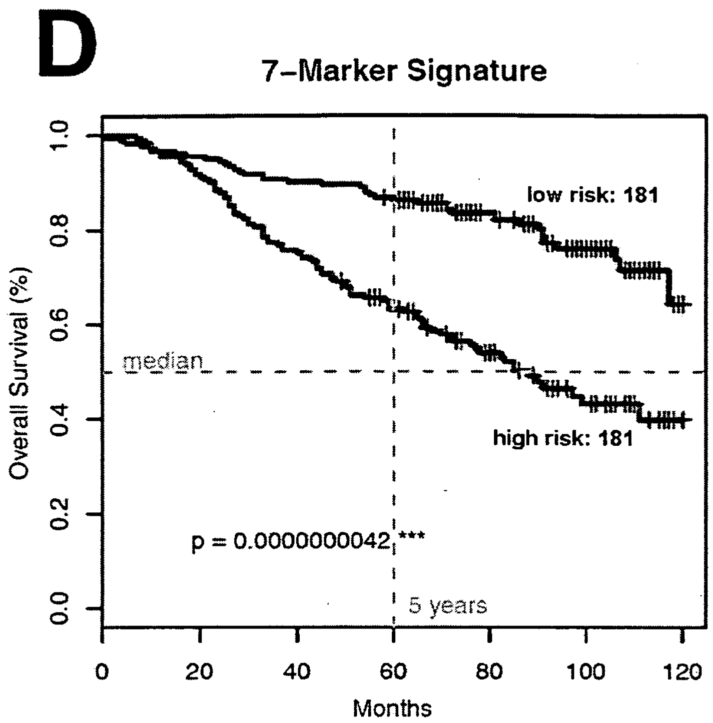


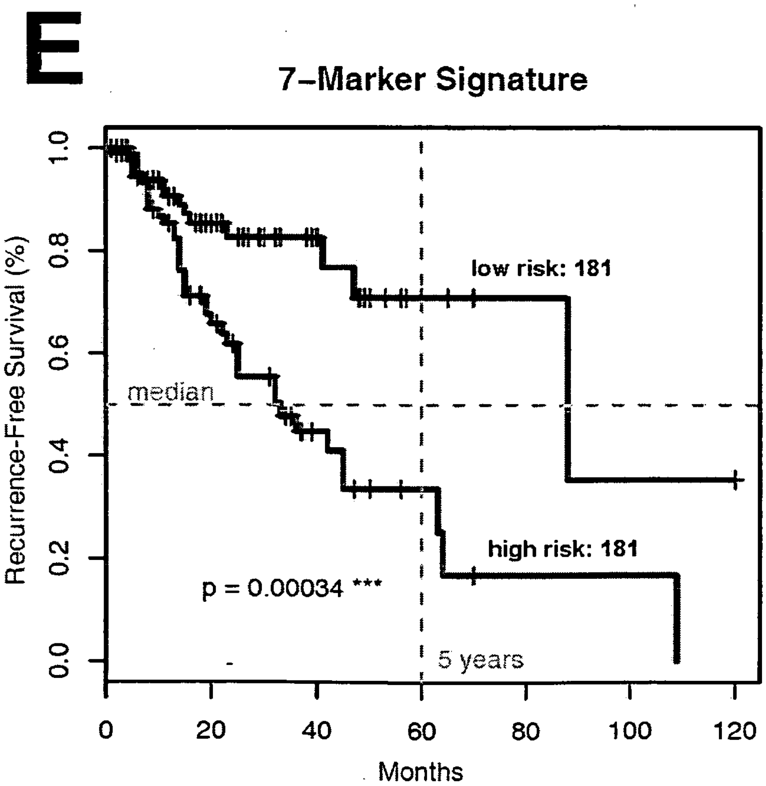


Figure 1 continued

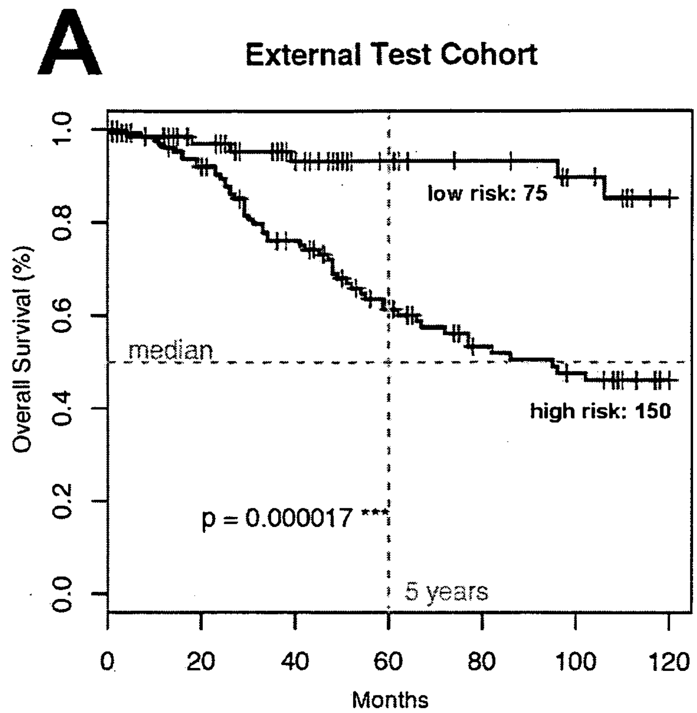
A
External Test Cohort
Overall Survival (%)
1.0
0.8
0.6
0.4
0.2
0.0
low risk: 75
median
high risk: 150
p = 0.000017 ***
5 years
0
20
40
60
80
100
120
Months

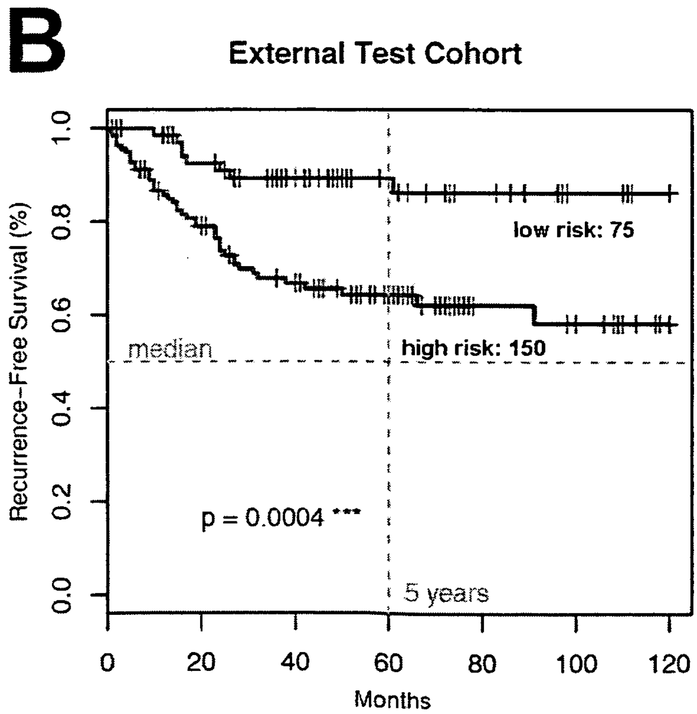
B
External Test Cohort
Recurrence-Free Survival (%)
1.0
0.8
0.6
0.4
0.2
0.0
low risk: 75
median
high risk: 150
p = 0.0004 ***
5 years
0
20
40
60
80
100
120
Months

Figure 2

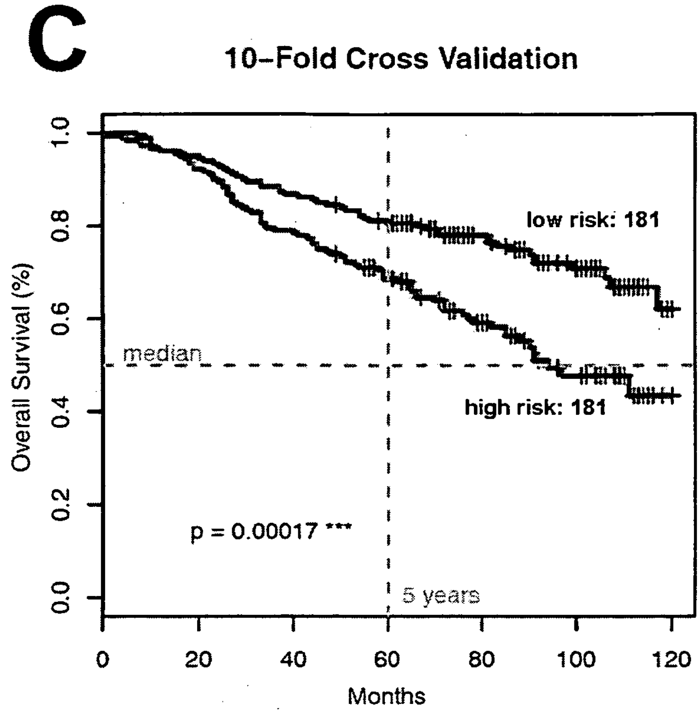


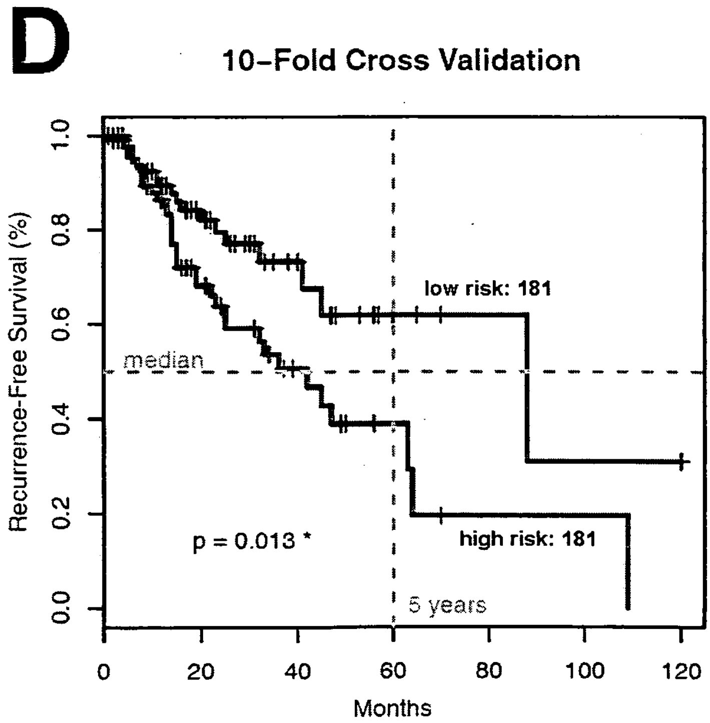


Figure 2 continued

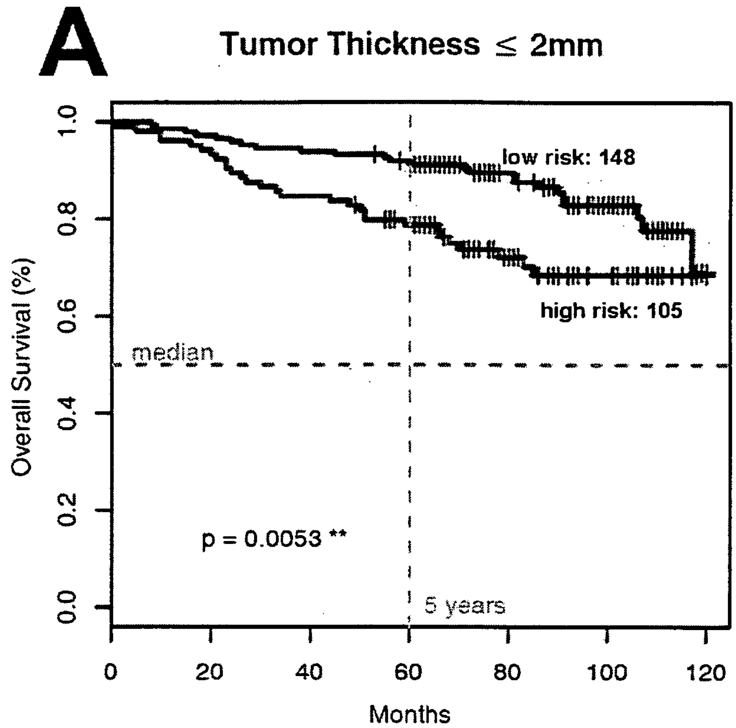
A
Tumor Thickness ≤ 2mm
Overall Survival (%)
1.0
0.8
0.6
0.4
0.2
0.0
low risk: 148
high risk: 105
median
p = 0.0053 **
5 years
0
20
40
60
80
100
120
Months

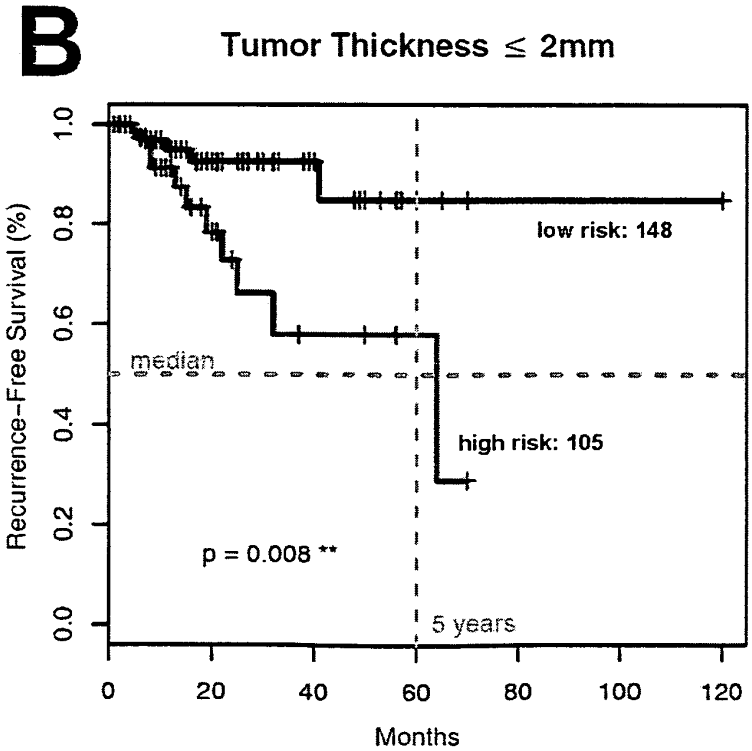
B
Tumor Thickness ≤ 2mm
Recurrence-Free Survival (%)
1.0
0.8
0.6
0.4
0.2
0.0
low risk: 148
median
high risk: 105
p = 0.008 **
5 years
0
20
40
60
80
100
120
Months

Figure 3

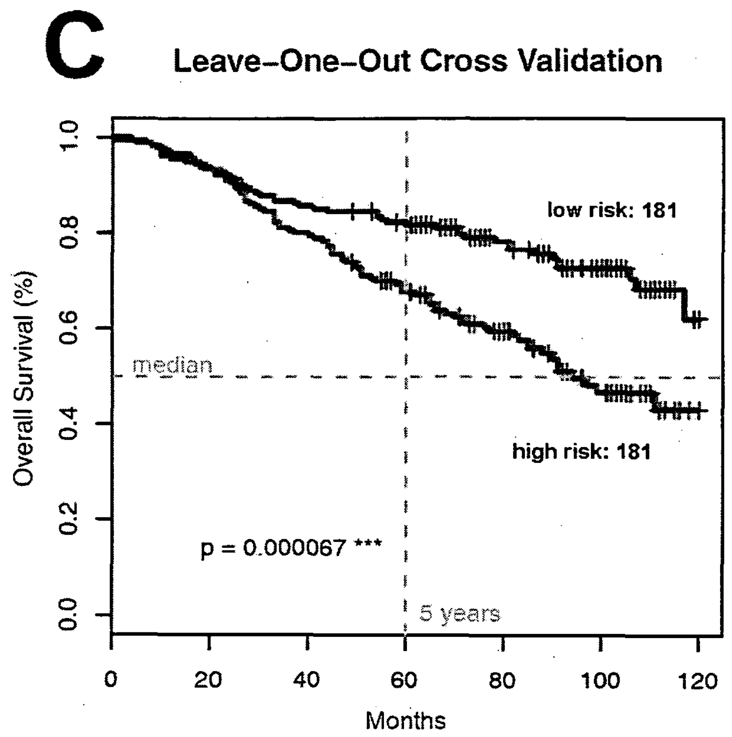


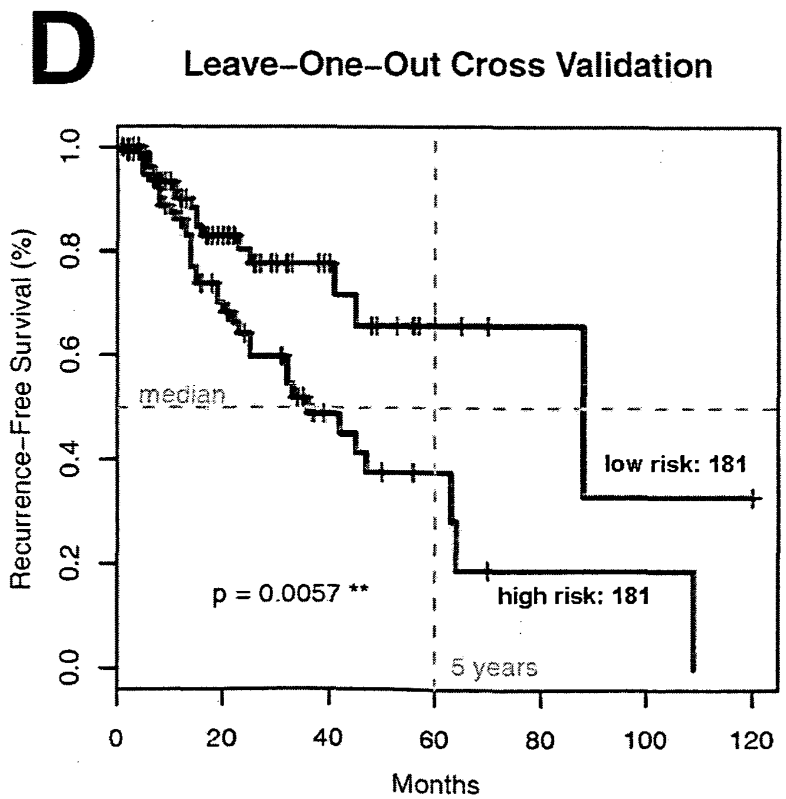


Figure 3 continued

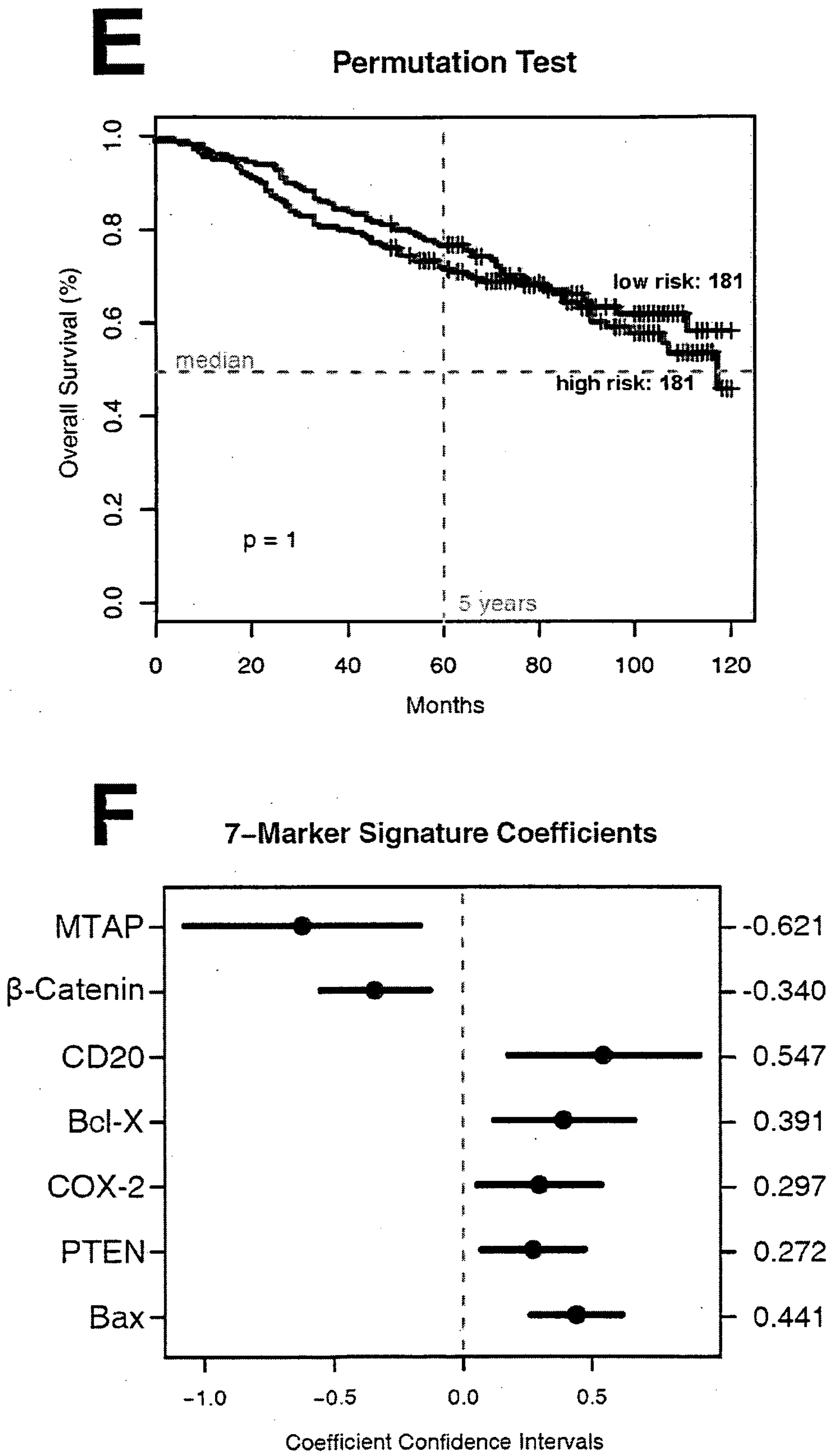


**Figure 3 continued**

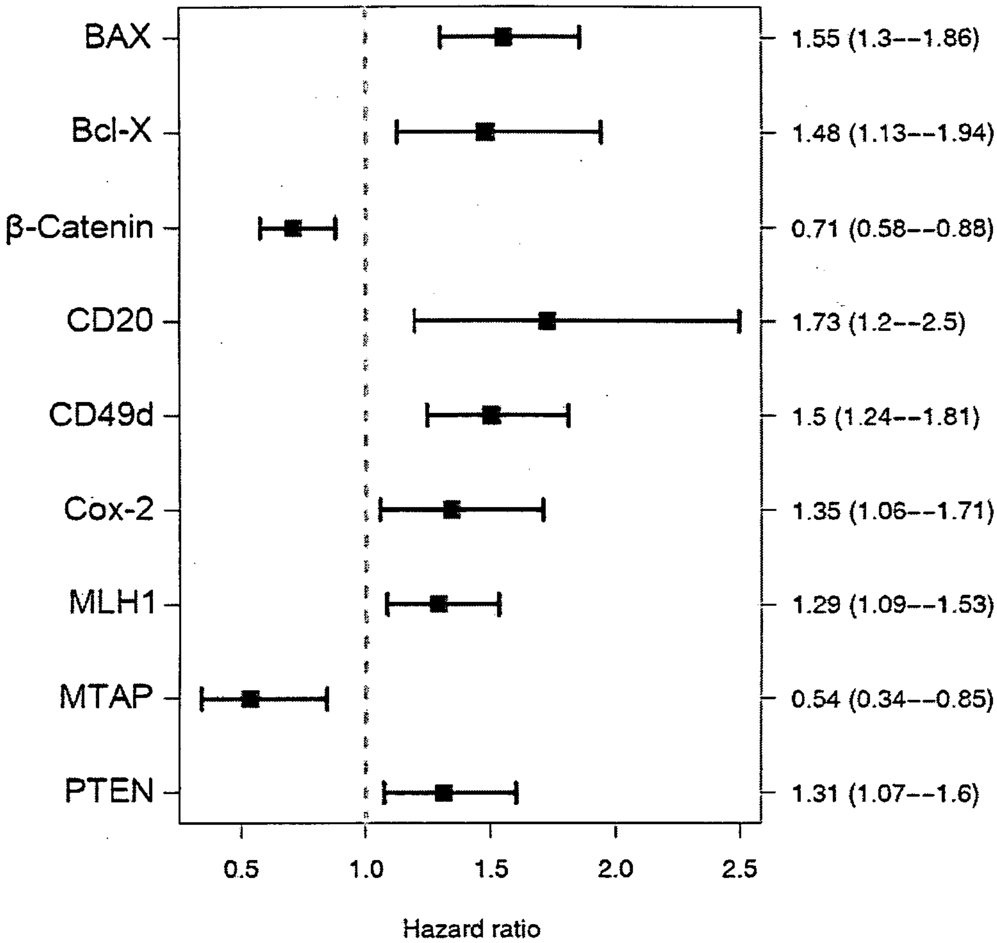
BAX
Bcl-X
β-Catenin
CD20
CD49d
Cox-2
MLH1
MTAP
PTEN
1.55 (1.3--1.86)
1.48 (1.13--1.94)
0.71 (0.58--0.88)
1.73 (1.2--2.5)
1.5 (1.24--1.81)
1.35 (1.06--1.71)
1.29 (1.09--1.53)
0.54 (0.34--0.85)
1.31 (1.07--1.6)
0.5
1.0
1.5
2.0
2.5
Hazard ratio

Figure 4

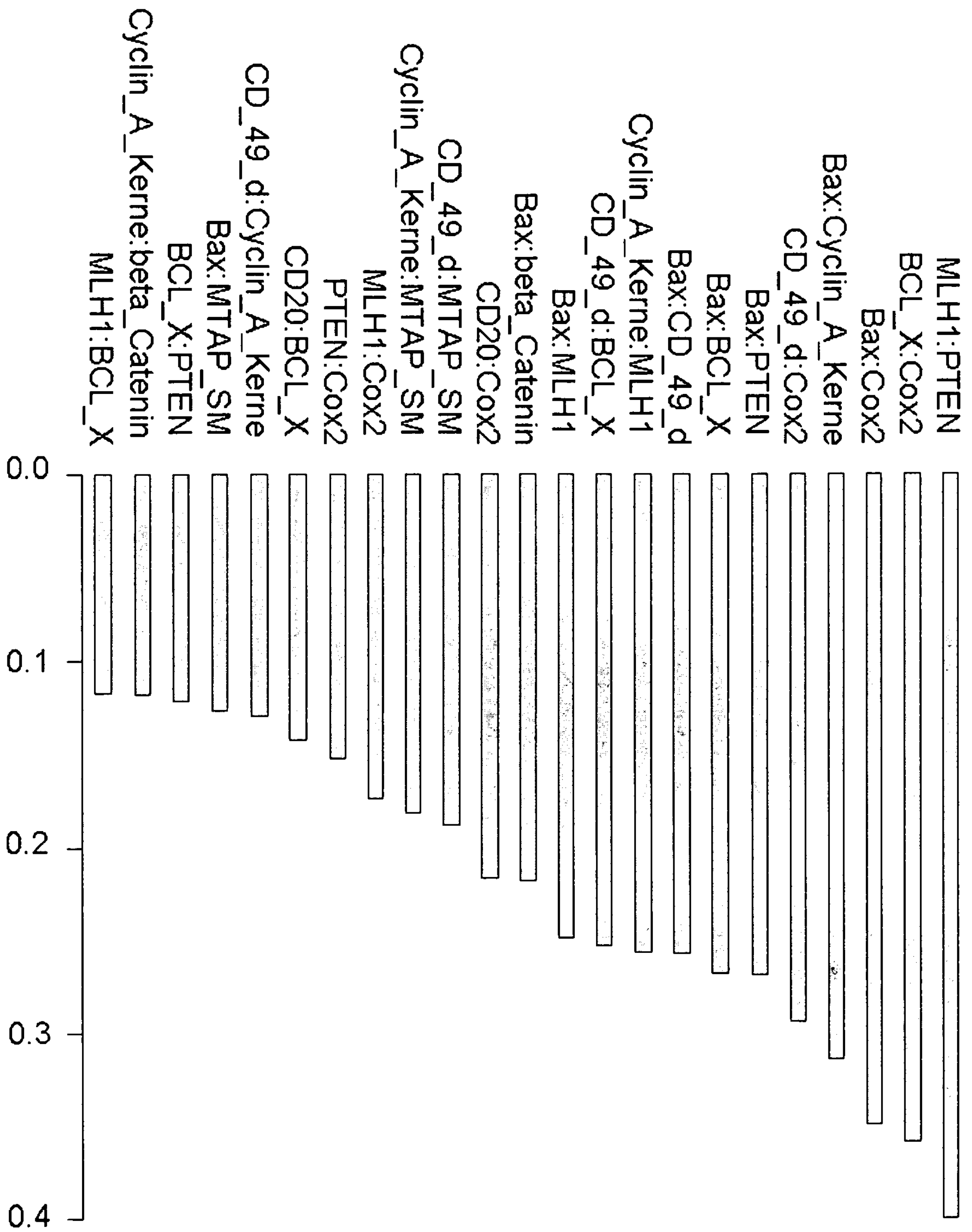
MLH1:BCL_X
Cyclin_A_Kerne:beta_Catenin
BCL_X:PTEN
Bax:MTAP_SM
CD_49_d:Cyclin_A_Kerne
CD20:BCL_X
PTEN:Cox2
MLH1:Cox2
Cyclin_A_Kerne:MTAP_SM
CD_49_d:MTAP_SM
CD20:Cox2
Bax:beta_Catenin
Bax:MLH1
CD_49_d:BCL_X
Cyclin_A_Kerne:MLH1
Bax:CD_49_d
Bax:BCL_X
Bax:PTEN
CD_49_d:Cox2
Bax:Cyclin_A_Kerne
Bax:Cox2
BCL_X:Cox2
MLH1:PTEN
0.0
0.1
0.2
0.3
0.4

Figure 5

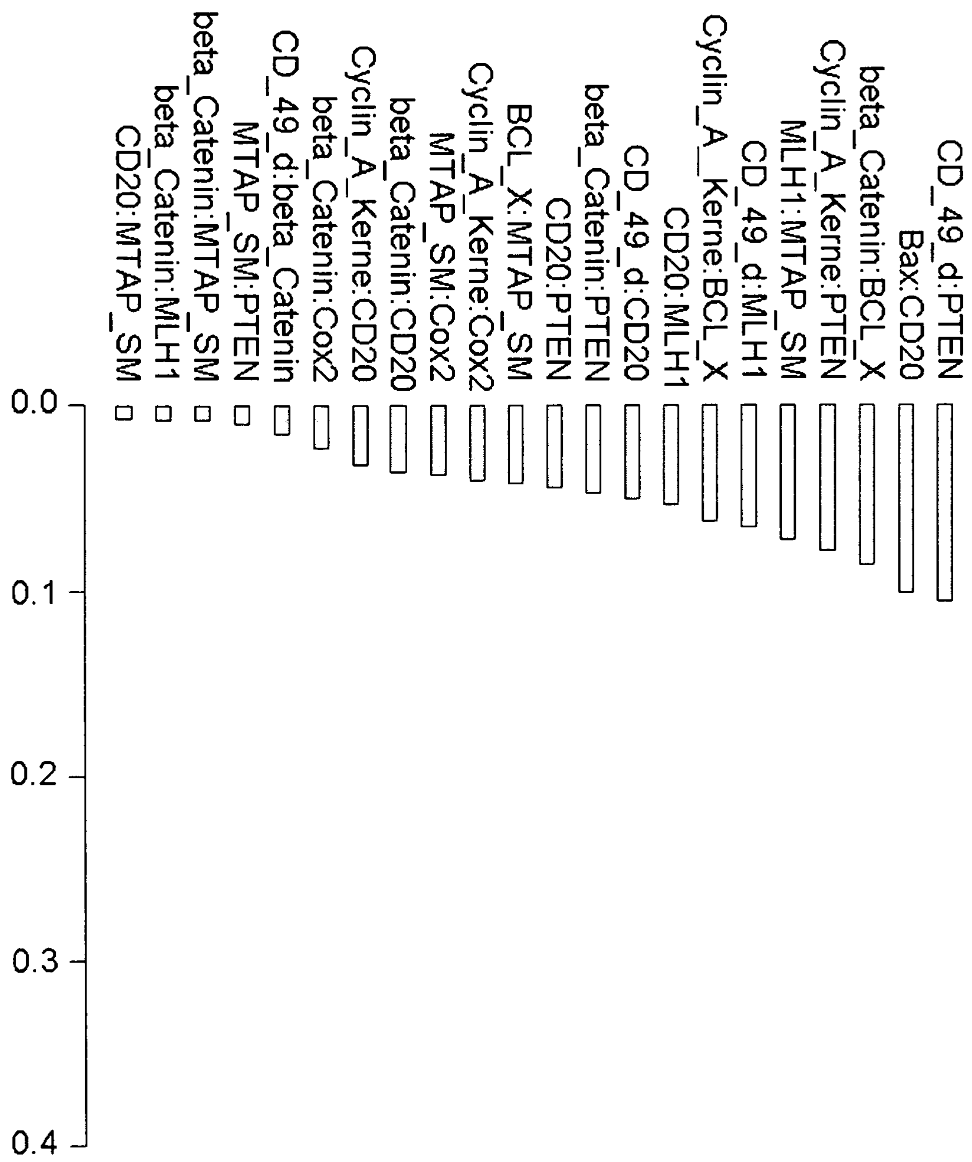


**Figure 5 continued**

Europäisches Patentamt
European Patent Office
Office européen des brevets

# EUROPEAN SEARCH REPORT

Application Number
EP 11 00 2747

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X<br><br>A | US 2007/099209 A1 (CLARKE MICHAEL F [US] ET AL) 3 May 2007 (2007-05-03)<br>* the whole document *<br>* In particular: paragraphs 244-246 for the kit in combination with tables 4-9; Table 4, p. 17 for Bcl-X (mentioned as BCL2L13) and p. 18 for CD20 (mentioned as MS4A2); Table 5,p. 21 for Cox-2 (mentioned as ACOX2) and p. 24 for CD20 (mentioned as MS4A2); Table 7b, p. 33 for PTEN, Cox-2 (mentioned as PTGS2) and for CD49 (mentioned as ITGA4); Table 7b, p. 34 for CD49 (mentioned as ITGA4); Table 7c, p. 35 for MTAP and p. 37 for Bcl-X (mentioned as BCL2L13); Table 7d p. 37-38 for PTEN, CD49 (mentioned as ITGA4), MLH1 and Bax. *<br>----- | 14,15<br><br>1-13 | INV.<br>G01N33/574 |
| X<br><br>A | US 2003/152923 A1 (YAKHINI ZOHAR [IL] ET AL) 14 August 2003 (2003-08-14)<br>* the whole document *<br>* In particular: Claims 1-20 *<br>----- | 1-3,5-7,9-13<br>4,8 | |
| | | | TECHNICAL FIELDS SEARCHED (IPC) |
| X<br><br><br>A | WO 2008/069881 A2 (SOURCE PREC MEDICINE INC [US]; UNIVERISTY OF COLORADO [US]; BANKAITIS-) 12 June 2008 (2008-06-12)<br>* the whole document *<br>* In particular: Claims 1-23. *<br>----- | 1-3,5-7,9-13<br><br>4,8 | G01N |

The present search report has been drawn up for all claims

1

| Place of search | Date of completion of the search | Examiner |
|---|---|---|
| The Hague | 10 August 2011 | C.F. Angioni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 00 2747

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2011

| Patent document cited in search report | Publication date | Patent family member(s) | Publication date |
|---|---|---|---|
| US 2007099209 A1 | 03-05-2007 | NONE | |
| US 2003152923 A1 | 14-08-2003 | NONE | |
| WO 2008069881 A2 | 12-06-2008 | AU 2007328427 A1 | 12-06-2008 |
| | | CA 2668831 A1 | 12-06-2008 |
| | | EP 2092075 A2 | 26-08-2009 |
| | | US 2010248225 A1 | 30-09-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008141275 A
- US 74612191 A
- US 4946778 A
- US 6080560 A
- US 5736137 A
- US 5595721 A
- US 5677180 A


### Non-patent literature cited in the description


- **GONZALGO et al.** *Cancer Res.,* 1997, vol. 57, 594-599
- **SINGER-SAM et al.** *Nucl. Acids Res.,* 1990, vol. 18, 687
- **FROMMER et al.** *PNAS,* 1992, vol. 89, 1827-1831
- **NEWTON et al.** *Nucleic Acids Res.,* 1989, vol. 17, 2503-2516
- Protocols for Oligonucleotides and Analogs: Synthesis and Properties (Methods in Molecular Biology, 20. Humana Press, 1993, vol. 20
- **HAQUE et al.** *Diagn. Mol. Pathol.,* 1998, vol. 7, 248-252
- PCR Applications: Protocols for Functional Genomics. Academic Press, 1999
- PCR Cloning Protocols: From Molecular Cloning to Genetic. Humana Press, 2002
- **PISSARD et al.** *Clin. Chem.,* 2002, vol. 48, 769-772
- **BLONDAL et al.** *Nucleic Acids Res,* 2003, vol. 31, e155
- **STEEMERS et al.** *Nature Meth.,* 2006, vol. 3, 31-33
- **KAKAVAS et al.** *J. Clin. Lab. Anal.,* 2006, vol. 20, 1-7
- Automated DNA Sequencing and Analysis. Academic Press, 1994
- **ALPHEY.** DNA Sequencing: From Experimental Methods to Bioinformatics. Springer Verlag Publishing, 1997
- **RAMON et al.** *J. Transl. Med.,* 2003, vol. 1, 9
- **MENG et al.** *J. Clin. Endocrinol. Metab.,* 2005, vol. 90, 3419-3422
- **BARNES et al.** *Nucleic Acids Res.,* 2005, vol. 33, 5914-5923
- **FAN et al.** *Biotechniques,* 2005, vol. 39, 583-588
- **SHEN et al.** *Mutat. Res.-Fund. Mol. M.,* 2005, vol. 573, 70-82
- **STEEMERS ; GUNDERSON.** *Pharmacogenomics,* 2005, vol. 6, 777-782
- **EISENBERG et al.** *Trends Genet,* 2003, vol. 19, 362-5
- **VELCULESCU et al.** *Nat Genet,* 1999, vol. 23, 387-8
- **STAFFORD.** *Methods in microarray Normalisation,* 2008, ISBN 13: 978-1420052787
- **QUAKENBUSH.** *Nat. Gene,* 2002, vol. 32, 496-501
- *J Natl Cancer Institute,* 2005, vol. 97, 1180-4
- **LAM et al.** *Nature,* 1991, vol. 354, 82-84
- **HOUGHTEN et al.** *Nature,* 1991, vol. 354, 84-86
- **SONGYANG et al.** *Cell,* 1993, vol. 72, 767-778
- **HARLOW ; LANE.** Antibodies, A Laboratory Manual. Cold Spring Harbor Laboratory Press, 1988
- **HARLOW ; LANE.** Using Antibodies: A Laboratory Manual. Cold Spring Harbor Laboratory Press, 1999
- **KÖHLER ; MILSTEIN.** *Nature,* 1975, vol. 256, 495-497
- **KOZBOR.** *Immunology Today,* 1983, vol. 4, 72
- **COLE et al.** Monoclonal Antibodies and Cancer Therapy. Alan R. Liss, Inc, 1985, 77-96
- **SCHIER.** *Human Antibodies Hybridomas,* 1996, vol. 7, 97-105
- **MALMBORG.** *J. Immunol. Methods,* 1995, vol. 183, 7-13
- **OSBORNE.** *Current Opinion in Chemical Biology,* 1997, vol. 1, 5-9
- **STULL ; SZOKA.** *Pharmaceutical Research,* 1995, vol. 12 (4), 465-483
- **FOUGEROLLES et al.** *Current Opinion in Pharmacology,* 2008, vol. 8, 280-285
- Delivering Small Interfering RNA for Novel Therapeutics. **LU et al.** Methods in Molecular Biology, vol. 437: Drug Delivery Systems. 2008, vol. 437
- **MELANI et al.** *Cancer Res.,* 1991, vol. 51, 2897-2901

- **KUBINYI.** Hausch-Analysis and Related Approaches. VCH Verlag, 1992
- **HOLZGRABE ; BECHTOLD.** *Deutsche Apotheker Zeitung,* 2000, vol. 140 (8), 813-823
- **PRESS et al.** *Blood,* 1987, vol. 69 (2), 584-591
- **VALENTINE et al.** Leukocyte Typing. Oxford University Press, 1987, vol. III, 440
- **ZAMORE.** *Nat. Struct. Biol.,* 2001, vol. 8 (9), 746
- **TUSCHL.** *Chembiochem.,* 2001, vol. 2 (4), 239
- **JEMAL A ; SIEGEL R ; WARD E et al.** *Cancer statistics, 2008. CA Cancer J Clin,* 2008, vol. 58, 71-96
- **LENS MB ; DAWES M.** Global perspectives of contemporary epidemiological trends of cutaneous malignant melanoma. *Br J Dermatol,* 2004, vol. 150, 179-85
- **BALCH CM ; BUZAID AC ; SOONG SJ et al.** Final version of the American Joint Committee on Cancer staging system for cutaneous melanoma. *J Clin Oncol,* 2001, vol. 19, 3635-48
- **AGARWALA SS.** Current systemic therapy for metastatic melanoma. *Expert Rev Anticancer Ther,* 2009, vol. 9, 587-95
- **BALCH CM ; GERSHENWALD JE ; SOONG SJ et al.** Final Version of 2009 AJCC Melanoma Staging and Classification. *J Clin Oncol,* 2009, vol. 27, 6199-206
- **BRESLOW A.** Thickness, cross-sectional areas, and depth of invasion in the prognosis of cutaneous melanoma. *Ann Surg,* 1970, vol. 172, 902-8
- **GRANDE SARPA H ; REINKE K ; SHAIKH L et al.** Prognostic significance of extent of ulceration in primary cutaneous melanoma. *Am J Surg Pathol,* 2006, vol. 30, 1396-400
- **MORTON DL ; THOMPSON JF ; COCHRAN AJ et al.** Sentinel-node biopsy or nodal observation in melanoma. *N Engl J Med,* 2006, vol. 355, 1307-17
- **GOULD ROTHBERG BE ; BRACKEN MB ; RIMM DL.** Tissue biomarkers for prognosis in cutaneous melanoma: a systematic review and meta-analysis. *J Natl Cancer Inst,* 2009, vol. 101, 452-74
- **GOULD ROTHBERG BE ; RIMM DL.** Biomarkers: The useful and the not so useful - an assessment of molecular prognostic markers for cutaneous melanoma. *J Invest Dermatol,* 2010, vol. 130, 1971-87
- Reporting recommendations for tumour marker prognostic studies (REMARK. *J Natl Cancer Institute,* 2005, vol. 97, 1180-4
- **ALONSO SR ; ORTIZ P ; POLLAN M et al.** Progression in cutaneous malignant melanoma is associated with distinct expression profiles: a tissue microarray-based study. *Am J Pathol,* 2004, vol. 164, 193-203
- **WILD PJ ; MEYER S ; BATAILLE F et al.** Tissue microarray analysis of MTAP expression in melanocytic skin tumours. *Arch Dermatol,* 2006, vol. 142, 471-6
- **MEYER S ; VOGT T ; LANDTHALER M et al.** Cyclooxygenase 2 (COX2) and Peroxisome Proliferator-Activated Receptor Gamma (PPARG) are stage-dependent prognostic markers of malignant melanoma. *PPAR Res,* 20 July 2009
- **ISHWARAN H ; KOGALUR UB ; BLACKSTONE EH ; LAUER MS.** Random survival forests. *Ann Appl Stat,* 2008, vol. 2, 841-60
- **HOTHORN T ; BUEHLMANN P ; DUDOIT S ; MOLINARO A ; VAN DER LAAN MJ.** Survival ensembles. *Biostatistics,* 2006, vol. 7, 355-73
- **COX DR.** Regression models and life-tables. *J R Stat Soc Series B,* 1972, vol. 34, 187-220
- **BENJAMINI Y ; HOCHBERG Y.** Controlling the false discovery rate: a practical and powerful approach to multiple testing. *J R Stat Soc Series B Stat Methodol,* 1995, vol. 57, 289-300
- **KAPLAN EL ; MEIER P.** Nonparametric estimation from incomplete observations. *J Amer Statist Assn,* 1958, vol. 53, 457-81
- **MANTEL N.** Evaluation of survival data and two new rank order statistics arising in its consideration. *Cancer Chemother Rep,* 1966, vol. 50, 163-70
- R: A language and environment for statistical computing. R Foundation for Statistical Computing, 2009
- **LOWE SW ; CEPERO E ; EVAN G.** Intrinsic tumour suppression. *Nature,* 2004, vol. 432, 307-15
- **DELMAS V ; BEERMANN F ; MARTINOZZI S et al.** Beta-catenin induces immortalization of melanocytes by suppressing p161NK4a expression and cooperates with N-Ras in melanoma development. *Genes Dev,* 2007, vol. 21, 2923-35
- **ZABIEROWSKI SE ; HERLYN M.** Melanoma stem cells: the dark seed of melanoma. *J Clin Oncol,* 2008, vol. 26, 2890-4
- **KUPHAL S ; BAUER R ; BOSSERHOFF AK.** Integrin signaling in malignant melanoma. *Cancer Metastasis Rev,* 2005, vol. 24, 195-222
- **KORABIOWSKA M ; BRINCK U ; STACHURA J et al.** Exonic deletions of mismatch repair genes MLH1 and MSH2 correlate with prognosis and protein expression levels in malignant melanomas. *Anticancer Res,* 2006, vol. 26, 1231-5
- **BEHRMANN I ; WALLNER S ; KOMYOD W et al.** Characterization of methylthioadenosine phosphorylase (MTAP) expression in malignant melanoma. *Am J Pathol,* 2003, vol. 163, 683-90
- **ZHANG S ; YU D.** PI(3)king apart PTEN's role in cancer. *Clin Cancer Res,* 2010, vol. 16, 4325-30
- **AVIVI I ; ROBINSON S ; GOLDSTONE A.** Clinical use of rituximab in haematological malignancies. *Br J Cancer,* 2003, vol. 89, 1389-94

- **HLA T ; NEILSON K.** Human cyclooxygenase-2 cDNA. *Proc Natl Acad Sci U S A,* 1992, vol. 89, 7384-8
- **DENKERT C ; KÖBEL M ; BERGER S et al.** Expression of cyclooxygenase 2 in human malignant melanoma. *Cancer Res,* 2001, vol. 61, 1733-40
- **THUN MJ ; HENLEY SJ ; GANSLER T.** Inflammation and cancer: an epidemiological perspective. *Novartis Found Symp,* 2004, vol. 256, 6-21
- **REICHLE A ; VOGT T ; CORAS B et al.** Targeted combined anti-inflammatory and angiostatic therapy in advanced melanoma: a randomized phase II trial. *Melanoma Res,* 2007, vol. 17, 360-4
- **ASCIERTO PA ; KIRKWOOD JM.** Adjuvant therapy of melanoma with interferon: lessons of the past decade. *J Transl Med,* 2008, vol. 6, 62
- **MEYER S ; WILD PJ ; VOGT T et al.** Methylthioadenosine phosphorylase represents a predictive marker for response to adjuvant interferon therapy in patients with malignant melanoma. *Exp Dermatol,* 2010, vol. 19, e251-7
- **AZMI AS ; MOHAMMAD RM.** Non-peptidic small molecule inhibitors against Bcl-2 for cancer therapy. *J Cell Physiol,* 2009, vol. 218, 13-21
- **HEERE-RESS E ; THALLINGER C ; LUCAS T et al.** Bcl-X(L) is a chemoresistance factor in human melanoma cells that can be inhibited by antisense therapy. *Int J Cancer,* 2002, vol. 99, 29-34
- **HAUSCHILD A ; AGARWALA SS ; TREFZER U et al.** Results of a phase III, randomized, placebo-controlled study of sorafenib in combination with carboplatin and paclitaxel as second-line treatment in patients with unresectable stage III or stage IV melanoma. *J Clin Oncol,* 2009, vol. 27, 2823-30
- **HODI FS ; O'DAY SJ ; MCDERMOTT DF et al.** Improved survival with ipilimumab in patients with metastatic melanoma. *N Engl J Med,* 2010, vol. 363, 711-23
- **MEYER S ; WILD PJ ; VOGT T ; BATAILLE F ; EHRET C ; GANTNER S ; LANDTHALER M ; KLINKHAMMER-SCHALKE M ; HOFSTAEDTER F ; BOSSERHOFF AK.** Methylthioadenosine phosphorylase represents a predictive marker for response to adjuvant interferon therapy in patients with malignant melanoma. *Experimental Dermatology,* 2010, vol. 19 (8), e251-e257
- **BAKSHI RP et al.** Functional and regulatory characteristics of eukaryotic type II DNA topoisomease (review. *Crit Rev Biochem Mol Biol.,* 2001, vol. 36, 1-37
- **BETTSTETTER M ; DECHANT S ; RUEMMELE P ; VOGEL C ; KURZ K ; MORAK M ; KELLER G ; HOLINSKI-FEDER E ; HOFSTAEDTER F ; DIETMAIER W.** MethyQESD, a robust and fast method for quantitative methylation analyses in HNPCC diagnostics using formalin-fixed and paraffin-embedded tissue samples. *Lab Invest.,* December 2008, vol. 88 (12), 1367-75
- **MIRMOHAMMADSADEGH A ; MARINI A ; NAMBIAR S ; HASSAN M ; TANNAPFEL A ; RUZICKA T ; HENGGE UR.** Epigenetic silencing of the PTEN gene in melanoma. *Cancer Res.,* 01 July 2006, vol. 66 (13), 6546-52
- **LAHTZ C ; STRANZENBACH R ; FIEDLER E ; HELMBOLD P ; DAMMANN RH.** Methylation of PTEN as a prognostic factor in malignant melanoma of the skin. *J Invest Dermatol.,* February 2010, vol. 130 (2), 620-2
- **ZHOU XP ; GIMM O ; HAMPEL H ; NIEMANN T ; WALKER MJ ; ENG C.** Epigenetic PTEN silencing in malignant melanomas without PTEN mutation. *Am J Pathol.,* October 2000, vol. 157 (4), 1123-8
- **SONG L et al.** Identification and functional analysis of candidate genes regulating mesenchymal stem cell self-renewal and multipotency. *Stem Cells.,* 2006, vol. 24, 1707-18
- **WEINSTEIN, B ; JOE, A.** Oncogene Addiction. *Cancer Res,* 01 May 2008, vol. 68, 3077

SEQUENCE LISTING

<110> Universität Regensburg

<120> A prognostic and therapeutic signature for malignant melanoma

<130> T1012 EP

<160> 22

<170> PatentIn version 3.5

<210> 1
<211> 4937
<212> DNA
<213> Homo sapiens

<400> 1
ctccgcactg ctcactcccg cgcagtgagg ttggcacagc caccgctctg tggctcgctt 60

ggttccctta gtcccgagcg ctcgcccact gcagattcct ttcccgtgca gacatggcct 120

ctggcaccac caccaccgcc gtgaagattg gaataattgg tggaacaggc ctggatgatc 180

cagaaatttt agaaggaaga actgaaaaat atgtggatac tccatttggc aagccatctg 240

atgccttaat tttggggaag ataaaaaatg ttgattgcgt cctccttgca aggcatggaa 300

ggcagcacac catcatgcct tcaaaggtca actaccaggc gaacatctgg gctttgaagg 360

aagagggctg tacacatgtc atagtgacca cagcttgtgg ctccttgagg gaggagattc 420

agcccggcga tattgtcatt attgatcagt tcattgacag gaccactatg agacctcagt 480

ccttctatga tggaagtcat tcttgtgcca gaggagtgtg ccatattcca atggctgagc 540

cgttttgccc caaaacgaga gaggttctta tagagactgc taagaagcta ggactccggt 600

gccactcaaa ggggacaatg gtcacaatcg agggacctcg ttttagctcc cgggcagaaa 660

gcttcatgtt ccgcacctgg ggggcggatg ttatcaacat gaccacagtt ccagaggtgg 720

ttcttgctaa ggaggctgga atttgttacg caagtatcgc catggcgaca gattatgact 780

gctggaagga gcacgaggaa gcagtttcgg tggaccgggt cttaaagacc ctgaaagaaa 840

acgctaataa agccaaaagc ttactgctca ctaccatacc tcagataggg tccacagaat 900

ggtcagaaac cctccataac ctgaagaata tggcccagtt ttctgtttta ttaccaagac 960

attaaagtag catggctgcc caggagaaaa gaagacattc taattccagt cattttggga 1020

attcctgctt aacttgaaaa aaatatggga aagacatgca gctttcatgc ccttgcctat 1080

caaagagtat gttgtaagaa agacaagaca ttgtgtgtat tagagactcc tgaatgattt 1140

agacaacttc aaaatacaga agaaaagcaa atgactagta aacatgtggg aaaaaatatt 1200

acattttaag gggaaaaaaa aaacccacca ttctcttctc cccctattaa atttgcaaca 1260

ataaagggtg gagggtaatc tctactttcc tatactgcca aagaatgtga ggaagaaatg 1320

ggactctttg gttatttatt gatgcgactg taaattggta cagtatttct ggagggcaat 1380

ttggtaaaat gcatcaaaag acttaaaaat acggacgtac tttgtgctgg gaactctaca 1440

tctagcaatt tctctttaaa accatatcag agatgcatac aaagaattat atataaagaa 1500

gggtgtttaa taatgatagt tataataata aataattgaa acaatctgaa tcccttgcaa 1560
ttggaggtaa attatgtctt agttataatt agattgtgaa tcagccaact gaaaatcctt 1620
tttgcatatt tcaatgtcct aaaaagacac ggttgctcta tatatgaagt gaaaaaagga 1680
tatggtagca ttttatagta ctagttttgc tttaaaatgc tatgtaaata tacaaaaaaa 1740
ctagaaagaa atatatataa ccttgttatt gtatttgggg gagggatact gggataattt 1800
ttattttctt tgaatctttc tgtgtcttca catttttcta cagtgaattt aatcaaatag 1860
taaagttgtt gtaaaaataa aagtggattt agaaagatcc agttcttgaa aacactgttt 1920
ctggtaatga agcagaattt aagttggtaa tattaaggtg aatgtcattt aagggagtta 1980
catctttatt ctgctaaaga agaggatcat tgatttctgt acagtcagaa cagtacttgg 2040
gtttgcaaca gctttctgag aaaagctagg tgtttaatag tttaactgaa agtttaacta 2100
tttaaaagac taaatgcaca ttttatggta tctgatattt taaaaagtaa tgtttgattc 2160
tcctttttat gagttaaatt attttatacg agttggtaat ttttgctttt taataaagtg 2220
gaagcttgct tttttaactc ttttttttatt gttattttat agaaatgctt tttgttggcc 2280
gggcacagtt gctcatccat gtaatcccag cactgtggga ggccgagacg ggtggatcac 2340
aaggtcagga gatcgagacc atcctggcta atgcgttgaa actccgtctc tactaaaaat 2400
acaaaaaatt agctgggcgt ggtggtgggc acctgtagtc ccagctactc aggaggctga 2460
ggcaggagaa tggtgtgaac ctgggaggtg gagcttgcag tgagcagagc ttgcagtgag 2520
acgagcttgt gccactgcac tccagcctgg gcaacagagt aagactcagt ctcaaaaaaa 2580
aaaaaaagag tgaaatgctt tttgtttgct tcagtttttt atcatgggga gatcttttc 2640
ctcagaattg ttttcttttc actgtaggct attacaggat acttcaggat caagatacag 2700
aaccttttat ttaaagagtt tgtaaagtca atgtgtttgt ttgtgtctct gagattgact 2760
tcaagataat aagctgctaa ttgtaaacaa aacagttacc ctccagtatt aatatgactc 2820
attagtgtga gccatttggg tcaagtatga ttatgaccct tggacttcct gatgtagtat 2880
taaatttcaa ctctggttat ccattagcaa tctgtagaga acttaatgaa cctgaaccca 2940
ggcttctcta gctctggtaa cgtgtgattg ttttcactac aatatgatac atagatggta 3000
ccttactttt cctcattctt aataggtgtc taagaatgtc agggcaaaag tatgggcatt 3060
tttcttgcta tgttcagaaa gtacagttct ctccaacttg cagaggtact tttcttgatt 3120
aaatagcctt ctctagcaac atcattttca gactaactaa atgaatgcag tatactcttt 3180
tctttgttct caatcattca ctccttatgc aaagccaata taattttcct cataccttat 3240
gcttgaggat attgttgaag aacacttcct ggaacacttc tcacttgtga tgctgtacta 3300
attttttttt tttaatttaa gctagtatac taagtgaaca ccatggtcag ttgtgagcat 3360
tttggtttcc gcaaaggatg gatggtgagc atcatgggaa agctgtagtt tagtgactta 3420
gcccttagtg attaatagat ttgcatgtac atagaagtct ttgttggcct tataatctgc 3480
tgttatattt ggcatggatt ttcatggttt tgagaatgac atcctggccc tgtggtcccc 3540

gagggtcatg gtccttgtga cctggcccct gttcactgcc cccttcgcta gcacgagttg 3600
ctgtgcaggg ctggaggtag ctaccatggc ttgtttcaag gaaggaaact ctggtacggt 3660
ggcaccctca ggagtggagg acagtgaact tccttgaaga gggagtgact aaggtgacct 3720
ccaacctgcc ctgagccagc tgccctgcag gtgccacgtg agcctgctct ggcatccaca 3780
ggatgctcct ggagcctctt ctctggctgc tacctcaggg catggttgtg gccccaccaa 3840
cacctatttt ccaaataatt attcattctt gtgacagtgg cctgaacatg tttttaattt 3900
tctcaacaag catttagcca gcacttatcc agtgaaacaa tttgataagg tttcaaggag 3960
tatctgatgg gttaggaagt cacgaaatga ggagttcttg ccacatttgc agagtccctc 4020
cttgataagg tttggcggtg tccccaccca aatctcatgt tgaattgtag ttcccataat 4080
ccccacatgt tgtgggaggg acccagtggg aggtaattaa atcatggggg tggttacccc 4140
cacactgctg ttctcatgat actgagttct cacaagtcct gtttgtttta taaggggctt 4200
ttcccccttt tgctcaacac ttcttcctgc catcatgtga agaaggacgt gtttgtttcc 4260
ccttctgcca cgattgtaag tttcctgagg ccttcccagc tatgtggaac tgtgagttaa 4320
ttaaacctct ttcctttata aattacccag tcatgggcag tcctttacag cagcatgaga 4380
atggactaat acactcctca aatgttttga agattgttgc accttggaac taccagtgtg 4440
cacacaatct ggctcaatgt atatattggc ccagcaaggc aaagaactga agttccagga 4500
tggaagaacc tgtgttctcc tcataatagt atagaataat tcaagatagg caagaaggac 4560
agcagtaaat gaagaccatg gaagaaaaga aggaatgcca aagatcgagg aaatctacca 4620
agactagtag ggtagtccag aagaagctgt ttcagggcct gttgccagct atgcctttga 4680
gaacctcggg atcccaaaga atgaggggaa tttcttcaga aagacaatct cggcatgcat 4740
tatttctttg ttttgaagat tcactcatgt tgcatgcatc tgtagcttgt gcctttttta 4800
ttgcctagta gtattctgtc atatgcctat cttacaattt gattatctat tcacctgttg 4860
atgaatgttt gaatttttc catttgagga attttatgaa taaagctgct ataagcatga 4920
aaaaaaaaaa aaaaaaa 4937

<210> 2
<211> 283
<212> PRT
<213> Homo sapiens

<400> 2

Met Ala Ser Gly Thr Thr Thr Thr Ala Val Lys Ile Gly Ile Ile Gly
1               5                   10                  15

Gly Thr Gly Leu Asp Asp Pro Glu Ile Leu Glu Gly Arg Thr Glu Lys
            20                  25                  30

Tyr Val Asp Thr Pro Phe Gly Lys Pro Ser Asp Ala Leu Ile Leu Gly
        35                  40                  45

```
Lys Ile Lys Asn Val Asp Cys Val Leu Leu Ala Arg His Gly Arg Gln
    50                  55                  60

His Thr Ile Met Pro Ser Lys Val Asn Tyr Gln Ala Asn Ile Trp Ala
65                  70                  75                  80

Leu Lys Glu Glu Gly Cys Thr His Val Ile Val Thr Thr Ala Cys Gly
                85                  90                  95

Ser Leu Arg Glu Glu Ile Gln Pro Gly Asp Ile Val Ile Ile Asp Gln
            100                 105                 110

Phe Ile Asp Arg Thr Thr Met Arg Pro Gln Ser Phe Tyr Asp Gly Ser
        115                 120                 125

His Ser Cys Ala Arg Gly Val Cys His Ile Pro Met Ala Glu Pro Phe
    130                 135                 140

Cys Pro Lys Thr Arg Glu Val Leu Ile Glu Thr Ala Lys Lys Leu Gly
145                 150                 155                 160

Leu Arg Cys His Ser Lys Gly Thr Met Val Thr Ile Glu Gly Pro Arg
                165                 170                 175

Phe Ser Ser Arg Ala Glu Ser Phe Met Phe Arg Thr Trp Gly Ala Asp
            180                 185                 190

Val Ile Asn Met Thr Thr Val Pro Glu Val Val Leu Ala Lys Glu Ala
        195                 200                 205

Gly Ile Cys Tyr Ala Ser Ile Ala Met Ala Thr Asp Tyr Asp Cys Trp
    210                 215                 220

Lys Glu His Glu Glu Ala Val Ser Val Asp Arg Val Leu Lys Thr Leu
225                 230                 235                 240

Lys Glu Asn Ala Asn Lys Ala Lys Ser Leu Leu Leu Thr Thr Ile Pro
                245                 250                 255

Gln Ile Gly Ser Thr Glu Trp Ser Glu Thr Leu His Asn Leu Lys Asn
            260                 265                 270

Met Ala Gln Phe Ser Val Leu Leu Pro Arg His
        275                 280

<210>   3
<211>   5572
<212>   DNA
<213>   Homo sapiens

<400>   3
cctcccctcg cccggcgcgg tcccgtccgc ctctcgctcg cctcccgcct cccctcggtc        60
```

```
ttccgaggcg cccgggctcc cggcgcggcg gcggaggggg cgggcaggcc ggcgggcggt     120
gatgtggcgg gactctttat gcgctgcggc aggatacgcg ctcggcgctg ggacgcgact     180
gcgctcagtt ctctcctctc ggaagctgca gccatgatgg aagtttgaga gttgagccgc     240
tgtgaggcga ggccgggctc aggcgaggga gatgagagac ggcggcggcc gcggcccgga     300
gcccctctca gcgcctgtga gcagccgcgg gggcagcgcc ctcggggagc cggccggcct     360
gcggcggcgg cagcggcggc gtttctcgcc tcctcttcgt cttttctaac cgtgcagcct     420
cttcctcggc ttctcctgaa agggaaggtg gaagccgtgg gctcgggcgg gagccggctg     480
aggcgcggcg gcggcggcgg cacctcccgc tcctggagcg ggggggagaa gcggcggcgg     540
cggcggccgc ggcggctgca gctccaggga gggggtctga gtcgcctgtc accatttcca     600
gggctgggaa cgccggagag ttggtctctc cccttctact gcctccaaca cggcggcggc     660
ggcggcggca catccaggga cccgggccgg ttttaaacct cccgtccgcc gccgccgcac     720
cccccgtggc ccgggctccg gaggccgccg gcggaggcag ccgttcggag gattattcgt     780
cttctcccca ttccgctgcc gccgctgcca ggcctctggc tgctgaggag aagcaggccc     840
agtcgctgca accatccagc agccgccgca gcagccatta cccggctgcg gtccagagcc     900
aagcggcggc agagcgaggg gcatcagcta ccgccaagtc cagagccatt tccatcctgc     960
agaagaagcc ccgccaccag cagcttctgc catctctctc ctcctttttc ttcagccaca    1020
ggctcccaga catgacagcc atcatcaaag agatcgttag cagaaacaaa aggagatatc    1080
aagaggatgg attcgactta gacttgacct atatttatcc aaacattatt gctatgggat    1140
ttcctgcaga aagacttgaa ggcgtataca ggaacaatat tgatgatgta gtaaggtttt    1200
tggattcaaa gcataaaaac cattacaaga tatacaatct ttgtgctgaa agacattatg    1260
acaccgccaa atttaattgc agagttgcac aatatccttt tgaagaccat aacccaccac    1320
agctagaact tatcaaaccc ttttgtgaag atcttgacca atggctaagt gaagatgaca    1380
atcatgttgc agcaattcac tgtaaagctg gaaagggacg aactggtgta atgatatgtg    1440
catatttatt acatcggggc aaatttttaa aggcacaaga ggccctagat ttctatgggg    1500
aagtaaggac cagagacaaa aagggagtaa ctattcccag tcagaggcgc tatgtgtatt    1560
attatagcta cctgttaaag aatcatctgg attatagacc agtggcactg ttgtttcaca    1620
agatgatgtt tgaaactatt ccaatgttca gtggcggaac ttgcaatcct cagtttgtgg    1680
tctgccagct aaaggtgaag atatattcct ccaattcagg acccacacga cgggaagaca    1740
agttcatgta ctttgagttc cctcagccgt tacctgtgtg tggtgatatc aaagtagagt    1800
tcttccacaa acagaacaag atgctaaaaa aggacaaaat gtttcacttt tgggtaaata    1860
cattcttcat accaggacca gaggaaacct cagaaaaagt agaaaatgga agtctatgtg    1920
atcaagaaat cgatagcatt tgcagtatag agcgtgcaga taatgacaag gaatatctag    1980
tacttacttt aacaaaaaat gatcttgaca aagcaaataa agacaaagcc aaccgatact    2040
tttctccaaa ttttaaggtg aagctgtact tcacaaaaac agtagaggag ccgtcaaatc    2100
```

cagaggctag cagttcaact tctgtaacac cagatgttag tgacaatgaa cctgatcatt 2160
atagatattc tgacaccact gactctgatc cagagaatga accttttgat gaagatcagc 2220
atacacaaat tacaaaagtc tgaatttttt tttatcaaga gggataaaac accatgaaaa 2280
taaacttgaa taaactgaaa atggaccttt tttttttaa tggcaatagg acattgtgtc 2340
agattaccag ttataggaac aattctcttt tcctgaccaa tcttgtttta ccctatacat 2400
ccacagggtt ttgacacttg ttgtccagtt gaaaaaaggt tgtgtagctg tgtcatgtat 2460
atacctttt gtgtcaaaag gacatttaaa attcaattag gattaataaa gatggcactt 2520
tcccgtttta ttccagtttt ataaaaagtg gagacagact gatgtgtata cgtaggaatt 2580
ttttcctttt gtgttctgtc accaactgaa gtggctaaag agctttgtga tatactggtt 2640
cacatcctac ccctttgcac ttgtggcaac agataagttt gcagttggct aagagaggtt 2700
tccgaagggt tttgctacat tctaatgcat gtattcgggt taggggaatg gagggaatgc 2760
tcagaaagga aataatttta tgctggactc tggaccatat accatctcca gctatttaca 2820
cacacctttc tttagcatgc tacagttatt aatctggaca ttcgaggaat tggccgctgt 2880
cactgcttgt tgtttgcgca ttttttttta aagcatattg gtgctagaaa aggcagctaa 2940
aggaagtgaa tctgtattgg ggtacaggaa tgaaccttct gcaacatctt aagatccaca 3000
aatgaaggga tataaaaata atgtcatagg taagaaacac agcaacaatg acttaaccat 3060
ataaatgtgg aggctatcaa caaagaatgg gcttgaaaca ttataaaaat tgacaatgat 3120
ttattaaata tgttttctca attgtaacga cttctccatc tcctgtgtaa tcaaggccag 3180
tgctaaaatt cagatgctgt tagtacctac atcagtcaac aacttacact tattttacta 3240
gttttcaatc ataatacctg ctgtggatgc ttcatgtgct gcctgcaagc ttcttttttc 3300
tcattaaata taaaatattt tgtaatgctg cacagaaatt ttcaatttga gattctacag 3360
taagcgtttt ttttctttga agatttatga tgcacttatt caatagctgt cagccgttcc 3420
accctttga ccttacacat tctattacaa tgaattttgc agttttgcac attttttaaa 3480
tgtcattaac tgttagggaa ttttacttga atactgaata catataatgt ttatattaaa 3540
aaggacattt gtgttaaaaa ggaaattaga gttgcagtaa actttcaatg ctgcacacaa 3600
aaaaaagaca tttgattttt cagtagaaat tgtcctacat gtgctttatt gatttgctat 3660
tgaaagaata gggttttttt tttttttttt tttttttttt ttaaatgtgc agtgttgaat 3720
catttcttca tagtgctccc ccgagttggg actagggctt caatttcact tcttaaaaaa 3780
aatcatcata tatttgatat gcccagactg catacgattt taagcggagt acaactacta 3840
ttgtaaagct aatgtgaaga tattattaaa aaggtttttt tttccagaaa tttggtgtct 3900
tcaaattata ccttcacctt gacatttgaa tatccagcca ttttgtttct taatggtata 3960
aaattccatt ttcaataact tattggtgct gaaattgttc actagctgtg gtctgaccta 4020
gttaatttac aaatacagat tgaataggac ctactagagc agcatttata gagtttgatg 4080
gcaaatagat taggcagaac ttcatctaaa atattcttag taaataatgt tgacacgttt 4140

```
tccatacctt gtcagtttca ttcaacaatt tttaaatttt taacaaagct cttaggattt      4200
acacatttat atttaaacat tgatatatag agtattgatt gattgctcat aagttaaatt      4260
ggtaaagtta gagacaacta ttctaacacc tcaccattga aatttatatg ccaccttgtc      4320
tttcataaaa gctgaaaatt gttacctaaa atgaaaatca acttcatgtt ttgaagatag      4380
ttataaatat tgttctttgt tacaatttcg ggcaccgcat attaaaacgt aactttattg      4440
ttccaatatg taacatggag ggccaggtca taaataatga cattataatg ggcttttgca      4500
ctgttattat ttttcctttg gaatgtgaag gtctgaatga gggttttgat tttgaatgtt      4560
tcaatgtttt tgagaagcct tgcttacatt ttatggtgta gtcattggaa atggaaaaat      4620
ggcattatat atattatata tataaatata tattatacat actctcctta ctttatttca      4680
gttaccatcc ccatagaatt tgacaagaat tgctatgact gaaaggtttt cgagtcctaa      4740
ttaaaacttt atttatggca gtattcataa ttagcctgaa atgcattctg taggtaatct      4800
ctgagtttct ggaatatttt cttagacttt ttggatgtgc agcagcttac atgtctgaag      4860
ttacttgaag gcatcacttt taagaaagct tacagttggg ccctgtacca tcccaagtcc      4920
tttgtagctc ctcttgaaca tgtttgccat acttttaaaa gggtagttga ataaatagca      4980
tcaccattct ttgctgtggc acaggttata aacttaagtg gagtttaccg gcagcatcaa      5040
atgtttcagc tttaaaaaat aaaagtaggg tacaagttta atgtttagtt ctagaaattt      5100
tgtgcaatat gttcataacg atggctgtgg ttgccacaaa gtgcctcgtt tacctttaaa      5160
tactgttaat gtgtcatgca tgcagatgga aggggtggaa ctgtgcacta aagtgggggc      5220
tttaactgta gtatttggca gagttgcctt ctacctgcca gttcaaaagt tcaacctgtt      5280
ttcatataga atatatatac taaaaaattt cagtctgtta aacagcctta ctctgattca      5340
gcctcttcag atactcttgt gctgtgcagc agtggctctg tgtgtaaatg ctatgcactg      5400
aggatacaca aaaataccaa tatgatgtgt acaggataat gcctcatccc aatcagatgt      5460
ccatttgtta ttgtgtttgt taacaaccct ttatctctta gtgttataaa ctccacttaa      5520
aactgattaa agtctcattc ttgtcaaaaa aaaaaaaaaa aaaaaaaaaa aa              5572
```

<210> 4
<211> 403
<212> PRT
<213> Homo sapiens

<400> 4

```
Met Thr Ala Ile Ile Lys Glu Ile Val Ser Arg Asn Lys Arg Arg Tyr
1               5                   10                  15

Gln Glu Asp Gly Phe Asp Leu Asp Leu Thr Tyr Ile Tyr Pro Asn Ile
            20                  25                  30

Ile Ala Met Gly Phe Pro Ala Glu Arg Leu Glu Gly Val Tyr Arg Asn
        35                  40                  45
```

```
Asn Ile Asp Asp Val Val Arg Phe Leu Asp Ser Lys His Lys Asn His
    50                  55                  60

Tyr Lys Ile Tyr Asn Leu Cys Ala Glu Arg His Tyr Asp Thr Ala Lys
65                  70                  75                  80

Phe Asn Cys Arg Val Ala Gln Tyr Pro Phe Glu Asp His Asn Pro Pro
                85                  90                  95

Gln Leu Glu Leu Ile Lys Pro Phe Cys Glu Asp Leu Asp Gln Trp Leu
            100                 105                 110

Ser Glu Asp Asp Asn His Val Ala Ala Ile His Cys Lys Ala Gly Lys
        115                 120                 125

Gly Arg Thr Gly Val Met Ile Cys Ala Tyr Leu Leu His Arg Gly Lys
    130                 135                 140

Phe Leu Lys Ala Gln Glu Ala Leu Asp Phe Tyr Gly Glu Val Arg Thr
145                 150                 155                 160

Arg Asp Lys Lys Gly Val Thr Ile Pro Ser Gln Arg Arg Tyr Val Tyr
                165                 170                 175

Tyr Tyr Ser Tyr Leu Leu Lys Asn His Leu Asp Tyr Arg Pro Val Ala
            180                 185                 190

Leu Leu Phe His Lys Met Met Phe Glu Thr Ile Pro Met Phe Ser Gly
        195                 200                 205

Gly Thr Cys Asn Pro Gln Phe Val Val Cys Gln Leu Lys Val Lys Ile
    210                 215                 220

Tyr Ser Ser Asn Ser Gly Pro Thr Arg Arg Glu Asp Lys Phe Met Tyr
225                 230                 235                 240

Phe Glu Phe Pro Gln Pro Leu Pro Val Cys Gly Asp Ile Lys Val Glu
                245                 250                 255

Phe Phe His Lys Gln Asn Lys Met Leu Lys Lys Asp Lys Met Phe His
            260                 265                 270

Phe Trp Val Asn Thr Phe Phe Ile Pro Gly Pro Glu Glu Thr Ser Glu
        275                 280                 285

Lys Val Glu Asn Gly Ser Leu Cys Asp Gln Glu Ile Asp Ser Ile Cys
    290                 295                 300

Ser Ile Glu Arg Ala Asp Asn Asp Lys Glu Tyr Leu Val Leu Thr Leu
305                 310                 315                 320
```

Thr Lys Asn Asp Leu Asp Lys Ala Asn Lys Asp Lys Ala Asn Arg Tyr
325 330 335

Phe Ser Pro Asn Phe Lys Val Lys Leu Tyr Phe Thr Lys Thr Val Glu
340 345 350

Glu Pro Ser Asn Pro Glu Ala Ser Ser Ser Thr Ser Val Thr Pro Asp
355 360 365

Val Ser Asp Asn Glu Pro Asp His Tyr Arg Tyr Ser Asp Thr Thr Asp
370 375 380

Ser Asp Pro Glu Asn Glu Pro Phe Asp Glu Asp Gln His Thr Gln Ile
385 390 395 400

Thr Lys Val

<210> 5
<211> 741
<212> DNA
<213> Homo sapiens

<400> 5
tcacgtgacc cgggcgcgct gcggccgccc gcgcggaccc ggcgagaggc ggcggcggga 60
gcggcggtga tggacgggtc cggggagcag cccagaggcg gggggcccac cagctctgag 120
cagatcatga agacaggggc ccttttgctt caggggatga ttgccgccgt ggacacagac 180
tccccccgag aggtcttttt ccgagtggca gctgacatgt tttctgacgg caacttcaac 240
tggggccggg ttgtcgccct tttctacttt gccagcaaac tggtgctcaa ggccctgtgc 300
accaaggtgc cggaactgat cagaaccatc atgggctgga cattggactt cctccgggag 360
cggctgttgg gctggatcca agaccagggt ggttgggacg gcctcctctc ctactttggg 420
acgcccacgt ggcagaccgt gaccatcttt gtggcgggag tgctcaccgc ctcactcacc 480
atctggaaga agatgggctg aggcccccag ctgccttgga ctgtgttttt cctccataaa 540
ttatggcatt tttctgggag gggtggggat tgggggacgt gggcattttt cttacttttg 600
taattattgg ggggtgtggg gaagagtggt cttgaggggg taataaacct ccttcgggac 660
acaaaaaaaa aaaaaaaaaa aaaaaaaaaa aaaaaaaaaa aaaaaaaaaa aaaaaaaaaa 720
aaaaaaaaaa aaaaaaaaaa a 741

<210> 6
<211> 143
<212> PRT
<213> Homo sapiens

<400> 6

Met Asp Gly Ser Gly Glu Gln Pro Arg Gly Gly Gly Pro Thr Ser Ser
1 5 10 15

Glu Gln Ile Met Lys Thr Gly Ala Leu Leu Leu Gln Gly Met Ile Ala
20 25 30

Ala Val Asp Thr Asp Ser Pro Arg Glu Val Phe Phe Arg Val Ala Ala
35 40 45

Asp Met Phe Ser Asp Gly Asn Phe Asn Trp Gly Arg Val Val Ala Leu
50 55 60

Phe Tyr Phe Ala Ser Lys Leu Val Leu Lys Ala Leu Cys Thr Lys Val
65 70 75 80

Pro Glu Leu Ile Arg Thr Ile Met Gly Trp Thr Leu Asp Phe Leu Arg
85 90 95

Glu Arg Leu Leu Gly Trp Ile Gln Asp Gln Gly Gly Trp Asp Gly Leu
100 105 110

Leu Ser Tyr Phe Gly Thr Pro Thr Trp Gln Thr Val Thr Ile Phe Val
115 120 125

Ala Gly Val Leu Thr Ala Ser Leu Thr Ile Trp Lys Lys Met Gly
130 135 140

<210> 7
<211> 2575
<212> DNA
<213> Homo sapiens

<400> 7
ggaggaggaa gcaagcgagg gggctggttc ctgagcttcg caattcctgt gtcgccttct 60
gggctcccag cctgccgggt cgcatgatcc ctccggccgg agctggtttt tttgccagcc 120
accgcgaggc cggctgagtt accggcatcc ccgcagccac ctcctctccc gacctgtgat 180
acaaaagatc ttccgggggc tgcacctgcc tgcctttgcc taaggcggat ttgaatctct 240
ttctctccct tcagaatctt atcttggctt tggatcttag aagagaatca ctaaccagag 300
acgagactca gtgagtgagc aggtgttttg gacaatggac tggttgagcc catccctatt 360
ataaaaatgt ctcagagcaa ccgggagctg gtggttgact ttctctccta caagctttcc 420
cagaaaggat acagctggag tcagtttagt gatgtggaag agaacaggac tgaggcccca 480
gaagggactg aatcggagat ggagaccccc agtgccatca atggcaaccc atcctggcac 540
ctggcagaca gccccgcggt gaatggagcc actggccaca gcagcagttt ggatgcccgg 600
gaggtgatcc ccatggcagc agtaaagcaa gcgctgaggg aggcaggcga cgagtttgaa 660
ctgcggtacc ggcgggcatt cagtgacctg acatcccagc tccacatcac cccagggaca 720
gcatatcaga gctttgaaca ggtagtgaat gaactcttcc gggatggggt aaactggggt 780
cgcattgtgg cctttttctc cttcggcggg gcactgtgcg tggaaagcgt agacaaggag 840
atgcaggtat tggtgagtcg gatcgcagct tggatggcca cttacctgaa tgaccaccta 900

gagccttgga tccaggagaa cggcggctgg gatactttg tggaactcta tgggaacaat 960
gcagcagccg agagccgaaa gggccaggaa cgcttcaacc gctggttcct gacgggcatg 1020
actgtggccg gcgtggttct gctgggctca ctcttcagtc ggaaatgacc agacactgac 1080
catccactct accctcccac ccccttctct gctccaccac atcctccgtc cagccgccat 1140
tgccaccagg agaaccacta catgcagccc atgcccacct gcccatcaca gggttgggcc 1200
cagatctggt cccttgcagc tagttttcta gaatttatca cacttctgtg agacccccac 1260
acctcagttc ccttggcctc agaattcaca aaatttccac aaaatctgtc caaaggaggc 1320
tggcaggtat ggaagggttt gtggctgggg gcaggagggc cctacctgat tggtgcaacc 1380
cttacccctt agcctccctg aaaatgtttt tctgccaggg agcttgaaag ttttcagaac 1440
ctcttcccca gaaaggagac tagattgcct ttgttttgat gtttgtggcc tcagaattga 1500
tcattttccc cccactctcc ccacactaac ctgggttccc tttccttcca tccctacccc 1560
ctaagagcca tttaggggcc acttttgact agggattcag gctgcttggg ataaagatgc 1620
aaggaccagg actccctcct cacctctgga ctggctagag tcctcactcc cagtccaaat 1680
gtcctccaga agcctctggc tagaggccag ccccacccag gagggagggg gctatagcta 1740
caggaagcac cccatgccaa agctagggtg gcccttgcag ttcagcacca ccctagtccc 1800
ttcccctccc tggctcccat gaccatactg agggaccaac tgggcccaag acagatgccc 1860
cagagctgtt tatggcctca gctgcctcac ttcctacaag agcagcctgt ggcatctttg 1920
ccttgggctg ctcctcatgg tgggttcagg ggactcagcc ctgaggtgaa agggagctat 1980
caggaacagc tatgggagcc ccagggtctt ccctacctca ggcaggaagg gcaggaagga 2040
gagcctgctg catggggtgg ggtagggctg actagaaggg ccagtcctgc ctggccaggc 2100
agatctgtgc cccatgcctg tccagcctgg gcagccaggc tgccaaggcc agagtggcct 2160
ggccaggagc tcttcaggcc tccctctctc ttctgctcca cccttggcct gtctcatccc 2220
caggggtccc agccaccccg ggctctctgc tgtacatatt tgagactagt ttttattcct 2280
tgtgaagatg atatactatt tttgttaagc gtgtctgtat ttatgtgtga ggagctgctg 2340
gcttgcagtg cgcgtgcacg tggagagctg gtgcccggag attggacggc ctgatgctcc 2400
ctcccctgcc ctggtccagg gaagctggcc gagggtcctg gctcctgagg ggcatctgcc 2460
cctcccccaa cccccacccc acacttgttc cagctctttg aaatagtctg tgtgaaggtg 2520
aaagtgcagt tcagtaataa actgtgttta ctcagtgaaa aaaaaaaaaa aaaaa 2575

<210> 8
<211> 233
<212> PRT
<213> Homo sapiens

<400> 8

Met Ser Gln Ser Asn Arg Glu Leu Val Val Asp Phe Leu Ser Tyr Lys
1 5 10 15

```
Leu Ser Gln Lys Gly Tyr Ser Trp Ser Gln Phe Ser Asp Val Glu Glu
            20                  25                  30

Asn Arg Thr Glu Ala Pro Glu Gly Thr Glu Ser Glu Met Glu Thr Pro
        35                  40                  45

Ser Ala Ile Asn Gly Asn Pro Ser Trp His Leu Ala Asp Ser Pro Ala
    50                  55                  60

Val Asn Gly Ala Thr Gly His Ser Ser Ser Leu Asp Ala Arg Glu Val
65                  70                  75                  80

Ile Pro Met Ala Ala Val Lys Gln Ala Leu Arg Glu Ala Gly Asp Glu
                85                  90                  95

Phe Glu Leu Arg Tyr Arg Arg Ala Phe Ser Asp Leu Thr Ser Gln Leu
            100                 105                 110

His Ile Thr Pro Gly Thr Ala Tyr Gln Ser Phe Glu Gln Val Val Asn
        115                 120                 125

Glu Leu Phe Arg Asp Gly Val Asn Trp Gly Arg Ile Val Ala Phe Phe
    130                 135                 140

Ser Phe Gly Gly Ala Leu Cys Val Glu Ser Val Asp Lys Glu Met Gln
145                 150                 155                 160

Val Leu Val Ser Arg Ile Ala Ala Trp Met Ala Thr Tyr Leu Asn Asp
                165                 170                 175

His Leu Glu Pro Trp Ile Gln Glu Asn Gly Gly Trp Asp Thr Phe Val
            180                 185                 190

Glu Leu Tyr Gly Asn Asn Ala Ala Ala Glu Ser Arg Lys Gly Gln Glu
        195                 200                 205

Arg Phe Asn Arg Trp Phe Leu Thr Gly Met Thr Val Ala Gly Val Val
    210                 215                 220

Leu Leu Gly Ser Leu Phe Ser Arg Lys
225                 230
```

<210> 9
<211> 3256
<212> DNA
<213> Homo sapiens

<400> 9

```
aggatacagc ggcttctgcg cgacttataa gagctccttg tgcggcgcca ttttaagcct      60

ctcggtctgt ggcagcagcg ttggcccggc cccgggagcg gagagcgagg ggaggcggag     120

acggaggaag gtctgaggag cagcttcagt ccccgccgag ccgccaccgc aggtcgagga     180
```

```
cggtcggact cccgcggcgg gaggagcctg ttcccctgag ggtatttgaa gtataccata     240
caactgtttt gaaaatccag cgtggacaat ggctactcaa gctgatttga tggagttgga     300
catggccatg gaaccagaca gaaaagcggc tgttagtcac tggcagcaac agtcttacct     360
ggactctgga atccattctg gtgccactac cacagctcct tctctgagtg gtaaaggcaa     420
tcctgaggaa gaggatgtgg atacctccca agtcctgtat gagtgggaac agggattttc     480
tcagtccttc actcaagaac aagtagctga tattgatgga cagtatgcaa tgactcgagc     540
tcagagggta cgagctgcta tgttccctga gacattagat gagggcatgc agatcccatc     600
tacacagttt gatgctgctc atcccactaa tgtccagcgt ttggctgaac catcacagat     660
gctgaaacat gcagttgtaa acttgattaa ctatcaagat gatgcagaac ttgccacacg     720
tgcaatccct gaactgacaa aactgctaaa tgacgaggac caggtggtgg ttaataaggc     780
tgcagttatg gtccatcagc tttctaaaaa ggaagcttcc agacacgcta tcatgcgttc     840
tcctcagatg gtgtctgcta ttgtacgtac catgcagaat acaaatgatg tagaaacagc     900
tcgttgtacc gctgggacct tgcataacct ttcccatcat cgtgagggct tactggccat     960
ctttaagtct ggaggcattc ctgccctggt gaaaatgctt ggttcaccag tggattctgt    1020
gttgttttat gccattacaa ctctccacaa ccttttatta catcaagaag gagctaaaat    1080
ggcagtgcgt ttagctggtg ggctgcagaa aatggttgcc ttgctcaaca aaacaaatgt    1140
taaattcttg gctattacga cagactgcct tcaaatttta gcttatggca accaagaaag    1200
caagctcatc atactggcta gtggtggacc ccaagcttta gtaaatataa tgaggaccta    1260
tacttacgaa aaactactgt ggaccacaag cagagtgctg aaggtgctat ctgtctgctc    1320
tagtaataag ccggctattg tagaagctgg tggaatgcaa gctttaggac ttcacctgac    1380
agatccaagt caacgtcttg ttcagaactg tctttggact ctcaggaatc tttcagatgc    1440
tgcaactaaa caggaaggga tggaaggtct ccttgggact cttgttcagc ttctgggttc    1500
agatgatata aatgtggtca cctgtgcagc tggaattctt tctaacctca cttgcaataa    1560
ttataagaac aagatgatgg tctgccaagt gggtggtata gaggctcttg tgcgtactgt    1620
ccttcgggct ggtgacaggg aagacatcac tgagcctgcc atctgtgctc ttcgtcatct    1680
gaccagccga caccaagaag cagagatggc ccagaatgca gttcgccttc actatggact    1740
accagttgtg gttaagctct tacacccacc atcccactgg cctctgataa aggctactgt    1800
tggattgatt cgaaatcttg ccctttgtcc cgcaaatcat gcacctttgc gtgagcaggg    1860
tgccattcca cgactagttc agttgcttgt tcgtgcacat caggataccc agcgccgtac    1920
gtccatgggt gggacacagc agcaatttgt ggagggggtc cgcatggaag aaatagttga    1980
aggttgtacc ggagcccttc acatcctagc tcgggatgtt cacaaccgaa ttgttatcag    2040
aggactaaat accattccat tgtttgtgca gctgctttat tctcccattg aaaacatcca    2100
aagagtagct gcaggggtcc tctgtgaact tgctcaggac aaggaagctg cagaagctat    2160
tgaagctgag ggagccacag ctcctctgac agagttactt cactctagga atgaaggtgt    2220
```

ggcgacatat gcagctgctg ttttgttccg aatgtctgag gacaagccac aagattacaa 2280
gaaacggctt tcagttgagc tgaccagctc tctcttcaga acagagccaa tggcttggaa 2340
tgagactgct gatcttggac ttgatattgg tgcccaggga gaacccccttg gatatcgcca 2400
ggatgatcct agctatcgtt cttttcactc tggtggatat ggccaggatg ccttgggtat 2460
ggaccccatg atggaacatg agatgggtgg ccaccaccct ggtgctgact atccagttga 2520
tgggctgcca gatctggggc atgcccagga cctcatggat gggctgcctc caggtgacag 2580
caatcagctg gcctggtttg atactgacct gtaaatcatc ctttaggagt aacaatacaa 2640
atggattttg ggagtgactc aagaagtgaa gaatgcacaa gaatggatca caagatggaa 2700
tttatcaaac cctagccttg cttgttaaat tttttttttt tttttttaa gaatatctgt 2760
aatggtactg actttgcttg ctttgaagta gctctttttt tttttttttt tttttttttg 2820
cagtaactgt tttttaagtc tctcgtagtg ttaagttata gtgaatactg ctacagcaat 2880
ttctaatttt taagaattga gtaatggtgt agaacactaa ttcataatca ctctaattaa 2940
ttgtaatctg aataaagtgt aacaattgtg tagccttttt gtataaaata gacaaataga 3000
aaatggtcca attagtttcc tttttaatat gcttaaaata agcaggtgga tctatttcat 3060
gtttttgatc aaaaactatt tgggatatgt atgggtaggg taaatcagta agaggtgtta 3120
tttggaacct tgttttggac agtttaccag ttgcctttta tcccaaagtt gttgtaacct 3180
gctgtgatac gatgcttcaa gagaaaatgc ggttataaaa aatggttcag aattaaactt 3240
ttaattcatt cgattg 3256

<210> 10
<211> 781
<212> PRT
<213> Homo sapiens

<400> 10

Met Ala Thr Gln Ala Asp Leu Met Glu Leu Asp Met Ala Met Glu Pro
1 5 10 15

Asp Arg Lys Ala Ala Val Ser His Trp Gln Gln Gln Ser Tyr Leu Asp
20 25 30

Ser Gly Ile His Ser Gly Ala Thr Thr Thr Ala Pro Ser Leu Ser Gly
35 40 45

Lys Gly Asn Pro Glu Glu Glu Asp Val Asp Thr Ser Gln Val Leu Tyr
50 55 60

Glu Trp Glu Gln Gly Phe Ser Gln Ser Phe Thr Gln Glu Gln Val Ala
65 70 75 80

Asp Ile Asp Gly Gln Tyr Ala Met Thr Arg Ala Gln Arg Val Arg Ala
85 90 95

Ala Met Phe Pro Glu Thr Leu Asp Glu Gly Met Gln Ile Pro Ser Thr
100 105 110

Gln Phe Asp Ala Ala His Pro Thr Asn Val Gln Arg Leu Ala Glu Pro
115 120 125

Ser Gln Met Leu Lys His Ala Val Val Asn Leu Ile Asn Tyr Gln Asp
130 135 140

Asp Ala Glu Leu Ala Thr Arg Ala Ile Pro Glu Leu Thr Lys Leu Leu
145 150 155 160

Asn Asp Glu Asp Gln Val Val Val Asn Lys Ala Ala Val Met Val His
165 170 175

Gln Leu Ser Lys Lys Glu Ala Ser Arg His Ala Ile Met Arg Ser Pro
180 185 190

Gln Met Val Ser Ala Ile Val Arg Thr Met Gln Asn Thr Asn Asp Val
195 200 205

Glu Thr Ala Arg Cys Thr Ala Gly Thr Leu His Asn Leu Ser His His
210 215 220

Arg Glu Gly Leu Leu Ala Ile Phe Lys Ser Gly Gly Ile Pro Ala Leu
225 230 235 240

Val Lys Met Leu Gly Ser Pro Val Asp Ser Val Leu Phe Tyr Ala Ile
245 250 255

Thr Thr Leu His Asn Leu Leu Leu His Gln Glu Gly Ala Lys Met Ala
260 265 270

Val Arg Leu Ala Gly Gly Leu Gln Lys Met Val Ala Leu Leu Asn Lys
275 280 285

Thr Asn Val Lys Phe Leu Ala Ile Thr Thr Asp Cys Leu Gln Ile Leu
290 295 300

Ala Tyr Gly Asn Gln Glu Ser Lys Leu Ile Ile Leu Ala Ser Gly Gly
305 310 315 320

Pro Gln Ala Leu Val Asn Ile Met Arg Thr Tyr Thr Tyr Glu Lys Leu
325 330 335

Leu Trp Thr Thr Ser Arg Val Leu Lys Val Leu Ser Val Cys Ser Ser
340 345 350

Asn Lys Pro Ala Ile Val Glu Ala Gly Gly Met Gln Ala Leu Gly Leu
355 360 365

His Leu Thr Asp Pro Ser Gln Arg Leu Val Gln Asn Cys Leu Trp Thr
370 375 380

Leu Arg Asn Leu Ser Asp Ala Ala Thr Lys Gln Glu Gly Met Glu Gly
385 390 395 400

Leu Leu Gly Thr Leu Val Gln Leu Leu Gly Ser Asp Asp Ile Asn Val
405 410 415

Val Thr Cys Ala Ala Gly Ile Leu Ser Asn Leu Thr Cys Asn Asn Tyr
420 425 430

Lys Asn Lys Met Met Val Cys Gln Val Gly Gly Ile Glu Ala Leu Val
435 440 445

Arg Thr Val Leu Arg Ala Gly Asp Arg Glu Asp Ile Thr Glu Pro Ala
450 455 460

Ile Cys Ala Leu Arg His Leu Thr Ser Arg His Gln Glu Ala Glu Met
465 470 475 480

Ala Gln Asn Ala Val Arg Leu His Tyr Gly Leu Pro Val Val Val Lys
485 490 495

Leu Leu His Pro Pro Ser His Trp Pro Leu Ile Lys Ala Thr Val Gly
500 505 510

Leu Ile Arg Asn Leu Ala Leu Cys Pro Ala Asn His Ala Pro Leu Arg
515 520 525

Glu Gln Gly Ala Ile Pro Arg Leu Val Gln Leu Leu Val Arg Ala His
530 535 540

Gln Asp Thr Gln Arg Arg Thr Ser Met Gly Gly Thr Gln Gln Gln Phe
545 550 555 560

Val Glu Gly Val Arg Met Glu Glu Ile Val Glu Gly Cys Thr Gly Ala
565 570 575

Leu His Ile Leu Ala Arg Asp Val His Asn Arg Ile Val Ile Arg Gly
580 585 590

Leu Asn Thr Ile Pro Leu Phe Val Gln Leu Leu Tyr Ser Pro Ile Glu
595 600 605

Asn Ile Gln Arg Val Ala Ala Gly Val Leu Cys Glu Leu Ala Gln Asp
610 615 620

Lys Glu Ala Ala Glu Ala Ile Glu Ala Glu Gly Ala Thr Ala Pro Leu
625 630 635 640

```
Thr Glu Leu Leu His Ser Arg Asn Glu Gly Val Ala Thr Tyr Ala Ala
                645                 650                 655

Ala Val Leu Phe Arg Met Ser Glu Asp Lys Pro Gln Asp Tyr Lys Lys
            660                 665                 670

Arg Leu Ser Val Glu Leu Thr Ser Ser Leu Phe Arg Thr Glu Pro Met
        675                 680                 685

Ala Trp Asn Glu Thr Ala Asp Leu Gly Leu Asp Ile Gly Ala Gln Gly
    690                 695                 700

Glu Pro Leu Gly Tyr Arg Gln Asp Asp Pro Ser Tyr Arg Ser Phe His
705                 710                 715                 720

Ser Gly Gly Tyr Gly Gln Asp Ala Leu Gly Met Asp Pro Met Met Glu
                725                 730                 735

His Glu Met Gly Gly His His Pro Gly Ala Asp Tyr Pro Val Asp Gly
            740                 745                 750

Leu Pro Asp Leu Gly His Ala Gln Asp Leu Met Asp Gly Leu Pro Pro
        755                 760                 765

Gly Asp Ser Asn Gln Leu Ala Trp Phe Asp Thr Asp Leu
    770                 775                 780
```

<210> 11
<211> 1146
<212> DNA
<213> Homo sapiens

<400> 11

```
cctcaatgac actcatggag gaaatgctga gagaagcatt cagatgcatg acacaaggta      60
agactgccaa aaatcttgtt cttgctctcc tcattttgtt atttgtttta tttttaggag     120
ttttgagagc aaaatgacaa cacccagaaa ttcagtaaat gggactttcc cggcagagcc     180
aatgaaaggc cctattgcta tgcaatctgg tccaaaacca ctcttcagga ggatgtcttc     240
actggtgggc cccacgcaaa gcttcttcat gagggaatct aagactttgg gggctgtcca     300
gattatgaat gggctcttcc acattgccct ggggggtctt ctgatgatcc cagcagggat     360
ctatgcaccc atctgtgtga ctgtgtggta ccctctctgg ggaggcatta tgtatattat     420
ttccggatca ctcttggcag caacggagaa aaactctagg aagtgtttgg tcaaaggaaa     480
aatgataatg aattcattga gcctctttgc tgccatttct ggaatgattc tttcaatcat     540
ggacatactt aatattaaaa tttcccattt tttaaaaatg gagagtctga attttattag     600
agctcacaca ccatatatta acatatacaa ctgtgaacca gctaatccct ctgagaaaaa     660
ctccccatct acccaatact gttacagcat acaatctctg ttcttgggca ttttgtcagt     720
gatgctgatc tttgccttct tccaggaact tgtaatagct ggcatcgttg agaatgaatg     780
```

gaaaagaacg tgctccagac ccaaatctaa catagttctc ctgtcagcag aagaaaaaaa 840

agaacagact attgaaataa aagaagaagt ggttgggcta actgaaacat cttcccaacc 900

aaagaatgaa gaagacattg aaattattcc aatccaagaa gaggaagaag aagaaacaga 960

gacgaacttt ccagaacctc cccaagatca ggaatcctca ccaatagaaa atgacagctc 1020

tccttaagtg atttcttctg ttttctgttt cctttttta acattagtgt tcatagcttc 1080

caagagacat gctgactttc atttcttgag gtactctgca catacgcacc acatctctat 1140

ctggcc 1146

<210> 12
<211> 297
<212> PRT
<213> Homo sapiens

<400> 12

Met Thr Thr Pro Arg Asn Ser Val Asn Gly Thr Phe Pro Ala Glu Pro
1               5                   10                  15

Met Lys Gly Pro Ile Ala Met Gln Ser Gly Pro Lys Pro Leu Phe Arg
            20                  25                  30

Arg Met Ser Ser Leu Val Gly Pro Thr Gln Ser Phe Phe Met Arg Glu
        35                  40                  45

Ser Lys Thr Leu Gly Ala Val Gln Ile Met Asn Gly Leu Phe His Ile
    50                  55                  60

Ala Leu Gly Gly Leu Leu Met Ile Pro Ala Gly Ile Tyr Ala Pro Ile
65                  70                  75                  80

Cys Val Thr Val Trp Tyr Pro Leu Trp Gly Gly Ile Met Tyr Ile Ile
                85                  90                  95

Ser Gly Ser Leu Leu Ala Ala Thr Glu Lys Asn Ser Arg Lys Cys Leu
            100                 105                 110

Val Lys Gly Lys Met Ile Met Asn Ser Leu Ser Leu Phe Ala Ala Ile
        115                 120                 125

Ser Gly Met Ile Leu Ser Ile Met Asp Ile Leu Asn Ile Lys Ile Ser
    130                 135                 140

His Phe Leu Lys Met Glu Ser Leu Asn Phe Ile Arg Ala His Thr Pro
145                 150                 155                 160

Tyr Ile Asn Ile Tyr Asn Cys Glu Pro Ala Asn Pro Ser Glu Lys Asn
                165                 170                 175

Ser Pro Ser Thr Gln Tyr Cys Tyr Ser Ile Gln Ser Leu Phe Leu Gly
            180                 185                 190

Ile Leu Ser Val Met Leu Ile Phe Ala Phe Phe Gln Glu Leu Val Ile
195 200 205

Ala Gly Ile Val Glu Asn Glu Trp Lys Arg Thr Cys Ser Arg Pro Lys
210 215 220

Ser Asn Ile Val Leu Leu Ser Ala Glu Glu Lys Lys Glu Gln Thr Ile
225 230 235 240

Glu Ile Lys Glu Glu Val Val Gly Leu Thr Glu Thr Ser Ser Gln Pro
245 250 255

Lys Asn Glu Glu Asp Ile Glu Ile Ile Pro Ile Gln Glu Glu Glu Glu
260 265 270

Glu Glu Thr Glu Thr Asn Phe Pro Glu Pro Pro Gln Asp Gln Glu Ser
275 280 285

Ser Pro Ile Glu Asn Asp Ser Ser Pro
290 295

<210> 13
<211> 4507
<212> DNA
<213> Homo sapiens

<400> 13
gaccaattgt catacgactt gcagtgagcg tcaggagcac gtccaggaac tcctcagcag 60
cgcctccttc agctccacag ccagacgccc tcagacagca aagcctaccc ccgcgccgcg 120
ccctgcccgc cgctgcgatg ctcgcccgcg ccctgctgct gtgcgcggtc ctggcgctca 180
gccatacagc aaatccttgc tgttcccacc catgtcaaaa ccgaggtgta tgtatgagtg 240
tgggatttga ccagtataag tgcgattgta cccggacagg attctatgga gaaaactgct 300
caacaccgga atttttgaca agaataaaat tatttctgaa acccactcca aacacagtgc 360
actacatact tacccacttc aagggatttt ggaacgttgt gaataacatt cccttccttc 420
gaaatgcaat tatgagttat gtgttgacat ccagatcaca tttgattgac agtccaccaa 480
cttacaatgc tgactatggc tacaaaagct gggaagcctt ctctaacctc tcctattata 540
ctagagccct tcctcctgtg cctgatgatt gcccgactcc cttgggtgtc aaaggtaaaa 600
agcagcttcc tgattcaaat gagattgtgg aaaaattgct tctaagaaga aagttcatcc 660
ctgatcccca gggctcaaac atgatgtttg cattctttgc ccagcacttc acgcatcagt 720
tttcaagac agatcataag cgagggccag ctttcaccaa cgggctgggc catggggtgg 780
acttaaatca tatttacggt gaaactctgg ctagacagcg taaactgcgc cttttcaagg 840
atggaaaaat gaaatatcag ataattgatg gagagatgta tcctcccaca gtcaaagata 900
ctcaggcaga gatgatctac cctcctcaag tccctgagca tctacggttt gctgtggggc 960

```
aggaggtctt tggtctggtg cctggtctga tgatgtatgc cacaatctgg ctgcgggaac    1020
acaacagagt atgcgatgtg cttaaacagg agcatcctga atggggtgat gagcagttgt    1080
tccagacaag caggctaata ctgataggag agactattaa gattgtgatt gaagattatg    1140
tgcaacactt gagtggctat cacttcaaac tgaaatttga cccagaacta cttttcaaca    1200
aacaattcca gtaccaaaat cgtattgctg ctgaatttaa caccctctat cactggcatc    1260
cccttctgcc tgacaccttt caaattcatg accagaaata caactatcaa cagtttatct    1320
acaacaactc tatattgctg gaacatggaa ttacccagtt tgttgaatca ttcaccaggc    1380
aaattgctgg cagggttgct ggtggtagga atgttccacc cgcagtacag aaagtatcac    1440
aggcttccat tgaccagagc aggcagatga aataccagtc ttttaatgag taccgcaaac    1500
gctttatgct gaagccctat gaatcatttg aagaacttac aggagaaaag gaaatgtctg    1560
cagagttgga agcactctat ggtgacatcg atgctgtgga gctgtatcct gcccttctgg    1620
tagaaaagcc tcggccagat gccatctttg gtgaaaccat ggtagaagtt ggagcaccat    1680
tctccttgaa aggacttatg ggtaatgtta tatgttctcc tgcctactgg aagccaagca    1740
cttttggtgg agaagtgggt tttcaaatca tcaacactgc ctcaattcag tctctcatct    1800
gcaataacgt gaagggctgt ccctttactt cattcagtgt tccagatcca gagctcatta    1860
aaacagtcac catcaatgca agttcttccc gctccggact agatgatatc aatcccacag    1920
tactactaaa agaacgttcg actgaactgt agaagtctaa tgatcatatt tatttattta    1980
tatgaaccat gtctattaat ttaattattt aataatattt atattaaact ccttatgtta    2040
cttaacatct tctgtaacag aagtcagtac tcctgttgcg gagaaaggag tcatacttgt    2100
gaagactttt atgtcactac tctaaagatt ttgctgttgc tgttaagttt ggaaaacagt    2160
ttttattctg ttttataaac cagagagaaa tgagttttga cgtcttttta cttgaatttc    2220
aacttatatt ataagaacga aagtaaagat gtttgaatac ttaaacactg tcacaagatg    2280
gcaaaatgct gaaagttttt acactgtcga tgtttccaat gcatcttcca tgatgcatta    2340
gaagtaacta atgtttgaaa ttttaaagta cttttggtta tttttctgtc atcaaacaaa    2400
aacaggtatc agtgcattat taaatgaata tttaaattag acattaccag taatttcatg    2460
tctacttttt aaaatcagca atgaaacaat aatttgaaat ttctaaattc atagggtaga    2520
atcacctgta aaagcttgtt tgatttctta aagttattaa acttgtacat ataccaaaaa    2580
gaagctgtct tggatttaaa tctgtaaaat cagtagaaat tttactacaa ttgcttgtta    2640
aaatatttta taagtgatgt tcctttttca ccaagagtat aaaccttttt agtgtgactg    2700
ttaaaacttc cttttaaatc aaaatgccaa atttattaag gtggtggagc cactgcagtg    2760
ttatcttaaa ataagaatat tttgttgaga tattccagaa tttgtttata tggctggtaa    2820
catgtaaaat ctatatcagc aaaagggtct acctttaaaa taagcaataa caaagaagaa    2880
aaccaaatta ttgttcaaat ttaggtttaa acttttgaag caaacttttt tttatccttg    2940
tgcactgcag gcctggtact cagattttgc tatgaggtta atgaagtacc aagctgtgct    3000
```

```
tgaataatga tatgttttct cagattttct gttgtacagt ttaatttagc agtccatatc      3060
acattgcaaa agtagcaatg acctcataaa atacctcttc aaaatgctta aattcatttc      3120
acacattaat tttatctcag tcttgaagcc aattcagtag gtgcattgga atcaagcctg      3180
gctacctgca tgctgttcct tttcttttct tcttttagcc attttgctaa gagacacagt      3240
cttctcatca cttcgtttct cctattttgt tttactagtt ttaagatcag agttcacttt      3300
ctttggactc tgcctatatt ttcttacctg aacttttgca agttttcagg taaacctcag      3360
ctcaggactg ctatttagct cctcttaaga agattaaaag agaaaaaaaa aggccctttt      3420
aaaaatagta tacacttatt ttaagtgaaa agcagagaat tttatttata gctaatttta      3480
gctatctgta accaagatgg atgcaaagag gctagtgcct cagagagaac tgtacggggt      3540
ttgtgactgg aaaaagttac gttcccattc taattaatgc cctttcttat ttaaaaacaa      3600
aaccaaatga tatctaagta gttctcagca ataataataa tgacgataat acttcttttc      3660
cacatctcat tgtcactgac atttaatggt actgtatatt acttaattta ttgaagatta      3720
ttatttatgt cttattagga cactatggtt ataaactgtg tttaagccta caatcattga      3780
tttttttttg ttatgtcaca atcagtatat cttctttggg gttacctctc tgaatattat      3840
gtaaacaatc caaagaaatg attgtattaa gatttgtgaa taaattttta gaaatctgat      3900
tggcatattg agatatttaa ggttgaatgt ttgtccttag gataggccta tgtgctagcc      3960
cacaaagaat attgtctcat tagcctgaat gtgccataag actgaccttt taaaatgttt      4020
tgagggatct gtggatgctt cgttaatttg ttcagccaca atttattgag aaaatattct      4080
gtgtcaagca ctgtgggttt taatattttt aaatcaaacg ctgattacag ataatagtat      4140
ttatataaat aattgaaaaa aattttcttt tgggaagagg gagaaaatga aataaatatc      4200
attaaagata actcaggaga atcttcttta caattttacg tttagaatgt ttaaggttaa      4260
gaaagaaata gtcaatatgc ttgtataaaa cactgttcac tgtttttttt aaaaaaaaaa      4320
cttgatttgt tattaacatt gatctgctga caaaacctgg gaatttgggt tgtgtatgcg      4380
aatgtttcag tgcctcagac aaatgtgtat ttaacttatg taaaagataa gtctggaaat      4440
aaatgtctgt ttattttttgt actatttaaa aattgacaga tcttttctga agaaaaaaaa      4500
aaaaaaa                                                                4507
```

```
<210>  14
<211>  604
<212>  PRT
<213>  Homo sapiens

<400>  14

Met Leu Ala Arg Ala Leu Leu Leu Cys Ala Val Leu Ala Leu Ser His
1               5                   10                  15

Thr Ala Asn Pro Cys Cys Ser His Pro Cys Gln Asn Arg Gly Val Cys
            20                  25                  30
```

```
Met Ser Val Gly Phe Asp Gln Tyr Lys Cys Asp Cys Thr Arg Thr Gly
        35                  40                  45

Phe Tyr Gly Glu Asn Cys Ser Thr Pro Glu Phe Leu Thr Arg Ile Lys
    50                  55                  60

Leu Phe Leu Lys Pro Thr Pro Asn Thr Val His Tyr Ile Leu Thr His
65                  70                  75                  80

Phe Lys Gly Phe Trp Asn Val Val Asn Asn Ile Pro Phe Leu Arg Asn
                85                  90                  95

Ala Ile Met Ser Tyr Val Leu Thr Ser Arg Ser His Leu Ile Asp Ser
            100                 105                 110

Pro Pro Thr Tyr Asn Ala Asp Tyr Gly Tyr Lys Ser Trp Glu Ala Phe
        115                 120                 125

Ser Asn Leu Ser Tyr Tyr Thr Arg Ala Leu Pro Pro Val Pro Asp Asp
    130                 135                 140

Cys Pro Thr Pro Leu Gly Val Lys Gly Lys Lys Gln Leu Pro Asp Ser
145                 150                 155                 160

Asn Glu Ile Val Glu Lys Leu Leu Leu Arg Arg Lys Phe Ile Pro Asp
                165                 170                 175

Pro Gln Gly Ser Asn Met Met Phe Ala Phe Phe Ala Gln His Phe Thr
            180                 185                 190

His Gln Phe Phe Lys Thr Asp His Lys Arg Gly Pro Ala Phe Thr Asn
        195                 200                 205

Gly Leu Gly His Gly Val Asp Leu Asn His Ile Tyr Gly Glu Thr Leu
    210                 215                 220

Ala Arg Gln Arg Lys Leu Arg Leu Phe Lys Asp Gly Lys Met Lys Tyr
225                 230                 235                 240

Gln Ile Ile Asp Gly Glu Met Tyr Pro Pro Thr Val Lys Asp Thr Gln
                245                 250                 255

Ala Glu Met Ile Tyr Pro Pro Gln Val Pro Glu His Leu Arg Phe Ala
            260                 265                 270

Val Gly Gln Glu Val Phe Gly Leu Val Pro Gly Leu Met Met Tyr Ala
        275                 280                 285

Thr Ile Trp Leu Arg Glu His Asn Arg Val Cys Asp Val Leu Lys Gln
    290                 295                 300
```

```
Glu His Pro Glu Trp Gly Asp Glu Gln Leu Phe Gln Thr Ser Arg Leu
305                 310                 315                 320

Ile Leu Ile Gly Glu Thr Ile Lys Ile Val Ile Glu Asp Tyr Val Gln
                325                 330                 335

His Leu Ser Gly Tyr His Phe Lys Leu Lys Phe Asp Pro Glu Leu Leu
            340                 345                 350

Phe Asn Lys Gln Phe Gln Tyr Gln Asn Arg Ile Ala Ala Glu Phe Asn
        355                 360                 365

Thr Leu Tyr His Trp His Pro Leu Leu Pro Asp Thr Phe Gln Ile His
    370                 375                 380

Asp Gln Lys Tyr Asn Tyr Gln Gln Phe Ile Tyr Asn Asn Ser Ile Leu
385                 390                 395                 400

Leu Glu His Gly Ile Thr Gln Phe Val Glu Ser Phe Thr Arg Gln Ile
                405                 410                 415

Ala Gly Arg Val Ala Gly Gly Arg Asn Val Pro Pro Ala Val Gln Lys
            420                 425                 430

Val Ser Gln Ala Ser Ile Asp Gln Ser Arg Gln Met Lys Tyr Gln Ser
        435                 440                 445

Phe Asn Glu Tyr Arg Lys Arg Phe Met Leu Lys Pro Tyr Glu Ser Phe
    450                 455                 460

Glu Glu Leu Thr Gly Glu Lys Glu Met Ser Ala Glu Leu Glu Ala Leu
465                 470                 475                 480

Tyr Gly Asp Ile Asp Ala Val Glu Leu Tyr Pro Ala Leu Leu Val Glu
                485                 490                 495

Lys Pro Arg Pro Asp Ala Ile Phe Gly Glu Thr Met Val Glu Val Gly
            500                 505                 510

Ala Pro Phe Ser Leu Lys Gly Leu Met Gly Asn Val Ile Cys Ser Pro
        515                 520                 525

Ala Tyr Trp Lys Pro Ser Thr Phe Gly Gly Glu Val Gly Phe Gln Ile
    530                 535                 540

Ile Asn Thr Ala Ser Ile Gln Ser Leu Ile Cys Asn Asn Val Lys Gly
545                 550                 555                 560

Cys Pro Phe Thr Ser Phe Ser Val Pro Asp Pro Glu Leu Ile Lys Thr
                565                 570                 575
```

```
Val Thr Ile Asn Ala Ser Ser Ser Arg Ser Gly Leu Asp Asp Ile Asn
        580                 585                 590

Pro Thr Val Leu Leu Lys Glu Arg Ser Thr Glu Leu
    595                 600
```

<210> 15
<211> 4446
<212> DNA
<213> Homo sapiens

<400> 15

```
ggcgcatggc tgcggaagcg aggtgcagac cgaggtcccg agggatcgcc ctccgggaag      60
cggtgatgct gttgttgtac ttcggggtgc caaccgggca ctcctacaac ctggacccgg     120
agaatgcact gctgtaccag ggcccctccg gcacgctgtt tggctactcg gtggtgctgc     180
acagccacgg gtcgaagcgc tggctcatcg tgggggctcc cactgccagc tggctctcta     240
atgcctcagt ggtcaatcct ggggcgattt acagatgcgg gatcagaaag aatccaaacc     300
agacctgcga acagctccag ctgggtagcc ccagtggaga gccttgtggg aagacatgcc     360
tggaggagag ggataaccag tggctggggg tcaccctttc cagacagcct ggagaaaatg     420
gctctatcgt gacttgtggg cacaggtgga aaaatatttt ttacatgaag agcgataaca     480
aactccccac tggcatttgc tacgtcatgc cttctgattt gcggacagaa ctgagtaaaa     540
ggatggcgcc gtgttgcaaa gattatacga gaaaatttgg agaaaatttt gcatcatgtc     600
aagctggaat atctagtttt tacacacagg atttaattgt gatgggggcc ccgggatcat     660
cgtactggac tggcaccgtc tttgtctaca atataactac aaaccaatac aaagcatttg     720
tagacagaca gaaccaagta aaatttggaa gctacttagg ctactcagtt ggagctggac     780
attttcgaag tccacatact accgaagtcg tgggaggagc ccctcaacac gaacagatag     840
gaaaagcata tatatttagc attgatgaaa acgaactgaa catcgtatat gaaatgaaag     900
gtaaaaagct tggctcatac tttggagctt ctgtctgcgc tgtggacctc aatgcagatg     960
gcttctcaga tctccttgtt ggagctccca tgcagagcac catcagggag gaaggaagag    1020
tattcgtgta catcaactct ggcatgggag ctgtgatggt tgaaatggaa agggtccttg    1080
tcggaagtga caaatatgct gcaagatttg gggagtctat agcgaatctt ggcgacattg    1140
acaatgacgg ctttgaagat attgctattg gtgcaccaca agaagacgac ttgcgaggtg    1200
ctgtctacat ttacaatggc cgagtcgatg gaatctcctc cacctactca cagagaattg    1260
aaggacagca aatcagcaaa tcattaagga tgtttggaca atctatctca ggacaaattg    1320
atgcagacaa caatggatat gttgatgtag ccattggtgc atttcactct gattctgcag    1380
tgttgctaag gacaaggcct gtagtgattg ttgaagcatc tttaagccat cctgggtctg    1440
taaataggac aaagtttgac tgtactgaaa atggacttcc atctgtgtgc atgcatctta    1500
cactgtgttt ctcatataaa ggcaaagagg tcccaggcta catcgttttg ttttacaatg    1560
tgagcttgga tgtgcacagg aaggcagagt ctccatcaag attttatttc ttctctaatg    1620
```

```
ggacttctga cgtgattaca ggaagcatac aagtttcaag cagtagagag aaatgtagga   1680
cacaccaggc attcatgcgg aaagacgtgc gagacatcct tacccccatt catgtagagg   1740
ccacatacca ccttgggcat catgtgatca ccaaacgaaa cactgaggaa tttccaccac   1800
tccagccgat ccttcagcag aagaaagaaa aagacgttat tagaaaaatg ataaactttg   1860
caaggttttg tgcctatgaa aattgctctg ctgatctcca agtttctgca aaagttggat   1920
ttttgaagta agtatagttt actctgtttt tcatcaaaag agcaaaaagg gtaaatgcat   1980
gtgggttcta gcaacagggg atctcaccct cgaacgtcag catcatttaa aaatttgcta   2040
aataataatg ttttgctgtg agggactgag tgatgcttat atcactcatt ttacatgtaa   2100
aatgaatgat caaatatata tatatatata tatgcactat catcttttaa agtatgagag   2160
catatgagca tccctattat tttatgaagt ctgctttaag tcttttgttg taacttagaa   2220
ggtatttcag gatttaactg tgccttgaga tatcattatc acagtcataa ctatatttta   2280
acattttaaa ggcctggtaa caagagttta gttggaaaaa ataaacttta ctacacaatg   2340
gttagtatac tttatggcta gaaaatgggt tatgcaattc aaactgatat gcaaaatact   2400
atattttgac aaacttgaaa aagagtaaac taatttgttc taaattcatg gagtaagaag   2460
aaaatacaat gtgcttagaa tagcatctgt catagaggaa gaactaggta aataggaatg   2520
tctactgtta ctttttaatg gaaaaaaact gcaattatgt atcatgtaga ggtttcctac   2580
attcaatttg ctttgcacat ctatagacaa tgggaaatga aaaaaggtta aggatacaga   2640
gtgaccagga gcaggaatga gaaactagat tagaaggtgg gacataagca aaccatgcaa   2700
ctatgtagtt ttatgtcaat ttaactcaag aagagcattg gggaaaagga agtgccaata   2760
gacaatggct ccacaagatt ggcctacagg caagcttgtg acgaatttct tcattgactg   2820
attgatttgg gaaggcttag ccagtgtgga caagtgccgt ccctgggctg gctggtggtc   2880
ctggattcta caagaaaaca ggctgcacaa agtctctttt tcatagcctc tgcatcagct   2940
cctgcctcaa ggttcttgtc tggagttccc tggatgatgg actacaagct atagagaaaa   3000
tgaatccttt cttccccaag ttgattttgg tcatgatgtt ttatcacagc aataggaatc   3060
ttacctaaga aaatggagct ctggtacatg gctccttact tgaaaatcac tctcaatatc   3120
acatttagaa ttccctctac acattagaga tggcataggg atgagaaatg taaattttac   3180
aggatgaaca aacattgaga gtcattttat tcaaggatct taaaaacagt agaattcttt   3240
tcagtcaggc aatgttagca tagccatggg tatctatagt tcagaggcaa ggagccctcc   3300
cttgactggt aaaggaactt agtgcttaca agtgattcag ttggctcact caggtaattg   3360
agtacctgct ttgtgtatca gagcatggtg tgactttgtg ggtctattag tttgggaata   3420
tataattttc catttgagta ttttttctaa gatatgtagg tttttggttt atcaagagac   3480
tttgataaaa agtcgttgtg caggtgcatg tgcaaagagg gagatttgcg aagccataga   3540
tgaagtgatt ccctgtgctt gcatttgggt tctctgtatc ttcctgctcc tccttcattg   3600
ctaatcttca tgagaaatga gatttcaaag tttacgagaa aatgtcacta agaacgattt   3660
```

```
caacttattc aaataataga tagaatcatt aattcatttt ttcttgtttc ttgcaaagta    3720
attgtctact tagcattttt aatcagaagt gtttttccaa ttaaatatca agcctatgat    3780
tttaaaatta gcatcgggcc aaacataagg tggtgtttct ttaggatgtg ggtcctgagt    3840
catgtccagg aaaaatatcc acaatgtcta ttggaaatgt gtatcctcag ggcctagcaa    3900
acacagaagt ggatgatcac agtcagctat tggatgggtc acatggcccc taatggagga    3960
gctagagaaa ttacccaagg agctatagga aactgcaacc ctataggtgg aacaacaata    4020
taaactaacc agtacccggg agctcttgtc tttaactgca tatgtatcaa aagatggcct    4080
agtcggccat cactgcaaag agaggcccat tgaacttgca aactttatat gccccagtac    4140
aggggaacgc cagggccaaa aagggggagt gggtgggtag gggattgggg gggtgggtat    4200
gggggacctt tgggatagca ttgaaaatgt aaatgaggaa aatacctaat aaaaaaaaac    4260
tccacttcca ggagctacag agaaggctca gtggttacga gcgcatgtac agttcttgca    4320
ggggacccaa gttcacttcc caacatccac agtgggccac taagactgat actcatatat    4380
ctgagtccat gtgggtattt gtgtttctaa cacatatatg caaacaaacc caaaaataaa    4440
ctcctc                                                               4446
```

```
<210>  16
<211>  642
<212>  PRT
<213>  Homo sapiens

<400>  16

Arg Met Ala Ala Glu Ala Arg Cys Arg Pro Arg Ser Arg Gly Ile Ala
1               5                   10                  15

Leu Arg Glu Ala Val Met Leu Leu Leu Tyr Phe Gly Val Pro Thr Gly
            20                  25                  30

His Ser Tyr Asn Leu Asp Pro Glu Asn Ala Leu Leu Tyr Gln Gly Pro
        35                  40                  45

Ser Gly Thr Leu Phe Gly Tyr Ser Val Val Leu His Ser His Gly Ser
    50                  55                  60

Lys Arg Trp Leu Ile Val Gly Ala Pro Thr Ala Ser Trp Leu Ser Asn
65                  70                  75                  80

Ala Ser Val Val Asn Pro Gly Ala Ile Tyr Arg Cys Gly Ile Arg Lys
                85                  90                  95

Asn Pro Asn Gln Thr Cys Glu Gln Leu Gln Leu Gly Ser Pro Ser Gly
            100                 105                 110

Glu Pro Cys Gly Lys Thr Cys Leu Glu Glu Arg Asp Asn Gln Trp Leu
        115                 120                 125
```

Gly Val Thr Leu Ser Arg Gln Pro Gly Glu Asn Gly Ser Ile Val Thr
130 135 140

Cys Gly His Arg Trp Lys Asn Ile Phe Tyr Met Lys Ser Asp Asn Lys
145 150 155 160

Leu Pro Thr Gly Ile Cys Tyr Val Met Pro Ser Asp Leu Arg Thr Glu
165 170 175

Leu Ser Lys Arg Met Ala Pro Cys Cys Lys Asp Tyr Thr Arg Lys Phe
180 185 190

Gly Glu Asn Phe Ala Ser Cys Gln Ala Gly Ile Ser Ser Phe Tyr Thr
195 200 205

Gln Asp Leu Ile Val Met Gly Ala Pro Gly Ser Ser Tyr Trp Thr Gly
210 215 220

Thr Val Phe Val Tyr Asn Ile Thr Thr Asn Gln Tyr Lys Ala Phe Val
225 230 235 240

Asp Arg Gln Asn Gln Val Lys Phe Gly Ser Tyr Leu Gly Tyr Ser Val
245 250 255

Gly Ala Gly His Phe Arg Ser Pro His Thr Thr Glu Val Val Gly Gly
260 265 270

Ala Pro Gln His Glu Gln Ile Gly Lys Ala Tyr Ile Phe Ser Ile Asp
275 280 285

Glu Asn Glu Leu Asn Ile Val Tyr Glu Met Lys Gly Lys Lys Leu Gly
290 295 300

Ser Tyr Phe Gly Ala Ser Val Cys Ala Val Asp Leu Asn Ala Asp Gly
305 310 315 320

Phe Ser Asp Leu Leu Val Gly Ala Pro Met Gln Ser Thr Ile Arg Glu
325 330 335

Glu Gly Arg Val Phe Val Tyr Ile Asn Ser Gly Met Gly Ala Val Met
340 345 350

Val Glu Met Glu Arg Val Leu Val Gly Ser Asp Lys Tyr Ala Ala Arg
355 360 365

Phe Gly Glu Ser Ile Ala Asn Leu Gly Asp Ile Asp Asn Asp Gly Phe
370 375 380

Glu Asp Ile Ala Ile Gly Ala Pro Gln Glu Asp Asp Leu Arg Gly Ala
385 390 395 400

```
Val Tyr Ile Tyr Asn Gly Arg Val Asp Gly Ile Ser Ser Thr Tyr Ser
                405                 410                 415

Gln Arg Ile Glu Gly Gln Gln Ile Ser Lys Ser Leu Arg Met Phe Gly
            420                 425                 430

Gln Ser Ile Ser Gly Gln Ile Asp Ala Asp Asn Asn Gly Tyr Val Asp
        435                 440                 445

Val Ala Ile Gly Ala Phe His Ser Asp Ser Ala Val Leu Leu Arg Thr
    450                 455                 460

Arg Pro Val Val Ile Val Glu Ala Ser Leu Ser His Pro Gly Ser Val
465                 470                 475                 480

Asn Arg Thr Lys Phe Asp Cys Thr Glu Asn Gly Leu Pro Ser Val Cys
                485                 490                 495

Met His Leu Thr Leu Cys Phe Ser Tyr Lys Gly Lys Glu Val Pro Gly
            500                 505                 510

Tyr Ile Val Leu Phe Tyr Asn Val Ser Leu Asp Val His Arg Lys Ala
        515                 520                 525

Glu Ser Pro Ser Arg Phe Tyr Phe Phe Ser Asn Gly Thr Ser Asp Val
    530                 535                 540

Ile Thr Gly Ser Ile Gln Val Ser Ser Ser Arg Glu Lys Cys Arg Thr
545                 550                 555                 560

His Gln Ala Phe Met Arg Lys Asp Val Arg Asp Ile Leu Thr Pro Ile
                565                 570                 575

His Val Glu Ala Thr Tyr His Leu Gly His His Val Ile Thr Lys Arg
            580                 585                 590

Asn Thr Glu Glu Phe Pro Pro Leu Gln Pro Ile Leu Gln Gln Lys Lys
        595                 600                 605

Glu Lys Asp Val Ile Arg Lys Met Ile Asn Phe Ala Arg Phe Cys Ala
    610                 615                 620

Tyr Glu Asn Cys Ser Ala Asp Leu Gln Val Ser Ala Lys Val Gly Phe
625                 630                 635                 640

Leu Lys
```

<210> 17
<211> 2662
<212> DNA
<213> Homo sapiens

<400> 17

```
gaagagaccc agcaacccac agagttgaga aatttgactg gcattcaagc tgtccaatca      60
atagctgccg ctgaagggtg gggctggatg gcgtaagcta cagctgaagg aagaacgtga     120
gcacgaggca ctgaggtgat tggctgaagg cacttccgtt gagcatctag acgtttcctt     180
ggctcttctg gcgccaaaat gtcgttcgtg gcaggggtta ttcggcggct ggacgagaca     240
gtggtgaacc gcatcgcggc gggggaagtt atccagcggc cagctaatgc tatcaaagag     300
atgattgaga actgtttaga tgcaaaatcc acaagtattc aagtgattgt taaagaggga     360
ggcctgaagt tgattcagat ccaagacaat ggcaccggga tcaggaaaga agatctggat     420
attgtatgtg aaaggttcac tactagtaaa ctgcagtcct ttgaggattt agccagtatt     480
tctacctatg gctttcgagg tgaggctttg gccagcataa gccatgtggc tcatgttact     540
attacaacga aaacagctga tggaaagtgt gcatacagag caagttactc agatggaaaa     600
ctgaaagccc ctcctaaacc atgtgctggc aatcaaggga cccagatcac ggtggaggac     660
ctttttaca acatagccac gaggagaaaa gctttaaaaa atccaagtga agaatatggg     720
aaaattttgg aagttgttgg caggtattca gtacacaatg caggcattag tttctcagtt     780
aaaaaacaag gagagacagt agctgatgtt aggacactac ccaatgcctc aaccgtggac     840
aatattcgct ccatctttgg aaatgctgtt agtcgagaac tgatagaaat tggatgtgag     900
gataaaaccc tagccttcaa aatgaatggt tacatatcca atgcaaacta ctcagtgaag     960
aagtgcatct tcttactctt catcaaccat cgtctggtag aatcaacttc cttgagaaaa    1020
gccatagaaa cagtgtatgc agcctatttg cccaaaaaca cacacccatt cctgtacctc    1080
agtttagaaa tcagtcccca gaatgtggat gttaatgtgc accccacaaa gcatgaagtt    1140
cacttcctgc acgaggagag catcctggag cgggtgcagc agcacatcga gagcaagctc    1200
ctgggctcca attcctccag gatgtacttc acccagactt tgctaccagg acttgctggc    1260
ccctctgggg agatggttaa atccacaaca agtctgacct cgtcttctac ttctggaagt    1320
agtgataagg tctatgccca ccagatggtt cgtacagatt cccgggaaca gaagcttgat    1380
gcatttctgc agcctctgag caaacccctg tccagtcagc cccaggccat tgtcacagag    1440
gataagacag atatttctag tggcagggct aggcagcaag atgaggagat gcttgaactc    1500
ccagcccctg ctgaagtggc tgccaaaaat cagagcttgg agggggatac aacaaagggg    1560
acttcagaaa tgtcagagaa gagaggacct acttccagca accccagaaa gagacatcgg    1620
gaagattctg atgtggaaat ggtggaagat gattcccgaa aggaaatgac tgcagcttgt    1680
acccccgga gaaggatcat taacctcact agtgttttga gtctccagga agaaattaat    1740
gagcagggac atgaggttct ccgggagatg ttgcataacc actccttcgt gggctgtgtg    1800
aatcctcagt gggccttggc acagcatcaa accaagttat accttctcaa caccaccaag    1860
cttagtgaag aactgttcta ccagatactc atttatgatt ttgccaattt tggtgttctc    1920
aggttatcgg agccagcacc gctctttgac cttgccatgc ttgccttaga tagtccagag    1980
```

agtggctgga cagaggaaga tggtcccaaa gaaggacttg ctgaatacat tgttgagttt 2040
ctgaagaaga aggctgagat gcttgcagac tatttctctt tggaaattga tgaggaaggg 2100
aacctgattg gattacccct tctgattgac aactatgtgc cccctttgga gggactgcct 2160
atcttcattc ttcgactagc cactgaggtg aattgggacg aagaaaagga atgttttgaa 2220
agcctcagta aagaatgcgc tatgttctat tccatccgga agcagtacat atctgaggag 2280
tcgaccctct caggccagca gagtgaagtg cctggctcca ttccaaactc ctggaagtgg 2340
actgtggaac acattgtcta taaagccttg cgctcacaca ttctgcctcc taaacatttc 2400
acagaagatg gaaatatcct gcagcttgct aacctgcctg atctatacaa agtctttgag 2460
aggtgttaaa tatggttatt tatgcactgt gggatgtgtt cttctttctc tgtattccga 2520
tacaaagtgt tgtatcaaag tgtgatatac aaagtgtacc aacataagtg ttggtagcac 2580
ttaagactta tacttgcctt ctgatagtat tcctttatac acagtggatt gattataaat 2640
aaatagatgt gtcttaacat aa 2662

<210> 18
<211> 756
<212> PRT
<213> Homo sapiens

<400> 18

Met Ser Phe Val Ala Gly Val Ile Arg Arg Leu Asp Glu Thr Val Val
1 5 10 15

Asn Arg Ile Ala Ala Gly Glu Val Ile Gln Arg Pro Ala Asn Ala Ile
20 25 30

Lys Glu Met Ile Glu Asn Cys Leu Asp Ala Lys Ser Thr Ser Ile Gln
35 40 45

Val Ile Val Lys Glu Gly Gly Leu Lys Leu Ile Gln Ile Gln Asp Asn
50 55 60

Gly Thr Gly Ile Arg Lys Glu Asp Leu Asp Ile Val Cys Glu Arg Phe
65 70 75 80

Thr Thr Ser Lys Leu Gln Ser Phe Glu Asp Leu Ala Ser Ile Ser Thr
85 90 95

Tyr Gly Phe Arg Gly Glu Ala Leu Ala Ser Ile Ser His Val Ala His
100 105 110

Val Thr Ile Thr Thr Lys Thr Ala Asp Gly Lys Cys Ala Tyr Arg Ala
115 120 125

Ser Tyr Ser Asp Gly Lys Leu Lys Ala Pro Pro Lys Pro Cys Ala Gly
130 135 140

```
Asn Gln Gly Thr Gln Ile Thr Val Glu Asp Leu Phe Tyr Asn Ile Ala
145                 150                 155                 160

Thr Arg Arg Lys Ala Leu Lys Asn Pro Ser Glu Glu Tyr Gly Lys Ile
                165                 170                 175

Leu Glu Val Val Gly Arg Tyr Ser Val His Asn Ala Gly Ile Ser Phe
            180                 185                 190

Ser Val Lys Lys Gln Gly Glu Thr Val Ala Asp Val Arg Thr Leu Pro
        195                 200                 205

Asn Ala Ser Thr Val Asp Asn Ile Arg Ser Ile Phe Gly Asn Ala Val
    210                 215                 220

Ser Arg Glu Leu Ile Glu Ile Gly Cys Glu Asp Lys Thr Leu Ala Phe
225                 230                 235                 240

Lys Met Asn Gly Tyr Ile Ser Asn Ala Asn Tyr Ser Val Lys Lys Cys
                245                 250                 255

Ile Phe Leu Leu Phe Ile Asn His Arg Leu Val Glu Ser Thr Ser Leu
            260                 265                 270

Arg Lys Ala Ile Glu Thr Val Tyr Ala Ala Tyr Leu Pro Lys Asn Thr
        275                 280                 285

His Pro Phe Leu Tyr Leu Ser Leu Glu Ile Ser Pro Gln Asn Val Asp
    290                 295                 300

Val Asn Val His Pro Thr Lys His Glu Val His Phe Leu His Glu Glu
305                 310                 315                 320

Ser Ile Leu Glu Arg Val Gln Gln His Ile Glu Ser Lys Leu Leu Gly
                325                 330                 335

Ser Asn Ser Ser Arg Met Tyr Phe Thr Gln Thr Leu Leu Pro Gly Leu
            340                 345                 350

Ala Gly Pro Ser Gly Glu Met Val Lys Ser Thr Thr Ser Leu Thr Ser
        355                 360                 365

Ser Ser Thr Ser Gly Ser Ser Asp Lys Val Tyr Ala His Gln Met Val
    370                 375                 380

Arg Thr Asp Ser Arg Glu Gln Lys Leu Asp Ala Phe Leu Gln Pro Leu
385                 390                 395                 400

Ser Lys Pro Leu Ser Ser Gln Pro Gln Ala Ile Val Thr Glu Asp Lys
                405                 410                 415
```

```
Thr Asp Ile Ser Ser Gly Arg Ala Arg Gln Gln Asp Glu Glu Met Leu
            420                 425                 430

Glu Leu Pro Ala Pro Ala Glu Val Ala Ala Lys Asn Gln Ser Leu Glu
        435                 440                 445

Gly Asp Thr Thr Lys Gly Thr Ser Glu Met Ser Glu Lys Arg Gly Pro
    450                 455                 460

Thr Ser Ser Asn Pro Arg Lys Arg His Arg Glu Asp Ser Asp Val Glu
465                 470                 475                 480

Met Val Glu Asp Asp Ser Arg Lys Glu Met Thr Ala Ala Cys Thr Pro
                485                 490                 495

Arg Arg Arg Ile Ile Asn Leu Thr Ser Val Leu Ser Leu Gln Glu Glu
            500                 505                 510

Ile Asn Glu Gln Gly His Glu Val Leu Arg Glu Met Leu His Asn His
        515                 520                 525

Ser Phe Val Gly Cys Val Asn Pro Gln Trp Ala Leu Ala Gln His Gln
    530                 535                 540

Thr Lys Leu Tyr Leu Leu Asn Thr Thr Lys Leu Ser Glu Glu Leu Phe
545                 550                 555                 560

Tyr Gln Ile Leu Ile Tyr Asp Phe Ala Asn Phe Gly Val Leu Arg Leu
                565                 570                 575

Ser Glu Pro Ala Pro Leu Phe Asp Leu Ala Met Leu Ala Leu Asp Ser
            580                 585                 590

Pro Glu Ser Gly Trp Thr Glu Glu Asp Gly Pro Lys Glu Gly Leu Ala
        595                 600                 605

Glu Tyr Ile Val Glu Phe Leu Lys Lys Lys Ala Glu Met Leu Ala Asp
    610                 615                 620

Tyr Phe Ser Leu Glu Ile Asp Glu Glu Gly Asn Leu Ile Gly Leu Pro
625                 630                 635                 640

Leu Leu Ile Asp Asn Tyr Val Pro Pro Leu Glu Gly Leu Pro Ile Phe
                645                 650                 655

Ile Leu Arg Leu Ala Thr Glu Val Asn Trp Asp Glu Glu Lys Glu Cys
            660                 665                 670

Phe Glu Ser Leu Ser Lys Glu Cys Ala Met Phe Tyr Ser Ile Arg Lys
        675                 680                 685
```

```
Gln Tyr Ile Ser Glu Glu Ser Thr Leu Ser Gly Gln Gln Ser Glu Val
    690                 695                 700

Pro Gly Ser Ile Pro Asn Ser Trp Lys Trp Thr Val Glu His Ile Val
705                 710                 715                 720

Tyr Lys Ala Leu Arg Ser His Ile Leu Pro Pro Lys His Phe Thr Glu
                725                 730                 735

Asp Gly Asn Ile Leu Gln Leu Ala Asn Leu Pro Asp Leu Tyr Lys Val
            740                 745                 750

Phe Glu Arg Cys
        755
```

<210> 19
<211> 1126
<212> DNA
<213> Homo sapiens

<400> 19

```
tggtactggg tggtcctgca aaatccccaa ctttgatgtg cgtgaagttg tgaataatat      60
cagacggctt ctggatggag aggagcccct acccatgctc ccaagttata agaatttcaa     120
aggtactatt gaagaactgg cttcaaatca gtatgtgatt aatggagaag tagctattct     180
ggattctaca accattgaaa tctcagagct tcccatccga acgtggactc agacatataa     240
agaacaggtt ctagaaccca tgttgaatgg aactgagaag acaccctctc taataacaga     300
ctatagggaa taccatacag ataccactgt gaagtttgtc ataaagatga ctgaagaaaa     360
actggcagag gcagagagag tcggcctaca caaagtcttc aaactccaga gtagtctcac     420
ttgcaactct atggtgcttt ttgaccatgt aggttgctta aagaaatatg acactgtgtt     480
ggatattcta agagacttct ttgagctcag gcttaaatat tatggattaa gaaaagaatg     540
gcttctagga atgcttggtg cagaatcttc taaactgaat aatcaagctc gctttatatt     600
agagaaaata gatggcaaaa tagtcattga aaataaacct aaaaaagaat taattaaagt     660
tctgattcag agaggctacg actctgaccc tgtgaaagcc tggaaagaag ctcagcagaa     720
ggttccagat gaagaagaaa atgaagaaag tgacactgaa accagcacca gtgactctgc     780
agccgaggct gggccgacct tcaactacct tctcgacatg cccctgtggt atctgaccaa     840
ggagaagaag gatgagctgt gcaaacaaag aaacgagaag gaacaagagc tcaacacatt     900
aaagcaaaag agtccatcag atctgtggaa ggaagatctg gctgttttta ttgaagaact     960
ggaggttgtc gaggccaagg aaaagcagga tgaacaagtg ggccttcctg gaaaggcggg    1020
gaaagcaaag ggaagaaag cacagatgtg tgctgacgtc ttgccttctc ctcggggcaa    1080
aagagtcatc ccccaagtga ctgtggagat gaaagctgag gcagag                  1126
```

<210> 20
<211> 375
<212> PRT

<213> Homo sapiens

<400> 20

```
Gly Thr Gly Trp Ser Cys Lys Ile Pro Asn Phe Asp Val Arg Glu Val
1               5                   10                  15

Val Asn Asn Ile Arg Arg Leu Leu Asp Gly Glu Glu Pro Leu Pro Met
            20                  25                  30

Leu Pro Ser Tyr Lys Asn Phe Lys Gly Thr Ile Glu Glu Leu Ala Ser
        35                  40                  45

Asn Gln Tyr Val Ile Asn Gly Glu Val Ala Ile Leu Asp Ser Thr Thr
    50                  55                  60

Ile Glu Ile Ser Glu Leu Pro Ile Arg Thr Trp Thr Gln Thr Tyr Lys
65                  70                  75                  80

Glu Gln Val Leu Glu Pro Met Leu Asn Gly Thr Glu Lys Thr Pro Ser
                85                  90                  95

Leu Ile Thr Asp Tyr Arg Glu Tyr His Thr Asp Thr Thr Val Lys Phe
            100                 105                 110

Val Ile Lys Met Thr Glu Glu Lys Leu Ala Glu Ala Glu Arg Val Gly
        115                 120                 125

Leu His Lys Val Phe Lys Leu Gln Ser Ser Leu Thr Cys Asn Ser Met
    130                 135                 140

Val Leu Phe Asp His Val Gly Cys Leu Lys Lys Tyr Asp Thr Val Leu
145                 150                 155                 160

Asp Ile Leu Arg Asp Phe Phe Glu Leu Arg Leu Lys Tyr Tyr Gly Leu
                165                 170                 175

Arg Lys Glu Trp Leu Leu Gly Met Leu Gly Ala Glu Ser Ser Lys Leu
            180                 185                 190

Asn Asn Gln Ala Arg Phe Ile Leu Glu Lys Ile Asp Gly Lys Ile Val
        195                 200                 205

Ile Glu Asn Lys Pro Lys Lys Glu Leu Ile Lys Val Leu Ile Gln Arg
    210                 215                 220

Gly Tyr Asp Ser Asp Pro Val Lys Ala Trp Lys Glu Ala Gln Gln Lys
225                 230                 235                 240

Val Pro Asp Glu Glu Glu Asn Glu Glu Ser Asp Thr Glu Thr Ser Thr
                245                 250                 255
```

```
Ser Asp Ser Ala Ala Glu Ala Gly Pro Thr Phe Asn Tyr Leu Leu Asp
            260                 265                 270

Met Pro Leu Trp Tyr Leu Thr Lys Glu Lys Lys Asp Glu Leu Cys Lys
        275                 280                 285

Gln Arg Asn Glu Lys Glu Gln Glu Leu Asn Thr Leu Lys Gln Lys Ser
    290                 295                 300

Pro Ser Asp Leu Trp Lys Glu Asp Leu Ala Val Phe Ile Glu Glu Leu
305                 310                 315                 320

Glu Val Val Glu Ala Lys Glu Lys Gln Asp Glu Gln Val Gly Leu Pro
                325                 330                 335

Gly Lys Ala Gly Lys Ala Lys Gly Lys Lys Ala Gln Met Cys Ala Asp
            340                 345                 350

Val Leu Pro Ser Pro Arg Gly Lys Arg Val Ile Pro Gln Val Thr Val
        355                 360                 365

Glu Met Lys Ala Glu Ala Glu
    370                 375
```

<210> 21
<211> 3851
<212> DNA
<213> Homo sapiens

<400> 21

```
ctctcccaac cgcctcgtcg cactcctcag gctgagagca ccgctgcact cgcggccggc      60
gatgcgggac cccggcgcgg ccgctccgct ttcgtccctg ggcctctgtg ccctggtgct     120
ggcgctgctg ggcgcactgt ccgcgggcgc cggggcgcag ccgtaccacg gagagaaggg     180
catctccgtg ccggaccacg gcttctgcca gcccatctcc atcccgctgt gcacggacat     240
cgcctacaac cagaccatcc tgcccaacct gctgggccac acgaaccaag aggacgcggg     300
cctcgaggtg caccagttct acccgctggt gaaggtgcag tgttctcccg aactccgctt     360
tttcttatgc tccatgtatg cgcccgtgtg caccgtgctc gatcaggcca tcccgccgtg     420
tcgttctctg tgcgagcgcg cccgccaggg ctgcgaggcg ctcatgaaca agttcggctt     480
ccagtggccc gagcggctgc gctgcgagaa cttcccggtg cacggtgcgg gcgagatctg     540
cgtgggccag aacacgtcgg acggctccgg gggcccaggc ggcggcccca ctgcctaccc     600
taccgcgccc tacctgccgg acctgccctt caccgcgctg cccccggggg cctcagatgg     660
caggggggcgt cccgccttcc ccttctcatg cccccgtcag ctcaaggtgc cccgtacct     720
gggctaccgc ttcctgggtg agcgcgattg tggcgccccg tgcgaaccgg gccgtgccaa     780
cggcctgatg tactttaagg aggaggagag gcgcttcgcc cgcctctggg tgggcgtgtg     840
gtccgtgctg tgctgcgcct cgacgctctt taccgttctc acctacctgg tggacatgcg     900
```

gcgcttcagc tacccagagc ggcccatcat cttcctgtcg ggctgctact tcatggtggc 960
cgtggcgcac gtggccggct tccttctaga ggaccgcgcc gtgtgcgtgg agcgcttctc 1020
ggacgatggc taccgcacgg tggcgcaggg caccaagaag gagggctgca ccatcctctt 1080
catggtgctc tacttcttcg gcatggccag ctccatctgg tgggtcattc tgtctctcac 1140
ttggttcctg gcggccggca tgaagtgggg ccacgaggcc atcgaggcca actcgcagta 1200
cttccacctg gccgcgtggg ccgtgcccgc cgtcaagacc atcactatcc tggccatggg 1260
ccaggtagac ggggacctgc tgagcggggt gtgctacgtt ggcctctcca gtgtggacgc 1320
gctgcggggc ttcgtgctgg cgcctctgtt cgtctacctc ttcataggca cgtccttctt 1380
gctggccggc ttcgtgtccc tcttccgtat ccgcaccatc atgaaacacg acggcaccaa 1440
gaccgagaag ctggagaagc tcatggtgcg catcggcgtc ttcagcgtgc tctacacagt 1500
gcccgccacc atcgtcctgg cctgctactt ctacgagcag gccttccgcg agcactggga 1560
gcgcacctgg ctcctgcaga cgtgcaagag ctatgccgtg ccctgcccgc ccggccactt 1620
cccgcccatg agccccgact tcaccgtctt catgatcaag tacctgatga ccatgatcgt 1680
cggcatcacc actggcttct ggatctggtc gggcaagacc ctgcagtcgt ggcgccgctt 1740
ctaccacaga cttagccaca gcagcaaggg ggagactgcg gtatgagccc cggcccctcc 1800
ccacctttcc caccccagcc ctcttgcaag aggagaggca cggtagggaa aagaactgct 1860
gggtgggggc ctgtttctgt aactttctcc ccctctactg agaagtgacc tggaagtgag 1920
aagttctttg cagatttggg gcgaggggtg atttggaaaa gaagacctgg gtggaaagcg 1980
gtttggatga aaagatttca ggcaaagact tgcaggaaga tgatgataac ggcgatgtga 2040
atcgtcaaag gtacgggcca gcttgtgcct aatagaaggt tgagaccagc agagactgct 2100
gtgagtttct cccggctccg aggctgaacg gggactgtga gcgatccccc tgctgcaggg 2160
cgagtggcct gtccagaccc ctgtgaggcc ccgggaaagg tacagccctg tctgcggtgg 2220
ctgctttgtt ggaaagaggg agggcctcct gcggtgtgct tgtcaagcag tggtcaaacc 2280
ataatctctt ttcactgggg ccaaactgga gcccagatgg gttaatttcc agggtcagac 2340
attacggtct ctcctcccct gccccctccc gcctgttttt cctcccgtac tgctttcagg 2400
tcttgtaaaa taagcatttg gaagtcttgg gaggcctgcc tgctagaatc ctaatgtgag 2460
gatgcaaaag aaatgatgat aacattttga gataaggcca aggagacgtg gagtaggtat 2520
ttttgctact ttttcatttt ctggggaagg caggaggcag aaagacgggt gttttatttg 2580
gtctaatacc ctgaaaagaa gtgatgactt gttgcttttc aaaacaggaa tgcatttttc 2640
cccttgtctt tgttgtaaga gacaaaagag gaaacaaaag tgtctccctg tggaaaggca 2700
taactgtgac gaaagcaact tttataggca aagcagcgca aatctgaggt ttcccgttgg 2760
ttgttaattt ggttgagata aacattcctt tttaaggaaa agtgaagagc agtgtgctgt 2820
cacacaccgt taagccagag gttctgactt cgctaaagga aatgtaagag gttttgttgt 2880
ctgttttaaa taaatttaat tcggaacaca tgatccaaca gactatgtta aaatattcag 2940

ggaaatctct cccttcattt actttttctt gctataagcc tatatttagg tttcttttct 3000
atttttttct cccatttgga tcctttgagg taaaaaaaca taatgtcttc agcctcataa 3060
taaaggaaag ttaattaaaa aaaaaaagca aagagccatt ttgtcctgtt ttcttggttc 3120
catcaatctg tttattaaac atcatccata tgctgaccct gtctctgtgt ggttgggttg 3180
ggaggcgatc agcagatacc atagtgaacg aagaggaagg tttgaaccat gggccccatc 3240
tttaaagaaa gtcattaaaa gaaggtaaac ttcaaagtga ttctggagtt ctttgaaatg 3300
tgctggaaga cttaaattta ttaatcttaa atcatgtact tttttctgt aatagaactc 3360
ggattctttt gcatgatggg gtaaagctta gcagagaatc atgggagcta acctttatcc 3420
cacctttgac actaccctcc aatcttgcaa cactatcctg tttctcagaa cagtttttaa 3480
atgccaatca tagagggtac tgtaaagtgt acaagttact ttatatatgt aatgttcact 3540
tgagtggaac tgctttttac attaaagtta aaatcgatct tgtgtttctt caaccttcaa 3600
aactatctca tctgtcagat ttttaaaact ccaacacagg ttttggcatc ttttgtgctg 3660
tatcttttaa gtgcatgtga aatttgtaaa atagagataa gtacagtatg tatattttgt 3720
aaatctccca tttttgtaag aaaatatata ttgtatttat acatttttac tttggatttt 3780
tgttttgttg gctttaaagg tctaccccac tttatcacat gtacagatca caaataaatt 3840
tttttaaata c 3851

<210> 22
<211> 574
<212> PRT
<213> Homo sapiens

<400> 22

Met Arg Asp Pro Gly Ala Ala Ala Pro Leu Ser Ser Leu Gly Leu Cys
1 5 10 15

Ala Leu Val Leu Ala Leu Leu Gly Ala Leu Ser Ala Gly Ala Gly Ala
20 25 30

Gln Pro Tyr His Gly Glu Lys Gly Ile Ser Val Pro Asp His Gly Phe
35 40 45

Cys Gln Pro Ile Ser Ile Pro Leu Cys Thr Asp Ile Ala Tyr Asn Gln
50 55 60

Thr Ile Leu Pro Asn Leu Leu Gly His Thr Asn Gln Glu Asp Ala Gly
65 70 75 80

Leu Glu Val His Gln Phe Tyr Pro Leu Val Lys Val Gln Cys Ser Pro
85 90 95

Glu Leu Arg Phe Phe Leu Cys Ser Met Tyr Ala Pro Val Cys Thr Val
100 105 110

Leu Asp Gln Ala Ile Pro Pro Cys Arg Ser Leu Cys Glu Arg Ala Arg
115 120 125

Gln Gly Cys Glu Ala Leu Met Asn Lys Phe Gly Phe Gln Trp Pro Glu
130 135 140

Arg Leu Arg Cys Glu Asn Phe Pro Val His Gly Ala Gly Glu Ile Cys
145 150 155 160

Val Gly Gln Asn Thr Ser Asp Gly Ser Gly Gly Pro Gly Gly Gly Pro
165 170 175

Thr Ala Tyr Pro Thr Ala Pro Tyr Leu Pro Asp Leu Pro Phe Thr Ala
180 185 190

Leu Pro Pro Gly Ala Ser Asp Gly Arg Gly Arg Pro Ala Phe Pro Phe
195 200 205

Ser Cys Pro Arg Gln Leu Lys Val Pro Pro Tyr Leu Gly Tyr Arg Phe
210 215 220

Leu Gly Glu Arg Asp Cys Gly Ala Pro Cys Glu Pro Gly Arg Ala Asn
225 230 235 240

Gly Leu Met Tyr Phe Lys Glu Glu Glu Arg Arg Phe Ala Arg Leu Trp
245 250 255

Val Gly Val Trp Ser Val Leu Cys Cys Ala Ser Thr Leu Phe Thr Val
260 265 270

Leu Thr Tyr Leu Val Asp Met Arg Arg Phe Ser Tyr Pro Glu Arg Pro
275 280 285

Ile Ile Phe Leu Ser Gly Cys Tyr Phe Met Val Ala Val Ala His Val
290 295 300

Ala Gly Phe Leu Leu Glu Asp Arg Ala Val Cys Val Glu Arg Phe Ser
305 310 315 320

Asp Asp Gly Tyr Arg Thr Val Ala Gln Gly Thr Lys Lys Glu Gly Cys
325 330 335

Thr Ile Leu Phe Met Val Leu Tyr Phe Phe Gly Met Ala Ser Ser Ile
340 345 350

Trp Trp Val Ile Leu Ser Leu Thr Trp Phe Leu Ala Ala Gly Met Lys
355 360 365

Trp Gly His Glu Ala Ile Glu Ala Asn Ser Gln Tyr Phe His Leu Ala
370 375 380

```
Ala Trp Ala Val Pro Ala Val Lys Thr Ile Thr Ile Leu Ala Met Gly
385                 390                 395                 400

Gln Val Asp Gly Asp Leu Leu Ser Gly Val Cys Tyr Val Gly Leu Ser
                405                 410                 415

Ser Val Asp Ala Leu Arg Gly Phe Val Leu Ala Pro Leu Phe Val Tyr
            420                 425                 430

Leu Phe Ile Gly Thr Ser Phe Leu Leu Ala Gly Phe Val Ser Leu Phe
        435                 440                 445

Arg Ile Arg Thr Ile Met Lys His Asp Gly Thr Lys Thr Glu Lys Leu
    450                 455                 460

Glu Lys Leu Met Val Arg Ile Gly Val Phe Ser Val Leu Tyr Thr Val
465                 470                 475                 480

Pro Ala Thr Ile Val Leu Ala Cys Tyr Phe Tyr Glu Gln Ala Phe Arg
                485                 490                 495

Glu His Trp Glu Arg Thr Trp Leu Leu Gln Thr Cys Lys Ser Tyr Ala
            500                 505                 510

Val Pro Cys Pro Pro Gly His Phe Pro Pro Met Ser Pro Asp Phe Thr
        515                 520                 525

Val Phe Met Ile Lys Tyr Leu Met Thr Met Ile Val Gly Ile Thr Thr
    530                 535                 540

Gly Phe Trp Ile Trp Ser Gly Lys Thr Leu Gln Ser Trp Arg Arg Phe
545                 550                 555                 560

Tyr His Arg Leu Ser His Ser Ser Lys Gly Glu Thr Ala Val
                565                 570
```